# EUROPEAN PATENT SPECIFICATION

(11) **EP 4 373 433 B1**
(45) Date of publication and mention of the grant of the patent: **30.07.2025**
(21) Application number: 22783590.7
(22) Date of filing: 23.07.2022
(51) Int. Cl.: A61B 34/30, A61B 34/20, A61B 34/32, A61B 34/37

(54) **COAXIAL ENDOVASCULAR ASSEMBLY MANAGEMENT, TRACKING, AND CONTROL**
VERWALTUNG, VERFOLGUNG UND STEUERUNG EINER KOAXIALEN ENDOVASKULÄREN ANORDNUNG
GESTION, SUIVI ET COMMANDE D'ENSEMBLE ENDOVASCULAIRE COAXIAL

(30) Priority: 23.07.2021 US 202163225317 P; 22.02.2022 US 202263312798 P
(43) Date of publication of application: 29.05.2024
(60) Divisional application: 25178223.1
(73) Proprietor: Stryker Corporation, Portage, MI 49002-9711 (US); Stryker European Operations Limited, Carrigtwohill, Co. Cork T45 HX08 (IE)
(72) Inventor: PORTER, Stephen, Piedmont, California 94610 (US); ESCH, Brady, Kalamazoo, Michigan 49002 (US); KARABEY, Halil, Kalamazoo, Michigan 49002 (US); WANG, Han-Wei, Fremont, California 94538 (US); CHATURVEDI, Anirudh Anupam, Fremont, California 94538 (US)
(74) Representative: Hauck Patent- und Rechtsanwälte PartmbB
(86) International application number: PCT/US2022/038099
(87) International publication number: WO 2023/004175

(56) References cited:
- WO-A1-2021/011554
- WO-A1-2022/144266
- WO-A1-2022/165241
- US-A1- 2002 177 789
- US-A1- 2016 235 946
- US-A1- 2020 345 483

## Description

### FIELD

The present disclosure relates generally to intravascular catheters and procedures, and more particularly, to techniques for managing and controlling coaxial endovascular assemblies.

### BACKGROUND

The use of intravascular medical devices has become an effective method for diagnosing and/or treating many types of vascular disease. In general, a suitable intravascular device is inserted into the vascular system of the patient and navigated through the vasculature to a desired target site. Using this method, virtually any target site in the patient's vascular system may be accessed, including the coronary, cerebral, and peripheral vasculature.

During one conventional endovascular procedure, a guide sheath is inserted into a patient's femoral artery through an introducer and positioned proximate the target intravascular site. A guidewire is then inserted into the guide sheath and maneuvered through the patient's arterial system until the guidewire reaches the target intravascular site. A working catheter is then moved along the guidewire until the distal end of the working catheter is positioned proximate the target intravascular site. The working catheter can then be operated to perform the diagnostic and/or interventional procedure at the target intravascular site. Manipulation of the catheters and guidewire typically requires these devices to be manually advanced and rotated to facilitate their navigation through the tortuous vasculature of the patient. Rotary hemostasis valves (RHVs) are typically used between fluid manifolds and the respective catheters to provide fluid to the catheters and prevent backflow of blood through the catheters, while allowing rotation of the catheters during catheter manipulations.

Performing such endovascular procedures reliably and precisely is an extremely tedious process, requiring a substantial amount of time and skill, and potentially causing a high degree of fatigue in the surgeon. Such endovascular procedures are typically performed under fluoroscopy to allow surgeons to visualize the placement of the catheters and guidewire relative to the anatomy of the patient, thereby subjecting such surgeons to significant irradiation over time if they perform many catheterization procedures.

In order to facilitate precise and robust control of intravascular devices, as well as to reduce the risk of irradiation to surgeons, numerous robotic endovascular systems for manipulating endovascular devices (e.g., tracking the endovascular devices relative to each other) have been proposed and some implemented in the clinical setting. In general, such robotic endovascular systems generally carry out (as a mechanical surrogate) directed inputs in the form of input commands into a master input device (e.g., via a joystick or other control device manipulated by operating physician), and translate those directed inputs into movement instructions for the slave device of such robotic endovascular systems to manipulate the endovascular devices. Some fully automated algorithmic and/or augmented motions for robotic endovascular systems have been proposed, such as adding a guidewire dotter, twisting or retracting an endovascular device, or even pre-programmed simultaneous movement of multiple endovascular devices (e.g., movement of a catheter and a delivery wire to deploy a stent).

The advent of robotic endovascular systems has also made the work area much more streamlined and efficient as all of the equipment on the table, such as catheters, guidewires, rotary hemostasis valves, and fluid manifolds are necessarily organized and/or incorporated by the robotic system components. However, known robotic endovascular systems use arms that reach over the surgical table and manipulate the intravascular devices. The known robotic systems are large, clumsy to operate, and relatively expensive to manufacture. Furthermore, the presence of a robot at the surgical site is problematic particularly when the robot is large and may impede access to the patient during surgery.

Furthermore, to prevent catheter prolapse during advancement of the flexible catheters, current robotic systems tend to use friction roller advance or other drive systems, which require making a fair amount of catheter length unusable for access and may cause catheter stack-up issues. As most catheters today are designed with minimal unusable length set-ups (i.e., the length of a single RHV) in order to get full use out of the variable robotic endovascular systems, custom length catheters may be needed or certain intravascular procedures may be ruled out due to length compatibility issues with current catheter robotic systems.

Also, not every endovascular procedure is best performed via fully remote operation (i.e., remotely providing input commands to a master input device). In many situations, manual manipulation of the endovascular devices is required or highly preferable. However, known robotic systems, when loaded with the intravascular devices, are not easily accessible to be used in manual operation (i.e., allowing direct hands-on manual manipulation of the endovascular devices) if so desired or necessary for emergent situations, and are not easily dynamically adaptable to different catheter workflows. In other situations, manual manipulation of some of the endovascular devices during an endovascular procedure is required or highly preferable, while automated manipulation of others of the endovascular devices during the same endovascular procedure is required or highly preferable. However, known robotic systems are not capable of operating in such a hybrid manual/automated mode. In contrast, purely manual systems oftentimes require two persons (e.g., a physician and a trained assistant) to manually operate the different endovascular devices in synchrony to perform the desired medical procedure.

WO 2021011554 A1 describes a robotic drive system for driving one or more elongated medical devices, the robotic drive can include a linear member and at least four device modules coupled to the linear member. Each device module may be independently controllable. The plurality of device modules may be switched between a first configuration where each device module is populated with an elongated medical device and a second configuration where a subset of the at least four device modules is populated with an elongated medical device.

US 2016235946 A1 describes an instrument driver comprising a base and a jaw assembly coupled to the base. The jaw assembly includes a first jaw having a gripping surface and a second jaw having a gripping surface. The instrument driver further comprises a driver assembly operably coupled to the jaw assembly to advance an elongated member relative to the base by translating the first and second jaws toward each other, thereby closing the jaw assembly and gripping the member between the respective gripping surfaces of the first and second jaws, translating the jaw assembly in a first axial direction when the jaw assembly is closed, translating the first and second jaws away from each other, thereby opening the jaw assembly and releasing the member from between the respective gripping surfaces of the first and second jaws, and translating the jaw assembly in a second axial direction when the jaw assembly is opened.

US 2002177789 A1 describes an advancer system for moving an elongate medical device within a body. The system includes a drive unit having a motor. The drive unit is configured to translate movement of the motor to the device so as to alternately advance and retract the device relative to the body. The advancer system also includes a user-operable control system configured to control the drive unit. The control system can interface with a magnetic navigation system. The system allows an operating physician to control catheter advancement and retraction while remaining outside an x-ray imaging field.

US 2020345483 A1 describes a support device for releasably retaining a delivery system handle while delivering a medical device to a target within a human are described herein. The support device is configured to engage the delivery system handle and enable a user to maneuver a distal end portion of the delivery system by manipulating features of the support device.

There, thus, is a need for an improved endovascular management and tracking system that can be easily setup and customized to any one of a variety of different hospital environments and catheter workflows, prevents catheter prolapse, is relatively inexpensive, can be used with standardized length catheters, can be easily converted from robotic operation to manual operation, and/or may operate in a hybrid manual/automated mode.

The invention is based on the task of providing an improved endovascular management and tracking system of the type described above. The task is solved by a device according to claim 1. Advantageous embodiments are indicated in the dependent claims.

### SUMMARY

The invention concerns an endovascular management and tracking system according to claim 1.

According to an example that may, if applicable, provide details to further specify embodiments claimed or described in this application, an endovascular management and tracking system comprises a track (which may or may not be flexible) and at least one shuttle to which at least one elongate medical device (e.g., a guide sheath, a working catheter, and/or a guidewire) can be respectively affixed. Each of shuttle(s) is configured for being mechanically coupled to the track, and comprises a sled configured for riding on the track, an axial drive mechanism carried by the sled and configured for being actuated to axially translate the respective shuttle along the track, and a first actuator (e.g., a manual actuator or a motor) carried by the sled and configured for actuating the axial drive mechanism. Each of the shuttle(s) may optionally comprise a rotary drive mechanism carried by the sled and configured for being actuated to rotate the respective elongate medical device about its longitudinal axis, and a second actuator (e.g., a manual actuator or a motor) carried by the sled and configured for actuating the rotary drive mechanism.

In one embodiment, the endovascular management and tracking system comprises a plurality of shuttles to which a plurality of elongate medical devices can be respectively affixed. In this embodiment, each of the plurality of shuttles may be configured for being detachably mechanically coupled to the track, e.g., threaded onto the track or laterally mated to the track. In this embodiment, each subsequent one of the plurality of shuttles is configured for slidably receiving a distal end of a respective one of the plurality of elongate medical devices affixed to a preceding one of the plurality of shuttles, such that the respective elongate medical device affixed to the preceding shuttle is coaxially disposed within a respective one of the plurality of elongate medical devices affixed to the subsequent shuttle. In one optional embodiment, one of the plurality of shuttles may be a master shuttle, and another one of the plurality of shuttles may be a slave shuttle. In this embodiment, the endovascular management and tracking system further comprising a controller (e.g., an electrical controller or a mechanical controller) configured for actuating the axial drive mechanism of the slave shuttle in response to actuation of the axial drive mechanism of the master shuttle, such that the slave shuttle is axially translated along the track in synchronous coordination with the axial translation of the master shuttle along the track.

In another embodiment, each of the shuttle(s) further comprises an on-board fluid management control assembly carried by the sled and configured for being fluidly coupled to the elongate medical device affixed to the respective shuttle. In this embodiment, the on-board fluid management control assembly may comprise a rotary hemostasis valve (RHV) configured for being removed from the respective shuttle.

According to the invention, the endovascular management and tracking system further comprises a locking mechanism carried by the sled and configured for being manually operated to alternately engage and disengage the axial drive mechanism to the track.

In yet another embodiment, each of the elongate medical device(s) is flexible, and each of one or more of the shuttle(s) further comprises an anti-bucking mechanism configured for preventing the respective elongate medical device from prolapsing in response to axial compression applied to the respective elongate medical device. Each of the anti-buckling mechanism(s) may comprise a tubular member through which the respective elongate medical device is configured for being slidably disposed. Such tubular member may have a slit extending along a length of the tubular member. In this embodiment, a distal end of each of the anti-bucking mechanism(s) is configured for being affixed to a location, and each of the anti-bucking mechanism(s) is configured for lengthening when the respective shuttle is axially translated away from the location and shortening when the respective shuttle is axially translated toward the location. The location to which the distal end of one of the anti-buckling mechanisms is affixed may be a fixed location relative to a patient. If the endovascular management and tracking system comprises a plurality of shuttles, and location to which the distal end of the anti-buckling mechanism of a preceding one of the plurality of shuttles is affixed may be a subsequent one of the plurality of shuttles. In one embodiment, the anti-bucking mechanism comprises the axial drive mechanism.

A robotic endovascular system may comprise the aforementioned endovascular management and tracking system and a master input device configured for receiving manual commands from a user and transmitting control signals to the motor of each of the shuttle(s). In the robotic endovascular system, each of shuttle(s) may optionally comprise a manual actuator configured for actuating the axial drive mechanism.

In accordance with a second aspect of the present disclosure, an endovascular management and tracking system comprises a flexible track, and at least one shuttle to which at least one elongate medical device (e.g., a guide sheath, a working catheter, and/or a guidewire) can be respectively affixed. In one embodiment, the flexible track may have a top-to-bottom flexibility and a side-to-side flexibility less than the top-to-bottom flexibility. In an optional embodiment, the flexible track comprises a plurality of flexible track segments configured for being removably attached to each other. Each of the shuttle(s) is configured for being mechanically coupled to the track. In one embodiment, the flexible track is an open monorail on which the shuttle(s) rides.

The endovascular management and tracking system further comprises a drive assembly (e.g., manually actuated or motor actuated) configured for axially translating the shuttle(s) along the flexible track. In one embodiment, the flexible track is configured for being contoured by a user to a hospital room environment (e.g., one or more of a table, drape, and patient). The endovascular management and tracking system may further comprise a fastener configured for affixing a distal end of the flexible track relative to a patient. In one embodiment, the drive assembly comprises at least one axial drive mechanism and at least one first actuator. The shuttle(s) respectively comprise the axial drive mechanism(s) and at least one first actuator. Each of the axial drive mechanism(s) may be configured for being actuated to axially translate the respective shuttle along the flexible track, and each of the first actuator(s) may be configured for actuating the respective axial drive mechanism. In this embodiment, the drive assembly may further comprise at least one rotary drive mechanism and at least a second actuator. The shuttle(s) respectively comprise the rotary drive mechanism(s) and at least one second actuator. Each of the rotary drive mechanism(s) may be configured for being actuated to rotate the respective elongate medical device about its longitudinal axis relative to the respective shuttle, and each of the second actuator(s) may be configured for actuating the respective rotary drive mechanism. In this embodiment, the endovascular management and tracking system may further comprise a locking mechanism configured for being manually operated to alternately engage and disengage the axial drive mechanism to the flexible track.

In one embodiment, the endovascular management and tracking system further comprises a plurality of track support arms. Each of the plurality of track support arms has one end configured for being affixed to a procedure table and another end configured for being affixed to the track, such that the track may be suspended above the procedure table. In this embodiment, each of the plurality of track support arms may be configured for being adjusted relative to the procedure table to select a height of the other end of the respective arm relative to the procedure table. In this embodiment, the endovascular management and tracking system may further comprise a plurality of feet affixed to the other ends of the plurality of track support arms, and the flexible track may be configured for being affixed atop the plurality of feet. Each of the plurality of feet may be configured for being adjusted relative to the track support arms to select a height of the respective foot relative to the procedure table. The flexible track may comprise a channel along the length of the flexible track, and each of the plurality of feet may comprise a cleat configured for being detachably affixed to the channel of the flexible track.

In another embodiment, the flexible track comprises a track rack configured for stably rested on a drape, and a track rail affixed to the track rack, such that the track rail may be raised above the drape. The shuttle(s) is configured for being mechanically coupled to the track rail. In this embodiment, the track rack may comprise an elongated base configured for being stably rested on the drape, and one or more pedestals affixed on top of the elongated base and to which the track rail is affixed. The elongated base may take the form of a rectangular frame having a pair of elongated frame members and a series of crossbars affixing the pair of elongated frame members together.

In still another embodiment, the endovascular management and tracking system comprises a plurality of shuttles to which a plurality of elongate medical devices can be respectively affixed. In this embodiment, each of the plurality of shuttles may be configured for being detachably mechanically coupled to the flexible track, e.g., threaded onto the flexible track or laterally mated to the flexible track. In this embodiment, each subsequent one of the plurality of shuttles is configured for slidably receiving a distal end of a respective one of the plurality of elongate medical devices affixed to a preceding one of the plurality of shuttles, such that the respective elongate medical device affixed to the preceding shuttle is coaxially disposed within a respective one of the plurality of elongate medical devices affixed to the subsequent shuttle. In one optional embodiment, one of the plurality of shuttles may be a master shuttle, and another one of the plurality of shuttles may be a slave shuttle. In this embodiment, the endovascular management and tracking system further comprising a controller (e.g., an electrical controller or a mechanical controller) configured for actuating the axial drive mechanism of the slave shuttle in response to actuation of the axial drive mechanism of the master shuttle, such that the slave shuttle is axially translated along the flexible track in synchronous coordination with the axial translation of the master shuttle along the flexible track.

In yet another embodiment, each of the shuttle(s) further comprises an on-board fluid management control assembly carried by the sled and configured for being fluidly coupled to the elongate medical device affixed to the respective shuttle. In this embodiment, the on-board fluid management control assembly may comprise a rotary hemostasis valve (RHV) configured for being removed from the respective shuttle.

In yet another embodiment, each of the elongate medical device(s) is flexible, and each of one or more of the shuttle(s) further comprises an anti-bucking mechanism configured for preventing the respective elongate medical device from prolapsing in response to axial compression applied to the respective elongate medical device. Each of the anti-buckling mechanism(s) may comprise a tubular member through which the respective elongate medical device is configured for being slidably disposed. Such tubular member may have a slit extending along a length of the tubular member. In this embodiment, a distal end of each of the anti-bucking mechanism(s) may be configured for being affixed to a location, and each of the anti-bucking mechanism(s) may be configured for lengthening when the respective shuttle is axially translated away from the location and shortening when the respective shuttle is axially translated toward the location. The location to which the distal end of one of the anti-buckling mechanisms is affixed may be a fixed location relative to a patient. If the endovascular management and tracking system comprises a plurality of shuttles, and location to which the distal end of the anti-buckling mechanism of a preceding one of the plurality of shuttles is affixed may be a subsequent one of the plurality of shuttles. In one embodiment, the anti-bucking mechanism comprises the driver assembly.

A robotic endovascular system may comprise the aforementioned endovascular management and tracking system and a master input device configured for receiving manual commands from a user and transmitting control signals to the motor of each of the shuttle(s). In the robotic endovascular system, each of shuttle(s) may optionally comprise a manual actuator configured for actuating the axial drive mechanism.

In accordance with a third aspect of the present disclosure, an endovascular management and tracking system comprises a driver system (e.g., manually actuated or motor actuated) configured for axially translating at least one flexible elongate medical device (e.g., a guide sheath, a working catheter, and/or a guidewire) and optionally rotating each of the flexible elongate medical device(s) about its longitudinal axis. The endovascular management and tracking system further comprises at least one anti-buckling mechanism configured for preventing a respective one of the elongate medical device(s) from prolapsing in response to axial compression applied to the respective elongate medical device. Each of the anti-buckling mechanism(s) comprises an axially split sheath configured for being alternately furled and unfurled in response to axial translation of the respective elongate medical device. The axially split sheath has a flattened proximal portion configured for assuming a flattened state when furled, a tubular distal portion configured for assuming a tubular state when unfurled, and a lumen configured for slidably receiving the respective elongate medical device therein at the tubular distal portion.

In one embodiment, the axially split sheath is pre-shaped to assume a tubular shape, such that the flattened proximal portion of the axially split sheath assumes the flattened state only in response to an external force. In another embodiment, the axially split sheath has a bi-stable structure, such that the flattened proximal portion of the axially split sheath remains in the flattened state in the absence of an external force, and the tubular distal portion of the axially split sheath remains in the tubular state in the absence of an external force.

In still another embodiment, the endovascular management and tracking system further comprises a flattening mechanism configured for transitioning the axially split sheath from the tubular state into the flattened state, such that the flattened proximal portion of the axially split sheath extends proximally from the flattening mechanism. As one example, the flattening mechanism may comprise pinch rollers between which the axially split sheath is disposed. In this embodiment, the endovascular management and tracking system may further comprise a tube forming mechanism configured for transitioning the axially split sheath from the flattened state to the tubular state. The tube forming mechanism may be located distal to the flattening mechanism, such that the tubular distal portion of the axially split extends distally from the tube forming mechanism. The tube forming mechanism may comprise an arcuate bearing surface against which the axially split sheath contacts.

In yet another embodiment, each of the anti-buckling mechanism(s) further comprises a take-up reel disposed within the housing. The take-up reel is configured for alternately rolling the flattened proximal portion of the axially split sheath when furled, and unrolling the flattened proximal portion of the axially split sheath when unfurled. Each of the anti-buckling mechanism(s) may further comprise a take-up biasing mechanism configured for biasing the take-up reel to roll the flattened proximal portion of the axially split sheath when furled. In one example, the take-up biasing mechanism may comprise a tension spring affixed to the take-up reel for applying a constant tension to the flattened proximal portion of the axially split sheath. In another example, the take-up biasing mechanism may comprise a motor mechanically coupled to the take-up reel for applying a constant tension to the flattened proximal portion of the axially split sheath.

In another example, the take-up biasing mechanism may comprise an idler pulley affixed to the take-up reel, such that the take-up reel rotates in unison with the idler pulley, a driver pulley configured for rotating when the flattened proximal portion of the axially split sheath is furled, and a drive belt coupling the idler pulley to the driver pulley, such that the idler pulley rotates in response to rotation of the driver pulley. The diameter of the idler pulley may be less than a diameter of the driver pulley. The idler pulley may be optionally ratcheted, such that the idler pulley free spins when the take-up reel unrolls the flattened proximal portion of the axially split sheath when unfurled. In this example, the take-up biasing mechanism may further comprise a clutching mechanism (e.g., a frictional clutch between the idler pulley and the drive belt) configured for matching a linear speed of the flattened proximal portion of the axially split sheath wrapped around take-up reel to a linear speed of the tubular distal portion of the axially split sheath relative to the housing. In this example, the take-up biasing mechanism may further comprise pinch rollers between which the axially split sheath is frictionally disposed, such that the pinch rollers rotate as the axially split sheath is furled. In this case, the pulley may be affixed to one of the pinch rollers, such that driver pulley rotates in unison with the one pinch roller.

In yet another embodiment, the endovascular management and tracking system further comprises a track (which may or may not be flexible), and the driver system comprises at least one shuttle configured for being mechanically coupled to the track. In this embodiment, each of shuttle(s) comprises a sled to which a respective one of the elongate medical device(s) is configured for being affixed, an axial drive mechanism carried by the sled and configured for being actuated to axially translate the respective shuttle along the track, and a first actuator carried by the sled and configured for actuating the axial drive mechanism. Each of the shuttle(s) may optionally comprise a rotary drive mechanism carried by the sled and configured for being actuated to rotate the respective elongate medical device about its longitudinal axis, and a second actuator (e.g., a manual actuator or a motor) carried by the sled and configured for actuating the rotary drive mechanism. A respective one of the anti-buckling mechanism(s) is carried by the sled and is configured for preventing the respective elongate medical device from prolapsing in response to axial compression applied to the respective elongate medical device.

In this embodiment, the endovascular management and tracking system may comprise a plurality of shuttles to which a plurality of elongate medical devices can be respectively affixed. Each of the plurality of shuttles may be configured for being detachably mechanically coupled to the track, e.g., threaded onto the track or laterally mated to the track. Each subsequent one of the plurality of shuttles may be configured for slidably receiving a distal end of a respective one of the plurality of elongate medical devices affixed to a preceding one of the plurality of shuttles, such that the respective elongate medical device affixed to the preceding shuttle is coaxially disposed within a respective one of the plurality of elongate medical devices affixed to the subsequent shuttle. In one optional embodiment, one of the plurality of shuttles may be a master shuttle, and another one of the plurality of shuttles may be a slave shuttle. In this embodiment, the endovascular management and tracking system further comprising a controller (e.g., an electrical controller or a mechanical controller) configured for actuating the axial drive mechanism of the slave shuttle in response to actuation of the axial drive mechanism of the master shuttle, such that the slave shuttle is axially translated along the track in synchronous coordination with the axial translation of the master shuttle along the track.

In this embodiment, each of the shuttle(s) may further comprise an on-board fluid management control assembly carried by the sled and configured for being fluidly coupled to the elongate medical device affixed to the respective shuttle. The on-board fluid management control assembly may comprise a rotary hemostasis valve (RHV) configured for being removed from the respective shuttle. In this embodiment, the endovascular management and tracking system may further comprises a locking mechanism carried by the sled and configured for being manually operated to alternately engage and disengage the axial drive mechanism to the track. In this embodiment, the endovascular management and tracking system may further comprise a locking mechanism configured for being manually operated to alternately engage and disengage the axial drive mechanism to the track.

In this embodiment, a distal end of each of the anti-bucking mechanism(s) may be configured for being affixed to a location, and each of the anti-bucking mechanism(s) may be configured for lengthening when the respective shuttle is axially translated away from the location and shortening when the respective shuttle is axially translated toward the location. The location to which the distal end of one of the anti-buckling mechanisms is affixed may be a fixed location relative to a patient. If the endovascular management and tracking system comprises a plurality of shuttles, and location to which the distal end of the anti-buckling mechanism of a preceding one of the plurality of shuttles is affixed may be a subsequent one of the plurality of shuttles. In one embodiment, the anti-bucking mechanism comprises the driver system.

A robotic endovascular system may comprise the aforementioned endovascular management and tracking system and a master input device configured for receiving manual commands from a user and transmitting control signals to the motor of each of the shuttle(s). In the robotic endovascular system, each of shuttle(s) may optionally comprise a manual actuator configured for actuating the axial drive mechanism.

In accordance with a fourth aspect of the present disclosure, an endovascular management and tracking system comprises a track (which may or may not be flexible), a slave shuttle to which a first elongate medical device can be affixed, and a master shuttle to which a second elongate medical device can be affixed. The slave shuttle and master shuttle are configured for being mechanically coupled to the track. In one embodiment, each of the master shuttle and the slave shuttle is configured for being detachably mechanically coupled to the track. In another embodiment, one of the master shuttle and the slave shuttle is a subsequent shuttle, the other of the master shuttle and the slave shuttle is a preceding shuttle, and the master shuttle is configured for receiving a distal end of a respective one of the first and second elongate medical devices affixed to a preceding shuttle, such that the respective elongate medical device affixed to the preceding shuttle is coaxially disposed within a respective one of the first and second elongate medical devices affixed to the subsequent shuttle. In still another embodiment, each of first and second elongate medical devices is flexible, in which case, each of at least one of the master shuttle and the slave shuttle further comprises an anti-bucking mechanism configured for preventing the respective elongate medical device from prolapsing in response to axial compression applied to the respective elongate medical device.

The endovascular management and tracking system further comprises a first axial drive mechanism configured for being actuated to axially translate the slave shuttle along the track. In one embodiment, the master shuttle further comprises a sled configured for riding on the track, and the first axial drive mechanism and the manual actuator are carried by the sled. The endovascular management and tracking system further comprises a controller configured for actuating the first axial drive mechanism in response to axial translation of the master shuttle manually along the track, such that the slave shuttle is axially translated along the track in synchronous coordination with the axial translation of the master shuttle along the track. In an optional embodiment, the slave shuttle may comprise a first rotary drive mechanism configured for being actuated to rotate the first elongate medical device about its longitudinal axis relative to the slave shuttle, and the master shuttle may comprise a second rotary drive mechanism configured for being actuated to rotate the second elongate medical device about its longitudinal axis relative to the master shuttle.

In one embodiment, the controller is an electrical controller. In this embodiment, the endovascular management and tracking system may further comprise encoders indicative of a location of the master shuttle relative to track, and one or more sensors configured for reading the encoders and outputting an electrical signal indicating the location of the master shuttle relative to the track. The electrical controller may be configured for controlling the first axial drive mechanism based on the electrical signal. The encoders may be disposed along the track, and the sensor(s) may be disposed on the master shuttle. In this case, the encoders may comprise a series of fiducial elements extending along a length of the track, and the sensor(s) may comprise a fiducial element reader configured for reading the fiducial elements.

In another embodiment, the controller is a mechanical controller. In this embodiment, the slave shuttle may further comprise a first sled configured for riding on the track, and the master shuttle may further comprise a second sled configured for riding on the track. The first axial drive mechanism may be carried by the first sled and configured for being actuated to axially translate the slave shuttle along the track, and the second axial drive mechanism may be carried by the second sled and configured for being actuated to axially translate the master shuttle along the track. The master shuttle may further comprise a manual actuator carried by the second sled and configured for actuating the second axial drive mechanism, and the slave shuttle may further comprise another manual actuator carried by the first sled and configured for actuating the first axial drive mechanism. The mechanical controller may comprise a control shaft mechanically coupled between the first and second axial drive mechanisms (e.g., in a detachable manner).

The control shaft may be configured for being mechanically coupled between the first and second axial drive mechanisms, such that the control shaft and one of the slave shuttle and the master shuttle are configured for being axially translated in unison, while the control shaft and the other of the slave shuttle and the master shuttle are configured for being axially translated relative to each other. In this case, the control shaft may be configured for rotating about its longitudinal axis in response to actuation of the second axial drive mechanism, and the first axial drive mechanism may be configured for being actuated in response to rotation of the control shaft about its longitudinal axis. The first axial drive mechanism may comprise a first worm drive, the second axial drive mechanism may comprise a second worm drive, and the control shaft may be operably coupled between the first and second worm drives. The first worm drive may be removably disposed within the slave shuttle. The first worm drive may comprise a first worm screw and a first worm gear, the second worm drive may comprise a second worm screw and a second worm gear, the control shaft may be operably coupled between the first and second worm screws, the first worm gear may be operably coupled between the first manual actuator and the first worm screw, and the second worm gear may be operably coupled between the second worm screw and the track. Each of the first and second worm screws may have a bore through which the control shaft is configured for being disposed, and the bores of the first and second worm screws and the outer periphery of the control shaft may be keyed. The master shuttle may further comprise a rotatable manual actuator carried by the sled and configured for actuating the second axial drive mechanism, and the second worm gear may be operably coupled between the rotatable manual actuator and the track. The first and second worm screws may have different pitches and/or opposite pitches.

In still another embodiment, the slave shuttle is axially translated along the track in synchronous coordination with the axial translation of the master shuttle along the track in accordance with an axial translation ratio. The axial translation ratio may be a negative axial translation ratio or a positive axial translation ratio. If the axial translation ratio is a negative axial translation ratio, one of the first and second elongate medical devices may be a stent deployment catheter, and the other of the first and second elongate medical devices may be a pusher member disposed within the stent deployment catheter and affixed to a stent within the stent deployment catheter. If the axial translation ratio is a positive axial translation ratio and unity, the slave shuttle may be axially translated along the track in synchronous coordination with the axial translation of the master shuttle along the track at a predetermined distance from the master shuttle. The slave shuttle may not be axially translated along the track as the master shuttle axially translates along the track until the slave shuttle is a predetermined distance from the master shuttle. In this case, the first elongate medical device may be catheter, and the second elongate medical device may be a guidewire, or the first elongate medical device may be a stentriever, and the second elongate medical device may be a catheter.

In accordance with a fifth aspect of the present disclosure, a control station for use with a robotic endovascular management and tracking system configured for axially translating and robotically rotationally translating a plurality of elongate medical devices of a coaxial endovascular assembly relative to each other is provided.

The control station comprises a master input device comprising a linear array of control elements configured for being manually axially translated along a longitudinal axis, and for being manually rotationally translated about the longitudinal axis. In one embodiment, each of the control elements is cylindrical. In another embodiment, the cylindrical control elements have different diameters. The control station further comprises at least one processor configured for operably coupling the linear array of control elements respectively to the elongate medical devices by commanding the robotic endovascular management and tracking system to axially translate each of the elongate medical devices in response to manual axial translation of each of the linear array of control elements along the longitudinal axis, and to rotationally translate each of the elongate medical devices in response to manual rotational translation of each of the linear array of control elements about the longitudinal axis.

In an optional embodiment, the control station further comprises at least one force feedback sensor configured for detecting a force exerted on each of the elongate medical devices when advanced within a vasculature of a patient, and a haptic interface configured for communicating tactile feedback to the hand of an operator as the operator manually axially translates and manually rotationally translates each of the control elements. In one example, the haptic interface is configured for communicating the tactile feedback to the hand of an operator via the master input device. In another example, the haptic interface comprises one or more haptic feedback gloves. In still another example, the haptic interface comprises one or more ultrasound pads.

In one embodiment, the master input device comprises a rail, and the control elements are physical control elements that are axially and rotationally slidably disposed on the rail.

In an embodiment with physical control elements, the control station may further comprise a sensor assembly configured for detecting the manual axial translation of each of the control elements along the longitudinal axis, for detecting the manual rotational translation of each of the control elements about the longitudinal axis, and for outputting signals indicative of the detected axial translation of each of the control elements along the longitudinal axis, and indicative of the detected rotational translation of each of the control elements about the longitudinal axis. The processor(s) may be configured for commanding the robotic endovascular management and tracking system to axially translate and rotationally translate each of the elongate medical devices in response to the control signals output by the sensor assembly. In this embodiment, the sensor assembly may comprise passive components and at least one active component, in which case, the passive components may be affixed to the control elements, and the active component(s) may be located off of the control elements. The sensor assembly may comprise a plurality of active components affixed within the rail respectively adjacent the passive components. In one example, the passive components comprise fiducial elements affixed around the circumference of each of the control elements. In another example, the passive components comprise a plurality of passive electromagnetic transponders respectively affixed to the control elements, and the active component(s) comprises an electromagnetic transceiver. In still another example, the control station further comprises a sensor box containing the active component(s), the passive components comprise a plurality of mechanical arms cantilevered on the control elements, and free ends of the mechanical arms are configured for operably interacting with the at least one active component within the sensor box.

In this embodiment, the control station may further comprise a plurality of single-use sterile control element covers configured for being slid into place respectively over the control elements. The sterile control element covers may be configured for overlapping each other, such that slidable fluid seals are formed between adjacent control elements to prevent contaminated fluid transfer between any adjacent control elements.

In another embodiment, the control elements are virtual control elements. The control station may further comprise a gesture monitoring system configured for capturing hand gestures made by an operator while virtually interacting with the virtual control elements.

In an embodiment with virtual control elements, the master input device may comprise a three-dimensional (3D) touchscreen configured for displaying interactive 3D representations of the virtual control elements. In this case, the 3D touchscreen may have an arcuate cross-section, and the virtual control elements may be arcuate. For example, the 3D touchscreen may be cylindrical, and the virtual control elements may be cylindrical. In another embodiment with virtual control elements, the control station may further comprise a head-mounted extended reality (XR) system configured for displaying the virtual control elements in a three-dimensional (3D) environment. In still another embodiment with virtual control elements, the control station may further comprise a two-dimensional (2D) display screen configured for displaying the virtual control elements. In yet another embodiment with virtual control elements, the control station may further comprise a holographic machine configured for projecting the virtual control elements in a three-dimensional (3D) environment.

A robotic endovascular system may comprise the aforementioned control station and robotic endovascular management and tracking system. In one embodiment, the robotic endovascular management and tracking system comprises a track and a plurality of shuttles to which the plurality of elongate medical devices can be respectively affixed. Each of the shuttle(s) are configured for being mechanically coupled to the track, and comprise a sled configured for riding on the track, an axial drive mechanism carried by the sled and configured for being actuated to axially translate the respective shuttle along the track, a first axial motor carried by the sled and configured for actuating the axial drive mechanism, a rotary drive mechanism carried by the sled and configured for being actuated to rotate the respective elongate medical device about its longitudinal axis, and a second motor carried by the sled and configured for actuating the rotary drive mechanism.

Other and further aspects and features of embodiments will become apparent from the ensuing detailed description in view of the accompanying figures.

### BRIEF DESCRIPTION OF THE DRAWINGS

The drawings illustrate the design and utility of preferred embodiments of the present disclosure, in which similar elements are referred to by common reference numerals. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention, which is defined only by the appended claims. In addition, an illustrated embodiment of the disclosed inventions needs not have all the aspects or advantages shown. An aspect or an advantage described in conjunction with a particular embodiment of the disclosed inventions is not necessarily limited to that embodiment and can be practiced in any other embodiments even if not so illustrated. In order to better appreciate how the above-recited and the other advantages and objects of the present disclosure are obtained, a more particular description of the present disclosure briefly described above will be rendered by reference to specific embodiments thereof, which are illustrated in the accompanying drawings. Understanding that these drawings depict only typical embodiments of the disclosure and are not therefore to be considered limiting of its scope, the disclosure will be described and explained with additional specificity and detail through the use of the accompanying drawings in which:
**Fig. 1** is a profile view of one embodiment of a robotic endovascular system constructed in accordance with the present invention;
**Fig. 2** is a top view of the robotic endovascular system of **Fig. 1****;**
**Fig 3** is a plan view of a catheter assembly of the robotic endovascular system of **Fig. 1****;**
**Fig. 4** is a cross-sectional view of the catheter assembly of **Fig. 3****,** taken along the **line 4-4;**
**Fig. 5** is a plan view of a proximal adapter of a guide sheath or a working catheter of the catheter assembly of **Fig. 4****;**
**Fig. 6** is a plan view of a one embodiment of an endovascular management and tracking system of the robotic endovascular system of **Fig. 1****;**
**Figs. 7A-7D** are various views of one embodiment of a shuttle of the endovascular management and tracking system of **Fig. 6****;**
**Fig. 8** is a plan view of a rotatable hemostasis valve (RHV) of the shuttle of **Figs. 7A-7D****;**
**Fig. 9** is a perspective view of an axially split sheath of an anti-buckling mechanism of the shuttle of **Figs. 7A-7D****;**
**Fig. 10** is a cross-sectional view of the axially split sheath of **Fig. 9****,** taken along the **line 10-10;**
**Figs. 11A-11D** are plan views illustrating the endovascular management and tracking system of **Fig. 6****,** particularly showing different relative axial locations of the shuttles and the resulting changing lengths of the axially split sheath of **Fig. 9****;**
**Figs. 12A-12D** are plan views illustrating different architectures of an anti-buckling mechanism of the shuttle of **Figs. 7A-7D****;**
**Fig. 13A** is a plan view of an alternative embodiment of an anti-buckling mechanism that can be used by the shuttle of **Figs. 7A-7D****,** particularly showing the anti-buckling mechanism in a fully extended state;
**Fig. 13B** is a plan view of the anti-buckling mechanism of **Fig. 13A****,** particularly showing the anti-buckling mechanism in a fully contracted state;
**Fig. 14A** is a plan view of another alternative embodiment of an anti-buckling mechanism that can be used by the shuttle of **Figs. 7A-7D****,** particularly showing the anti-buckling mechanism in a fully extended state;
**Fig. 14B** is a plan view of the anti-buckling mechanism of **Fig. 14A****,** particularly showing the anti-buckling mechanism in a fully contracted state;
**Fig. 15A** is a plan view of still another alternative embodiment of an anti-buckling mechanism that can be used by the shuttle of **Figs. 7A-7D****,** particularly showing the anti-buckling mechanism in a fully extended state;
**Fig. 15B** is a plan view of the anti-buckling mechanism of **Fig. 15A****,** particularly showing the anti-buckling mechanism in a fully contracted state;
**Fig. 16A** is a plan view of yet another alternative embodiment of an anti-buckling mechanism that can be used by the shuttle of **Figs. 7A-7D****,** particularly showing the anti-buckling mechanism in a fully extended state;
**Fig. 16B** is a plan view of the anti-buckling mechanism of **Fig. 16A****,** particularly showing the anti-buckling mechanism in a fully contracted state;
**Fig. 17A** is a plan view of yet another alternative embodiment of an anti-buckling mechanism that can be used by the shuttle of **Figs. 7A-7D****,** particularly showing the anti-buckling mechanism in a fully extended state;
**Fig. 17B** is a plan view of the anti-buckling mechanism of **Fig. 17A****,** particularly showing the anti-buckling mechanism in a fully contracted state;
**Fig. 18** is a plan view illustrating one embodiment of a control station of the robotic endovascular system of **Fig. 1****,** particularly showing one embodiment of a sterile sleeve that covers a master input device of the control station;
**Fig. 18A** is a plan view of one specific embodiment of the control station of **Fig. 18****;**
**Fig. 18B** is a plan view of another specific embodiment of the control station of **Fig. 18****;**
**Fig. 18C** is a plan view of still another specific embodiment of the control station of **Fig. 18****;**
**Fig. 18D** is a plan view of yet another specific embodiment of the control station of **Fig. 18****;**
**Fig. 18E** is a perspective view of control elements used in the control station of **Fig. 18D****;**
**Fig. 18F** is an axial view of mechanical arms disposed on the control elements of **Fig. 18E****,** particularly showing the mechanical arms clocked from each other;
**Fig. 18G** is a plan view of yet another specific embodiment of the control station of **Fig. 18****;**
**Fig. 18H** is a plan view of yet another specific embodiment of the control station of **Fig. 18****;**
**Fig. 18I** is a plan view of yet another specific embodiment of the control station of **Fig. 18****;**
**Fig. 18J** is a plan view of yet another specific embodiment of the control station of **Fig. 18****;**
**Fig. 18K** is a plan view of yet another specific embodiment of the control station of **Fig. 18****;**
**Fig. 18L** is a plan view of yet another specific embodiment of the control station of **Fig. 18****;**
**Fig. 19** is a plan view illustrating the control station of **Fig. 18****,** particularly showing another embodiment of a plurality of sterile sleeves that covers the master input device of the control station;
**Fig. 20** is a plan view of the plurality of sterile sleeves of **Fig. 19** stacked together for storage;
**Fig. 21** is a perspective view of another embodiment of an endovascular management and tracking system of the robotic endovascular system of **Fig. 1****;**
**Fig. 22** is a profile view of the endovascular management and tracking system of **Fig. 21****;**
**Fig. 23** is a top view of the endovascular management and tracking system of **Fig. 21****;**
**Fig. 24** is a bottom view of the endovascular management and tracking system of **Fig. 21****;**
**Fig. 25** is a perspective view of a track segment of the endovascular management and tracking system of **Fig. 21****;**
**Fig. 26** is a profile view of the track segment of **Fig. 25****;**
**Fig. 27** is a top view of the track segment of **Fig. 25****;**
**Fig. 28** is a front view of the track segment of **Fig. 25****;**
**Fig. 29** is a rear view of the track segment of **Fig. 25****;**
**Fig. 30** is a perspective view of the interaction between a sled of a shuttle and track segment of the endovascular management and tracking system of **Fig. 21****;**
**Fig. 31** is a perspective view of a shuttle of the endovascular management and tracking system of **Fig. 21****,** particularly showing an upper casing portion removed from the shuttle;
**Fig. 32** is another perspective view of the shuttle of **Fig. 31****,** particularly showing the upper casing portion removed from the shuttle;
**Fig. 33** is still another perspective view of the shuttle of **Fig. 31****,** particularly showing the upper casing portion and RHV removed from the shuttle to expose an axial drive mechanism of the shuttle;
**Fig. 34** is yet another perspective view of the shuttle of **Fig. 31****,** particularly showing the upper casing portion, sled, and RHV removed from the shuttle to expose the axial drive mechanism of the shuttle, wherein the axial drive mechanism is disengaged from the track;
**Fig. 35** is yet another perspective view of the shuttle of **Fig. 31****,** particularly showing the upper casing portion, sled, and RHV removed from the shuttle to expose the axial drive mechanism of the shuttle, wherein the axial drive mechanism is engaged with the track;
**Fig. 36** is an exploded view of the axial drive mechanism of the shuttle of **Fig. 21****;**
**Fig. 37** is a close-up perspective view of the axial drive mechanism of the shuttle of **Fig. 21****;**
**Fig. 38** is a profile view of the axial drive mechanism of **Fig. 37****;**
**Fig. 39** is a top view of the axial drive mechanism of **Fig. 37****;**
**Fig. 40** is a perspective view of the shuttle of **Fig. 21****,** particularly showing a rotary drive mechanism of the shuttle;
**Fig. 41** is a front view of the shuttle of **Fig. 21****,** particularly showing the rotary drive mechanism;
**Fig. 42** is a bottom view of an upper casing and RHV of the shuttle of **Fig. 21****;**
**Fig. 43** is a bottom view of an upper casing of the shuttle of **Fig. 21****;**
**Fig. 44** is a perspective view of one embodiment of an anti-buckling mechanism of the shuttle of **Fig. 21****;**
**Fig. 45** is another perspective view of the anti-buckling mechanism of **Fig. 44****;**
**Fig. 46** is a top view of the anti-buckling mechanism of **Fig. 44****;**
**Fig. 47** is a profile view of the anti-buckling mechanism of **Fig. 44****;**
**Fig. 48** is a different profile view of the anti-buckling mechanism of **Fig. 44****;**
**Fig. 49** is a top view of the anti-buckling mechanism of **Fig. 44****,** particularly showing an optional take-up biasing mechanism;
**Fig. 50** is a schematic view of anti-buckling mechanism of **Fig. 44****,** particularly showing a matching of the linear speed of an axially split sheath of the anti-buckling mechanism with a linear speed of a take-up reel of the anti-buckling mechanism;
**Fig. 51** is a perspective view of another embodiment of an anti-buckling mechanism of the shuttle of **Fig. 21****;**
**Fig. 52** is another perspective view of the anti-buckling mechanism of **Fig. 51****;**
**Fig. 53** is a profile view of the anti-buckling mechanism of **Fig. 51****;**
**Fig. 54** is a bottom view of the anti-buckling mechanism of **Fig. 51****;**
**Fig. 55** is a top view of the anti-buckling mechanism of **Fig. 51****;**
**Fig. 56** is a schematic diagram of still another embodiment of an endovascular management and tracking system of the robotic endovascular system of **Fig. 1****;**
**Fig. 57** is a top view of an electrically operated version of the endovascular management and tracking system of **Fig. 56****;**
**Fig. 58** is a top view of a manually operated version of the endovascular management and tracking system of **Fig. 56****;**
**Fig. 59** is a cross-sectional view of a control shaft and worm screw of an axial drive mechanism of a shuttle of the endovascular management and tracking system of **Fig. 58****;**
**Fig. 60A** is a plan view of one arrangement of the endovascular management and tracking system of **Fig. 56** in use with a stent delivery catheter and a pusher wire;
**Fig. 60B** is a plan view of the endovascular management and tracking system of **Fig. 60A****,** particularly showing deployment of a stent from the stent delivery catheter;
**Fig. 60C** is a plan view of the endovascular management and tracking system of **Fig. 60A****,** particularly showing resheathing of the stent within the stent delivery catheter;
**Fig. 60D** is a plan view of another arrangement of the endovascular management and tracking system of **Fig. 56****,** particularly showing deployment of a stent from the stent delivery catheter;
**Fig. 60E** is a plan view of still another arrangement of the endovascular management and tracking system of **Fig. 56****,** particularly showing deployment of a stent from the stent delivery catheter;
**Fig. 61A** is a plan view of one arrangement of the endovascular management and tracking system of **Fig. 56** in use with a catheter and a guidewire;
**Fig. 61B** is a plan view of the endovascular management and tracking system of **Fig. 61A****,** particularly showing distal advancement of the catheter and guidewire;
**Fig. 61C** is a plan view of the endovascular management and tracking system of **Fig. 61A****,** particularly showing proximal retraction of the catheter and guidewire;
**Fig. 62A** is a plan view of one arrangement of the endovascular management and tracking system of **Fig. 56** in use with a catheter and a stentreiver;
**Fig. 62B** is a plan view of the endovascular management and tracking system of **Fig. 62A****,** particularly showing proximal retraction of the stentriever, along with a captured thrombus;
**Fig. 62C** is a plan view of the endovascular management and tracking system of **Fig. 61A****,** particularly showing proximal retraction of the catheter and the stentreiver;
**Fig. 63** is a perspective view of still another embodiment of an endovascular management and tracking system of the robotic endovascular system of **Fig. 1****;**
**Fig. 64** is a close-up perspective view of the endovascular management and tracking system of **Fig. 63****;** and
**Fig. 65** is a close-up perspective view of the endovascular management and tracking system of **Fig. 63****,** particularly showing the affixation of a track to a support arm of the endovascular management and tracking system.

### DETAILED DESCRIPTION OF THE ILLUSTRATED EMBODIMENTS

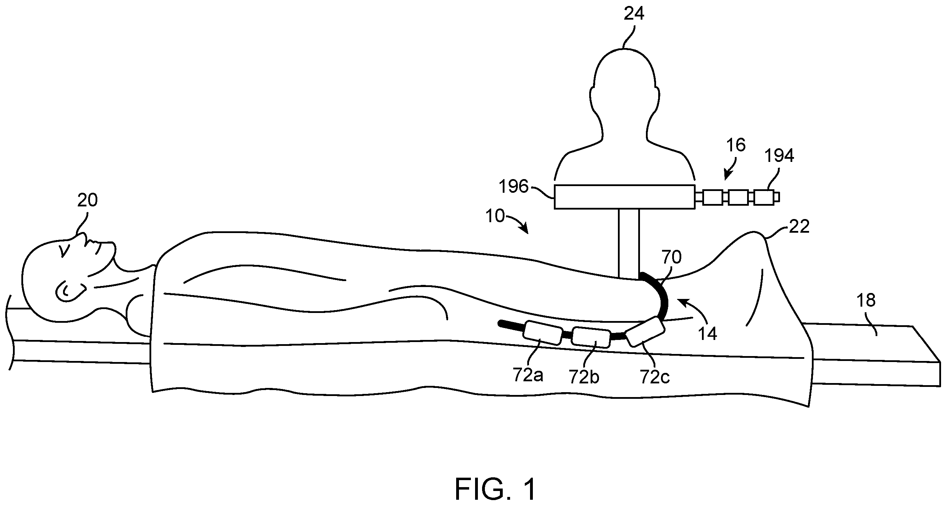

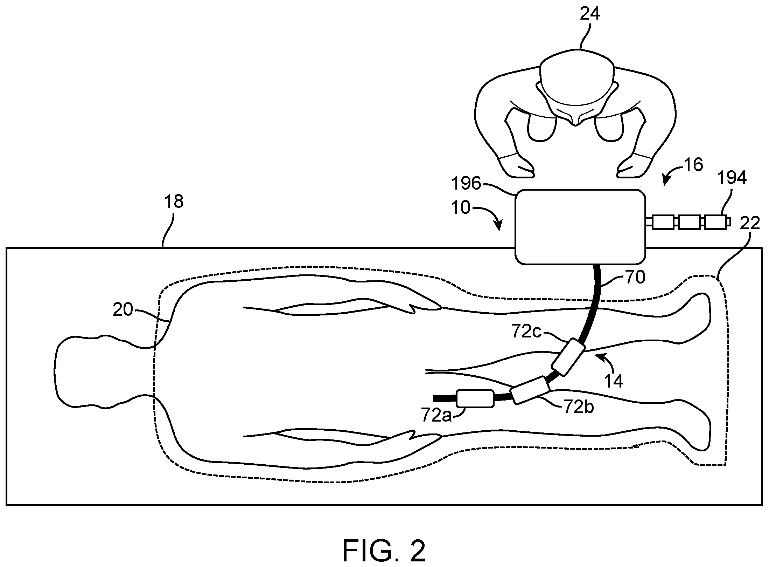

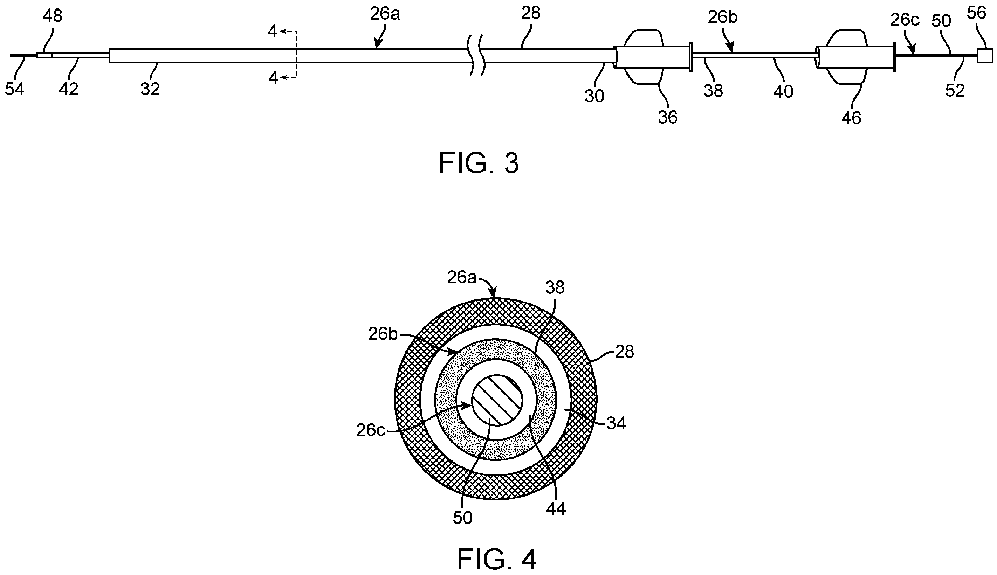

Referring to **Figs. 1-2****,** one embodiment of a robotic endovascular system 10 constructed in accordance with the present disclosure will now be described. The robotic endovascular system 10 generally comprises an endovascular assembly 12 (shown best in **Fig. 3****);** an endovascular management and tracking system 14 that can be closely associated with a procedure table 18 on which a patient 20 (which in the illustrated embodiment, is a human, but can be any animal) rests and to which the endovascular assembly 12 is operably mounted in a coaxial (or nested) relationship; and a control station 16 communicatively coupled to the endovascular management and tracking system 14, thereby allowing an operator 24 (e.g., a physician) to perform a medical procedure on the patient 20. The robotic endovascular system 10 may also comprise a drape 22 (shown in phantom in **Fig. 2****)** that at least partially covers the procedure table 18 and patient 20.

Referring to **Figs. 3-4****,** the endovascular assembly 12 comprises a plurality of elongate medical devices 26, and in this case, a guide sheath 26a, a working catheter 26b, and a guidewire 26c, coaxially arranged with respect to each other; that is, the guidewire 26c is sized to be slidably received within the working catheter 26b, and the working catheter 26b is sized to be slidably received within the guide sheath 26a. The endovascular assembly 12 may be, e.g., inserted through natural body lumens (e.g., blood vessels (artery, chamber of the heart, or vein), urinary system vessels (renal collection ducts, calix, ureter, bladder, or urethra), hepatobiliary vessels (hapatic and pancreatic ducts, chyle ducts, common or cystic duct), gastrointestinal tract (esophagus, stomach, small and large intestine, cecum, rectum) gynecological tract (cervix, uterus), fallopian tube or milk ducts and mammary canals of breast), nasophyarynx (eustacean tube, sinuses, tear duct), seminal vesicle, spinal canal, or ventricles of the brain). The endovascular assembly 12 may be introduced into the patient 20 by percutaneous access, surgical access, or through natural orifices (oral, rectal, nasal, otic, optic, or urethral).

Although the endovascular assembly 12 is described herein as comprising a tri-axial arrangement of a guide sheath, working catheter, and guidewire with which the endovascular management and tracking system 14 interacts, it should be appreciated that the endovascular assembly 12 may include any combination of a guide sheath, working catheter, and guidewire (including a bi-axial arrangement, and even a mono-axial arrangement (i.e., only one of a guide sheath, working catheter, guidewire, or any other flexible elongate medical instrument) with which the endovascular management and tracking system 14 interacts.

The guide sheath 26a is configured for facilitating access for the working catheter 26b to target tissue site in the vasculature of the patient 20. The guide sheath 26a generally includes an elongate sheath body 28 having a proximal end 30 and a distal end 32, a central lumen 34 (shown best in **Fig. 4****)** that extends entirely through the sheath body 28 between the proximal end 30 and distal end 32, and a proximal adapter 36 affixed to the proximal end 30 of the sheath body 28.

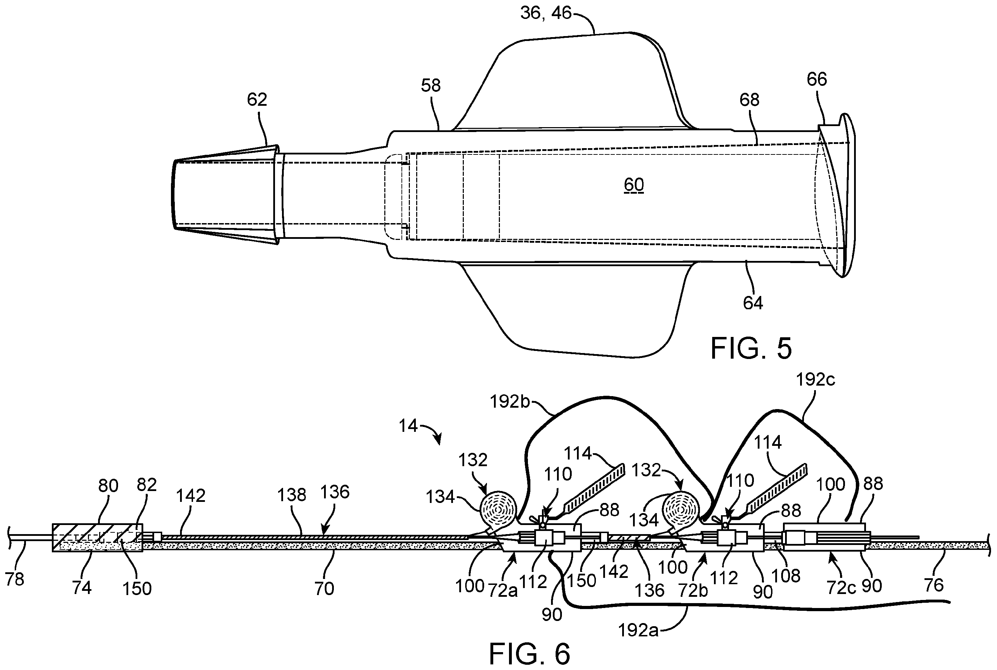

The sheath body 28 is substantially pliable or flexible, such that when it is advanced into the patient 20, an operator or surgeon may easily manipulate the sheath body 28 to conform, adopt, or match the shape or curvatures of the internal pathways (e.g., gastrointestinal tract, blood vessels, etc.) of the patient. In embodiment illustrated in **Fig. 6****,** the sheath body 28 is introduced into the patient 20 via an arterial access sheath 78, the proximal end of which is affixed to the patient 20 via a sheath anchor 80 having a loading/unloading port 82 through which the sheath body 28 may be introduced into or removed from the sheath anchor 80, although an arterial access sheath 78 is not required if the sheath body 28 is introduced into the patient 20 via a natural orifice.

In the illustrated embodiment, the sheath body 28 has a circular cross-section, although other cross-sectional geometries, such as rectangular, can be used. The sheath body 28 may be comprised of multiple layers of materials and/or multiple tube structures that exhibit a low bending stiffness, while providing a high axial stiffness along the neutral axis. Typical designs include a nitinol spine encapsulated in braid and any flexible, pliable, or suitable polymer material or bio-compatible polymer material or a braided plastic composite structure composed of low durometer plastics (e.g., nylon-12, Pebax^{®}, polyurethanes, polyethylenes, etc.).

The geometry and size of the central lumen 34 will be selected in accordance with the cross-sectional geometry and size of the working catheter 26b. The sheath body 28 may have a low-friction inner layer (e.g., a coating of silicone or polytetrafluoroethylene) to provide a low-friction surface to accommodate movement of the working catheter 26b within the working lumen 34 of the guide sheath 26a. As will be described in further detail below, the proximal adapter 36 is configured for both mechanically and fluidly coupling the guide sheath 26a to the endovascular management and tracking system 14.

In an optional embodiment, the guide sheath 26a may have active steering capability, such that the distal end 32 of the sheath body 28 may be articulated into simple or complex shapes or curvatures that may conform to various shapes or curvatures of internal pathways of the patient to reach a target tissue site in the vasculature of the patient 20. For example, the guide sheath 26a may have one or more steering elements (such as pull wires (not shown)), extending through the sheath body 28, which can be operated to effect the desired shape or curvature at the distal end 32 of the sheath body 28.

The working catheter 26b is configured for performing an interventional and/or diagnostic procedure at the target tissue site. For example, the working catheter 26b may be a stent delivery catheter, a balloon catheter, an electrophysiology catheter, ultrasound imaging catheter, atherectomy catheter, vaso-occlusive device delivery catheter, contrast/medicine delivery catheter, etc.

The working catheter 26b generally includes an elongate catheter body 38 having a proximal end 40 and a distal end 42, a central lumen 44 (shown best in **Fig.** 4) that extends entirely through the catheter body 38 between the proximal end 40 and distal end 42, and a proximal adapter 46 affixed to the proximal end 40 of the catheter body 38, and an operative element 48 (e.g., a stent, balloon, mapping electrodes, ultrasound element, tissue cutting blade, vaso-occlusive devices, fluid port, etc.) carried by the distal end 42 of the catheter body 38.

The working catheter 26b passes through the central lumen 34 of the guide sheath 26a, and is thus, moveable relative thereto. The working catheter 26b may be movably positioned within the central lumen 34 of the guide sheath 26a to enable relative insertion of the guide sheath 26a and working catheter 26b, relative rotation or "roll" of the guide sheath 26a and working catheter 26b, and optionally, relative steering or bending of the guide sheath 26a and working catheter 26b relative to each other, particularly when the distal end 42 of the catheter body 38 is inserted beyond the distal end 32 of the sheath body 28. As shown in **Fig. 3****,** the working catheter 26b projects distally with respect to the distal end 32 of the sheath body 28. Of course, the working catheter 26b may be withdrawn proximally such that the distal end 42 of the catheter body 38 is substantially flush with the distal end 32 of the sheath body 28, or withdrawn proximally even further such that the distal end 32 of the sheath body 28 is retracted within the distal end 32 of the sheath body 28.

The catheter body 38 is substantially pliable or flexible, such that it can be advanced through the central lumen 34 of the sheath body 28 when the sheath body 28 conforms to the shape or curvatures of the internal pathways of the patient. In the illustrated embodiment, the catheter body 38 has a circular cross-section, although other cross-sectional geometries, such as rectangular, can be used. The catheter body 38 may be comprised of multiple layers of materials and/or multiple tube structures that exhibit a low bending stiffness, while providing a high axial stiffness along the neutral axis. Typical designs include a nitinol spine encapsulated in braid and any flexible, pliable, or suitable polymer material or bio-compatible polymer material or a braided plastic composite structure composed of low durometer plastics (e.g., nylon-12, Pebax^{®}, polyurethanes, polyethylenes, etc.). The catheter body 38 may be comprised of multiple layers of materials and/or multiple tube structures that exhibit a low bending stiffness, while providing a high axial stiffness along the neutral axis. Typical designs include a nitinol spine encapsulated in braid and any flexible, pliable, or suitable polymer material or bio-compatible polymer material or a braided plastic composite structure composed of low durometer plastics (e.g., nylon-12, Pebax^{®}, polyurethanes, polyethylenes, etc.).

The geometry and size of the central lumen 44 will be selected in accordance with the cross-sectional geometry and size of the guidewire 26c. The catheter body 38 may have a low-friction inner layer (e.g., a coating of silicone or polytetrafluoroethylene) to provide a low-friction surface to accommodate movement of the guidewire 26c within the central lumen 44 of the working catheter 26b. As will be described in further detail below, the proximal adapter 46 is configured for both mechanically and fluidly coupling the working catheter 26b to the endovascular management and tracking system 14.

In an optional embodiment, the working catheter 26b may have active steering capability, such that the distal end 42 of the catheter body 38 may be articulated into simple or complex shapes or curvatures that may conform to various shapes or curvatures of internal pathways of the patient to reach a target tissue site in the vasculature of the patient 20. For example, the working catheter 26b may have one or more steering elements (such as pull wires (not shown)), extending through the catheter body 38, which can be operated to effect the desired shape or curvature at the distal end 42 of the catheter body 38.

The guidewire 26c may be conventional wire that includes a guidewire body 50 having a proximal end 52 and a distal end 54, and a collet 56 affixed to the proximal end 52 of the guidewire body 50. The guidewire body 50 is composed of a suitable material (e.g., metal or metal alloy, such as stainless steel and/or nickel-titanium alloy, or polymer) to provide the guidewire 26c with desirable flexibility/stiffness characteristics. The guidewire body 50 may have a suitable diameter, e.g., 0.3175 cmor less, and have a suitable length in the range of 50cm-350cm. The collet 56 is configured for mechanically coupling the guidewire 26c to the endovascular management and tracking system 14.

Referring now to Fig. 5, each of the proximal adapters 36, 46 comprises an adapter body 58 composed of a suitable material, such as polycarbonate, Acrylonitrile Butadiene Styrene (ABS), or acetal, and a lumen 60 extending axially through the center of the proximal adapter body 58. Each of the proximal adapters 36, 46 further comprises a male connector 62 over which the proximal end 30 of the sheath body 28 or the proximal end 40 of the catheter body 38 is suitably affixed (e.g., via bonding), such that the central lumen 34 of the guide sheath 26a or the central lumen 44 of the working catheter 26b is fluidly coupled to the lumen 60 of the proximal adapter 36, 46. Each of the proximal adapters 36, 46 may further comprise a strain relief (not shown) affixed to the proximal end 30 of the sheath body 28 or the proximal end 40 of the catheter body 38. The strain relief may be composed of a suitable material, such as silicone rubber.

Each of the proximal adapters 36, 46 is designed such that the guide sheath 26a or working catheter 26b may be removably coupled to the endovascular management and tracking system 14, and in this case, to a conventional male Touhy-Borst connector of a rotary hemostasis valve (RHV) (described in further detail below) contained within the endovascular management and tracking system 14. To this end, each of the proximal adapters 36, 46 further comprises a female Luer-lock port 64 formed at the proximal end of the proximal adapter body 58. The Luer-lock port 64 comprises at least one external thread 66 and an internal tapered surface 68. As such, each of the proximal adapters 36, 46 may be alternately screwed to and unscrewed from the Touhy-Borst connector, such that the guide sheath 26a or working catheter 26b may be alternately coupled to and uncoupled from the endovascular management and tracking system 14. Alternatively, each of the proximal adapters 36, 46 may be pushed onto the Touhy-Borst connector of the endovascular management and tracking system 14.

Referring back to **Figs. 1-2****,** the endovascular management and tracking system 14 is a modular system that lays on top of the procedure table 18 in the same area that elongate medical devices are currently handled in a traditional endovascular procedure and uses a flexible track upon which multiple sliding shuttles (or drive units) can be detachably affixed. As will be described in further detail below, the endovascular management and tracking system 14 may provide independent robotic insertion and retraction actuation, robotic rotation or roll actuation, and optional steering actuation, to the elongate medical devices 26 of the coaxial endovascular assembly 12 respectively coupled to the drive units in accordance with control signals transmitted from the control station 16. As will also be described in further detail below, the endovascular management and tracking system 14 can be dynamically switched between a fully automated (robotic) modality that is control of the control station 16 and a fully manual modality that is under manual control of a user without use of the control station 16. In other embodiments, the endovascular management and tracking system 14 can be operated in a fully manual modality in a dedicated manner (i.e., without the use of motors or controllers at all), thereby providing a "dumb" or "budget" version of the endovascular management and tracking system 14. Operation of the endovascular management and tracking system 14 in a fully manual modality is similar to the operating work flow that may be used in current manual catheterization procedures, but additionally provides a more uncluttered work environment, more precise device motions, and improved visibility of the elongate medical devices in use, as compared to current manual catheterization procedures.

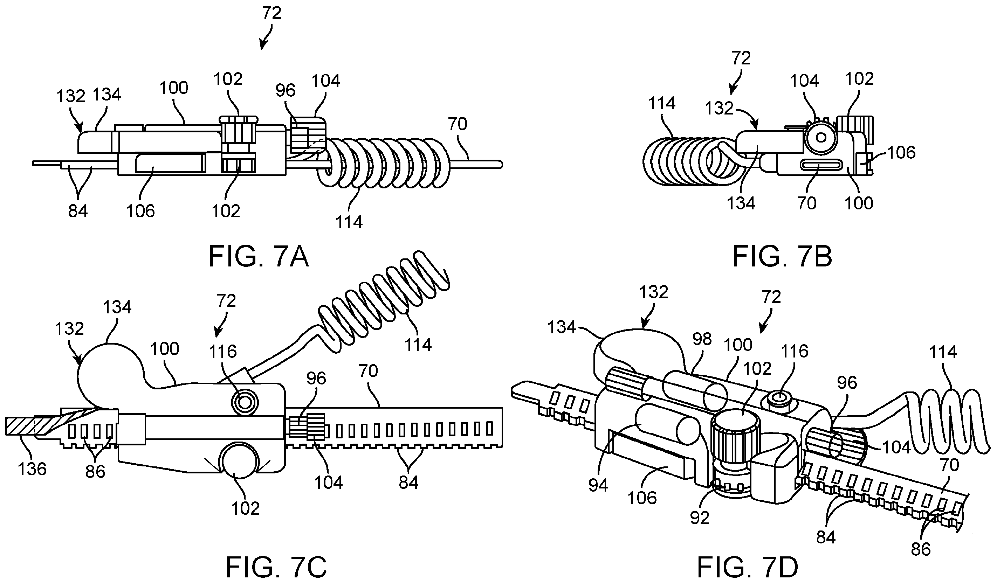

Referring further to **Fig. 6** and **7A****-7D,** the endovascular management and tracking system 14 generally comprises a track 70 and a plurality of shuttles 72 (72a-72c) configured for being mechanically coupled to and axially translated along the track 70.

Preferably, the track 70 is flexible enough, such that it can be contoured by the operator 24 or other hospital personnel to a hospital room environment (e.g., the procedure table 18, the patient 20, and/or the drape 22), yet rigid enough to support axial movement of the shuttles 72a-72c on the track 70. For example, the track 70 may have a relatively high top-to-bottom (vertical) flexibility, such that it conforms to the contours of the hospital room environment solely in response to gravity (e.g., when laid down on the procedure table 18, patient 20, and/or drape 22), but a relatively low side-to-side (horizontal) and torsional flexibility to allow the shuttles 72a-72c to apply the required drive forces to the track 70 in order to axially translate the shuttles 72a-72c along the track 70, as will be described in further detail below, as well as to help maintain the orientation and general position of the track 70 relative to the hospital room environment. For example, the track 70 may be a ribbon-like element (wide and thin rectangular extrusion) with a rectangular cross-section, as best illustrated in **Figs. 7A-7D****.** In one embodiment, the track 70 is malleable, such that the track 70 can be bent into conformance with the hospital room environment. For the purposes of this specification, the term "malleable" with respect to the track 70 means that track 70 can be repeatedly reshaped in response to forces manually exerted on the track 70, which shape is retained when such forces are removed from the track 70. In another embodiment, rather than being a ribbon-like element, the track 70 may take the form of a helical rod or screw to which the shuttles 72a-72c may engage (e.g., using threaded collars (not shown)). In this case, the track 70 would have a circular cross-section, and thus, will have the same top-to-bottom and side-to-side flexibility.

A fastener (e.g., a leg or arm band/strap, adhesive pad, etc.) can be used to affix the distal end 74 of the track 70 at or near the access site of the patient 20, while the proximal end 76 of the track 70, at least momentarily, remains free to allow loading and unloading of the shuttles 72a-72c on and off of the track 70, as will be described in further detail below. In one embodiment, the fastener is the sheath anchor 80 used to affix the proximal end of the sheath body 28 to the patient 20.

In the embodiment illustrated in **Figs. 7A-7D****,** the track 70 has at least one row of teeth 84 (only one row shown) along its length with which corresponding drive mechanisms of the shuttles 72a-72c (e.g., in a rack and pinion-type arrangement as described in further detail below) engage to facilitate axial translation of the shuttles 72a-72c along the track 70. Although the teeth 84 are illustrated as being disposed on the edge of the track 70, the teeth 84 may be located anywhere on the track 84, e.g., on the top surface of the track 70. Alternatively, the track 70 can have other types of elements that facilitate axial translation of the shuttles 72a-72c along the track 70, such as friction surfaces, helical elements, etc. In the illustrated embodiment, the track 70 takes the form of an open monorail on which the shuttles 72a-72c ride, although in alternative embodiments, the track 70 may have multiple rails (e.g., a pair of rails) on which the shuttles 72a-72c ride. In alternative embodiments, the track 70 may take the form of a table-mounted rigid rod, although without the advantages provided by flexible tracks, namely, customizing the shape of the track to the hospital room environment.

Because the robotic endovascular system 10 utilizes multiple independent drive systems in the form of shuttles 72, it is important that the location of any one shuttle 72 on the track 70 is known at any given time, so that the control station 16 can track the relative positioning between the shuttles 72. To this end, the track 70 comprises one or more encoders indicative of a location of the locations of the shuttles 72 relative to the track 70, and one or more sensors configured for reading the encoders and outputting an electrical signal indicating the location of each of the shuttles 72 relative to the track 70. In the illustrated embodiment, the encoder(s) take the form of a series of embedded or printed fiducial elements 86 (shown best in **Figs. 7C-7D****)** along its length, and the sensor(s) take the form of a fiducial element readers (not shown) located on the respective shuttles 72a-72c (e.g., on sleds (described in further detail below) for reading (e.g., optically, magnetically, capacitively, etc.), such that at any point in time, the control station 16 can be informed of the location of each shuttle 72 relative to the track 70. In alternative embodiments, encoder-sensor pairs may be incorporated into the respective shuttles 72 to inform the control station 16 of the locations of the shuttles 72 relative to the track 70. For example, fiducial elements (not shown) can be located on rotating parts of drive mechanisms (described in further detail below) carried by the respective shuttles 72, while the fiducial element sensors may be located on an inside surface of a housing or casing (described in further detail below) or otherwise a stationary frame structure within the respective shuttles 72. In other alternative embodiments, active electronics components (e.g., transmitter-receiver pairs) capable of performing location sensing using signaling (e.g., optical, ultrasonic, inductive, capacitive, radio frequency (RF), etc.) can be embedded into the track 70 and shuttles 72 to inform the control station 16 of the locations of the shuttles 72 relative to the track 70. In still other alternative embodiments, fiducial elements (not shown) can be disposed on the distal ends of the elongated medical devices 26, which may be visually sensed by a vision-based system, such as a fluoroscopic imaging device (not shown) to directly track the locations of the elongated medical devices 26 rather than directly tracking the locations of the shuttles 72 relative to the track 70 as a surrogate to the locations of the elongated medical devices 26.

As will be described in further detail below, the shuttles 72a-72c provide axial tracking of the elongate medical devices 26 relative to each other, rotational movement of the elongate medical devices relative to each other, and internal or external power and communications in a singular unit. In contrast to the shuttle 72c to which the guidewire 26c is affixed, which does not require fluid management capabilities, the shuttles 72a-72b also respectively provide fluid management capabilities for the guide sheath 26a and working catheter 26b.

The shuttles 72a-72c are configured for being mechanically coupled to the track 70 in a detachable manner. In particular, the shuttles 72a-72c may, one-by-one, be threaded onto the proximal end 76 of the track 70 on which they are driven, and, one-by-one, removed from the proximal end 76 of the track 70. The shuttles 72a-72c are configured for being respectively affixed to the elongate medical devices 26 in a detachable manner. In the illustrated embodiment, the guide sheath 26a is affixed to the distal-most shuttle 72a, the working catheter 26b is affixed to the medial shuttle 72b, and the guidewire 26c is affixed to the proximal-most shuttle 72c. Each subsequent one of the shuttles 72a-72b is configured for slidably receiving a distal end of a respective one of the elongate medical devices 26 affixed to a preceding one of the shuttles 72b-72c via a loading/unloading port 88, such that the guide sheath 26a, working catheter 26b, and guidewire 26c are in a coaxial arrangement (i.e., the distal-most shuttle 72a is configured for slidably receiving a distal end of the working catheter 26b, such that the working catheter 26b is coaxially disposed within the guide sheath 26a; and the medial shuttle 72b is configured for receiving a distal end of the guidewire 26c, such that the guidewire 26c is coaxially disposed within the working catheter 26b). As will be described in further detail below, the shuttles 72a, 72b are interchangeable, such that the shuttle 72a may be the medial shuttle, and the shuttle 72b may be the distal-most shuttle.

Each of the shuttles 72a-72c includes a sled 90 that is configured for riding on the track 70; an axial drive mechanism 92 (e.g., a geared or frictional coupling) (shown in **Figs. 7A** and **7D****)** carried by the sled 90 and configured for being actuated to axially translate the respective shuttle 72 (and thus, the respective one of the elongate medical devices 26 affixed to the respective shuttle 72) along the track 70; an axial actuator 94 (shown in **Fig. 7D****)** carried by the sled 90 and configured for actuating the axial drive mechanism 92; a rotary drive mechanism 96 (e.g., a geared or frictional coupling) carried by the sled 90 and configured for being actuated to rotate the respective one of the elongate medical devices 26 affixed to the respective shuttle 72 about its longitudinal axis; a rotary actuator 98 (shown in **Fig. 7D****)** carried by the sled 90 and configured for actuating the rotary drive mechanism 96; and an outer casing 100 mounted to the sled 90 and containing the axial drive mechanism 92, axial actuator 94, rotary drive mechanism 96, and rotary actuator 98.

In the illustrated embodiment, wherein the control station 16 operates the endovascular management and tracking system 14 in a master-slave arrangement (i.e., fully automated modality), the axial actuator 94 and rotary actuator 98 may comprise motors (e.g., stepper motors), such that the motors may, in response to control signals generated by the control station 16, actuate the respective drive mechanisms 92, 96 to axially translate the respective shuttle 72 along the track 70 and rotate the respective one of the elongate medical devices 26 about its longitudinal axis.

As briefly discussed above, the endovascular management and tracking system 14 may be advantageously designed to be dynamically switched between the fully automated modality and a fully manual modality.

To this end, each of the shuttles 72a-72c additionally comprises manual actuators 102, 104 (e.g., hand-operated dials, knobs, thumb wheels, sliders, or other hand-actuated mechanism) that can be manually operated to axially index the respective shuttle 72, such that axial movement of the respective shuttle 72 along the track 70 may be finely controlled, and/or rotationally index the respective one of the elongate medical devices 26), such that rotation of the respective elongate medical device 26 about its axis may be finely controlled. To axially index the respective shuttle 72 or rotationally index the respective elongate medical device 26, the manual actuators 102, 104 may respectively actuate the same drive mechanisms 92, 96 that are respectively actuated by the motors 94, 98, or may respectively actuate different drive mechanisms (not shown) that are completely independent of the drive mechanisms 92, 96 that are respectively actuated by the motors 94, 98.

Alternatively, any of the shuttles 72a-72c may not have manual actuators 102, 104, and instead, the respective shuttle 72 may be manually translated along the track 70 directly by hand and/or the respective one of the elongate medical devices 26 may be rotated about its longitudinal axis directly by hand). In an optional embodiment, each of the shuttles 72a-72c includes one or more actuators (not shown) (e.g., one or more buttons, a slider, touch sensor(s), etc. that can be manually operated to axially translate the respective shuttle 72 along the track 70 or rotate the respective one of the elongate medical devices 26 its longitudinal axis via operation of the respective motors 94, 98 and associated drive mechanisms 92, 96.

Switching the endovascular management and tracking system 14 between the fully automated modality and the fully manual modality may be accomplished in any one or more of a variety of manners.

As one example, each shuttle 72 includes a first clutching mechanism (not shown) configured for being operated to alternately engage (e.g., via meshing of gears, frictional coupling, motor activation, etc.) and disengage (e.g., de-meshing of gears, frictional decoupling, motor deactivation, etc.) at least a portion of the axial drive mechanism 92 to which the axial motor 94 is associated to and from the track 70, and a second clutching mechanism (not shown) configured for being operated to alternately engage (e.g., meshing of gears, frictional coupling, motor activation, etc.) and disengage (e.g., de-meshing of gears, frictional decoupling, motor deactivation, etc.) at least a portion of the rotary drive mechanism 96 to which the axial motor 94 is associated to and from the respective one of the elongate medical devices 26 affixed to the respective shuttle 72.

Thus, operating the clutching mechanisms to engage the entirety of the axial drive mechanism 92 to the track 70 and engage the entirety of the rotary drive mechanism 96 to the respective one of the elongate medical devices 26 places the endovascular management and tracking system 14 into the fully automated modality (such that the motors 94, 98 may, in response to control signals generated by the control station 16, actuate the respective drive mechanisms 92, 96 to axially translate the respective shuttle 72 along the track 70 and rotate the elongate medical device 26 about its longitudinal axis); whereas, operating the clutching mechanisms to disengage at least a portion of the axial drive mechanism 92 from the track 70 and disengage at least a portion of the rotary drive mechanism 96 from the respective elongate medical device 26 places the endovascular management and tracking system 14 into the (such that the manual actuators 102, 104 can be operated to finely control axial translation of the respective shuttle 72 along the track 70 and/or finely rotate the respective elongate medical device 26 about its longitudinal axis; or the respective shuttle 72 may be manually translated along the track 70 directly by hand and/or the respective elongate medical device 26 may be rotated about its longitudinal axis directly by hand).

The clutching mechanisms for each shuttle 72 may be operated via manual input via the respective shuttle 72, via another shuttle 72, and/or via input from the control station 16, and may optionally include a manual/auto switch with visual indication of engagement or disengagement.

When the endovascular management and tracking system 14 is operated in the fully manual modality, the manual actuator 102 may actuate the portion of the axial drive mechanism 92 that remains engaged with the track 70 (i.e., the portion of the axial drive mechanism 92 between the clutch point and the track 70) or a completely independent axial drive mechanism to finely control axial translation of the respective shuttle 72 along the track 70, and the manual actuator 104 may actuate the portion of the rotary drive mechanism 96 that remains engaged with the respective one of the elongate medical devices 26 ((i.e., the portion of the rotary drive mechanism 96 between the clutch point and the respective elongate medical device 26) or a completely independent axial drive mechanism to finely control rotation of the respective elongate medical device 26.

As another example, the motors 94, 98 and associated drive mechanisms 92, 96 may be designed to freely spin when electronically deactivated (e.g., powered down or otherwise placed in sleep mode) in response to a low force. The free spin capability of the motors 94, 98 and associated drive mechanisms 92, 96 will depend on the type of motors used, the mode of controlling the motors, and the design of the associated drive mechanisms 92, 96. Thus, electronically activating (e.g., powering on or otherwise waking up) the motors 94, 98 places the endovascular management and tracking system 14 into the fully automated modality (such that the motors 94, 98 may, in response to control signals generated by the control station 18, actuate the respective drive mechanisms 92, 96 to axially translate the respective shuttle 72 along the track 70 and rotate the respective one of the elongate medical devices 26 about its longitudinal axis), and powering down or otherwise putting the motors 94, 98 to sleep places the endovascular management and tracking system 14 into the fully manual modality (such that the manual actuators 102, 104 can be operated to finely control axial translation of the respective shuttle 72 along the track 70 and/or finely rotate the respective elongate medical device 26 about its longitudinal axis; or the respective shuttle 72 may be manually translated along the track 70 directly by hand (e.g., by grasping the outer casing 100 with a hand) and/or the respective elongate medical device 26 may be rotated about its longitudinal axis directly by hand (e.g., by rotating the proximal adapter 36 of the guide sheath 26a, the proximal adapter 46 of the working catheter 26b, or the collet 56 of the guidewire 26c).

It should be appreciated that if the endovascular management and tracking system 14 is designed to be operated in a fully manual modality in a dedicated manner (i.e., without motors), the respective shuttle 72 may comprise no actuators (e.g., the shuttle 72 may be manually translated along the track 70 directly by hand and/or the respective one of the elongate medical devices 26 may be rotated about its longitudinal axis directly by hand); or the axial actuator 94 and/or rotary actuator 98 may, instead of motors, comprise manual actuators (e.g., hand-operated dials, knobs, thumb wheel or other hand-actuated mechanism) that can be operated to axially index the shuttle 72, such that axial movement of the shuttle 72 along the track 70 may be finely controlled, and/or rotationally index the respective elongate medical device 26), such that rotation of the respective elongate medical devices 26 about their axes may be finely controlled.

In an optional embodiment, each of the shuttles 72a-72c may have a locking mechanism 106 (shown in **Figs. 7A** and **7D****)** for parking or locking the respective shuttle 72 in place on the track 70 to prevent un-commanded movement of the respective shuttle 72 on the track 70 when the shuttle 72 is either in the fully automated modality (in the case, where the motors are incapable of locking the shuttle 72 in place on the track via the first clutching mechanism) or the fully manual modality. In another optional embodiment, each of the shuttles 72a-72c comprises embedded or printed fiducial elements (not shown) around a portion of the rotary drive mechanism 96 that directly engages the respective one of the elongate medical devices 26, which can be read by optical or magnetic means, such that at any point in time, the control station 16 can be informed of the rotational positions of the elongate medical devices 26.

The proximal-most shuttle 72c to which the guidewire 26c is affixed may be in close proximity to, and in the illustrated embodiment, may be hard coupled to the medial shuttle 72b, such that the shuttles 72b-72c axially translate along the track 70 as a single unit. For example, the proximal-most shuttle 72c comprises a male plug 108 (shown in **Fig. 6****)** disposed on the proximal end of the outer casing 100 for removably mating to the loading/unloading port 88 of the medial shuttle 72b. In an alternative embodiment, rather than using a shuttle to axially translate the guidewire 26c along the track 70, an alternative conventional drive mechanism (e.g., pinch rollers or caterpillar tractor) may be used to axially translate the guidewire 26c along the track 70.

Each of the shuttles 72a-72b has an on-board fluid management control assembly 110 carried by the sled 90 within the outer casing 100 that is fluidly coupled to the respective one of the guide sheath 26a and the working catheter 26b, and in particular, allows for connections between fluid sources (e.g., saline and contrast) and the guide sheath 26a and the working catheter 26b affixed to the respective shuttle 72a-72b in a controlled manner. It is noted that the shuttle 72c to which the guidewire 26c is affixed need not have fluid management capability, and thus, the shuttle 72c does not have a fluid management control assembly.

In the illustrated embodiment, the on-board fluid management control assembly 110 of each of the shuttles 72a-72b comprises a rotatable hemostasis valve (RHV) 112, a flush line 114 in fluid communication with the RHV 112, and an on-board contrast injection port 116 affixed to the outer casing 100 in fluid communication with the RHV 112. The RHV 112 may be permanently integrated within the respective one of the shuttles 72a-72b, but preferably, the respective one of the shuttles 72a-72b removably receives the RHV 112. In this manner, the RHV 112 may be discarded after use and replaced with a new RHV 112, such that the shuttles 72a-72b may be reused.

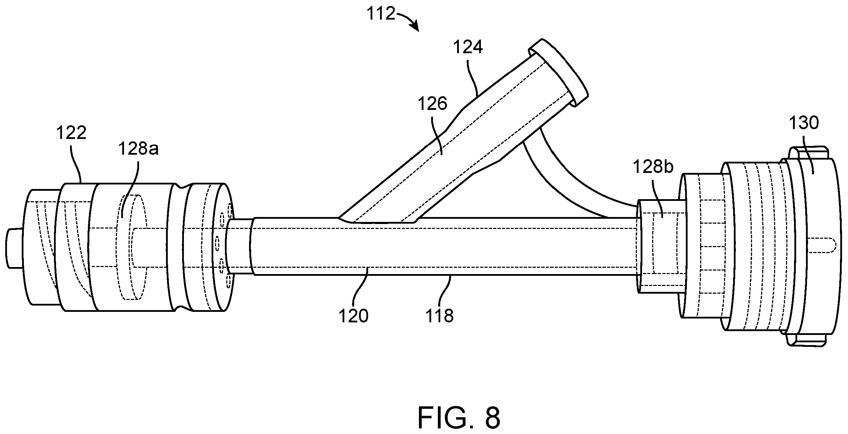

Referring to **Fig. 8****,** the RHV 112 comprises a cylindrical tube 118 having a central lumen 120, a conventional male Touhy-Borst connector 122 rotatably affixed to the distal end of the cylindrical tube 118, and a side arm 124 affixed to the cylindrical tube 118 and having a side lumen 126 in fluid communication with the central lumen 120 of the cylindrical tube 118. The flush line 114 is affixed to the side arm 124 in fluid communication with the side lumen 126 of the side arm 124, and the on-board contrast injection port 116 is in fluid communication with the central lumen 120 of the cylindrical tube 118.

The Touhy-Borst connector 122 may allow hand rotation and/or engage a rotary mechanism (as a portion of the rotary drive mechanism 96 described in further detail below) that can be manually actuated or remotely driven via a motor or manual actuator. The proximal adapter 36 of the guide sheath 26a or the proximal adapter 46 of the working catheter 26b (shown in **Fig. 3****)** is configured for being coupled to the Touhy-Borst connector 122 of the respective shuttle 72a or shuttle 72b, such that the central lumen 120 of the cylindrical tube 118, and thus, the side lumen 126 of the side arm 124, is in fluid communication with the lumen 60 of the proximal adapter 36 or the lumen 60 of the proximal adapter 46, and thus, the central lumen 34 of the guide sheath 26a or the central lumen 44 of the working catheter 26b. Because the Touhy-Borst connector 122 is rotatable relative to the cylindrical tube 118, the guide sheath 26a or working catheter 26b may be rotated about their longitudinal axes without rotating the cylindrical tube 118 and side arm 124.

The central lumen 120 of the cylindrical tube 118 is sized to receive the working catheter 26b via the loading/unloading port 88 formed in the proximal end of the outer casing 100 (if the RHV 112 is associated with the shuttle 72a to which the guide sheath 26a is affixed) or the guidewire 26c (if the RHV 112 is associated with the shuttle 72b to which the working catheter 26b is affixed). The RHV 112 further includes a proximal seal 128a and distal seal 128b disposed within the central lumen 120 of the cylindrical tube 118 for sealing fluid flow between the outer surface working catheter 26b or guidewire 24 and the central lumen 120 of the cylindrical tube 118. The RHV 112 further comprises a compression nut 130 configured for being rotated to compress the distal seal 128b against the outer surface of the working catheter 26b or guidewire 26c.

All of the fluid inputs may be integrated into each of the shuttles 72a-72b or exist as separate replacement elements that can be loaded into the shuttles 72a-72b. In the illustrated embodiment, the flush line 114 is hard mounted to the outer casing 100 in fluid communication with the side lumen 126 of the side arm 124, and has a female Luer-lock port (not shown) for connection to a saline bag (not shown). In an alternative embodiment, a female Luer-lock port (not shown) can be hard mounted to the outer casing 100 in fluid communication with the side lumen 126 of the side arm 124, in which case a flush line with a male Luer fitting may fluidly couple a saline bag to the female Luer-lock port. The female Luer-lock port may have a built-in septum to allow the flow of saline only when the male Luer-lock fitting to which the flush line is connected is coupled to the female Luer-lock port.

In the illustrated embodiment, the contrast injection port 116 takes the form of a female Luer-lock port hard mounted to the casing 100 in fluid communication with the central lumen 120 of the cylindrical tube 118. Tubing (not shown) with a male Luer fitting may fluidly couple a source of contrast to the female Luer-lock port. In an alternative embodiment, tubing is hard mounted to the outer casing 100 in fluid communication with the central lumen 120 of the cylindrical tube 118, and has a female Luer-lock port (not shown) for connection to the source of contrast. The female Luer-lock port may have a built-in septum to allow the flow of contrast only when the male Luer-lock fitting to which tubing is connected is coupled to the female Luer-lock port. In an optional embodiment, an automated injector configured for delivering contrast bolus can be connected to the contrast injection port 116 to allow contrast injection controlled from a remote location, and in particular, the control station 16.

A valve system 130 (not shown) for managing the coupling of saline and contrast to the guide sheath 26a or working catheter 26b can be used. Such valve system 130 can connect any two of the three channels (flush line 114, fluid injection port 124, and central lumen 120 of the cylindrical tube 118). The valve system may be constructed using a variety of different valve types (e.g., stop-cocks, ball valves, pinch valves, needle valves, diaphragm valves, solenoid valves, etc.), but generally would be manually operated with a physical lever, switch, push-button selector, thumb roller, etc. Alternatively, the valve system 130 may be automatically operated at the control station 16.

In an alternative embodiment, instead of a discrete valve system, the flush line 114 has a one-way valve to prevent back flow. Thus, normal saline flow always occurs through the flush line into the RHV 112 unless fluid is injected through the injection port 124, in which case, the one-way valve in the flush line 114 prevents saline flow and only infusion of the contrast into the RHV 112.

The RHV 112 and any valving system may be permanently incorporated into each of the shuttles 72a-72b or may be designed as a separate replacement element, e.g., into a single replaceable module, such that the shuttles 72a-72b can be cleaned and reused from procedure-to-procedure, while the necessarily sterile elements and fluid connections can be replaced for each procedure. If re-use of the shuttles 72a-72b is desired, the design may be specifically configured for ease of either in-hospital cleaning or return to a dedicated reprocessing facility, to clean, reload replaceable elements (fluid handling components, batteries, motors, gearing, etc.), retest for function, repackage, resterilize.

It should be appreciated that, because the axial drive mechanism 92 and RHV 112 are located in the same outer casing 100, the unusable lengthens of the guide sheath 26a or working catheter 26b (i.e., any length that cannot be inserted into the patient 20) is minimized. For example, the unusable length of the guide sheath 26a may be equal to the length of the sheath anchor 80 to which the arterial access sheath 78 is affixed, while the unusable length of the working catheter 26b may be equal to the sum of the length of the sheath anchor 80 to which the arterial access sheath 78 is affixed, and the length of the outer casing 100 (about the length of the RHV 112 contained within that outer casing 100) to which the guide sheath 26a is affixed and through which the working catheter 26b is slidably and rotatably disposed via the central lumen 120 of the cylindrical tube 118 of that RHV 112.

Although the rotary drive mechanism 96 associated with each of the shuttles 72a-72b may directly engage the guide sheath 26a or working catheter 26b, in one embodiment, the rotary drive mechanism 96 indirectly engages the guide sheath 26a or working catheter 26b via the Touhy-Borst connector 122 of the RHV 112. In other embodiments, the entirety of the rotary drive mechanism 96, as well as the rotary actuator 98, are incorporated into the RHV 112. The rotary drive mechanism 96 associated with the proximal-most shuttle 72c may directly engage the guidewire 26c via the collet 56 affixed to the proximal end 52 of the guidewire body 50.

Although the rotary drive mechanism 96 associated with each of the shuttles 72a-72c are designed to rotating the guide sheath 26a, working catheter 26b, and guide wire 26c about their longitudinal axes relative to the respective sled 90, in alternative embodiments, each of the shuttles 72a-72c may comprise an on-board rotary drive mechanism that rotates the entire respective shuttle 72, and thus, the respective one of the guide sheath 26a, working catheter 26b, and guide wire 26c affixed to the respective shuttle 72, about their longitudinal axes relative to the track 70. However, in this alternative case, the track 70 must be arranged relative to the hospital environment, such that the respective shuttles 72a-72c may freely rotate about the track 70 without impingement by the hospital environment.

In another alternative embodiment, rather than being incorporated into each of the shuttles 72a-72c, a rotary drive mechanism and rotary actuator may be external to one or more of the shuttles 72a-72c, in which case, the guide sheath 26a, working catheter 26b, and/or guidewire 26c may be fixed to the shuttles 72a-72c, such that they do not rotate relative to the shuttles 72a-72c, but instead, the rotary drive mechanism may rotate the entire shuttle 72 in order to rotate the guide sheath 26a, working catheter 26b, and/or guidewire 26c.

The sled 90 and outer casing 100 are preferably shaped in a manner that prevents the respective shuttle 72 from being caught or entangled with the hospital room environment, and in particular, the drape 22 upon which the track 70 will likely lay. Because the drape 22 will be lying on top of the procedure table 18 and patient 20, the drape 22 will not always be flat. Thus, the drape 22 may be folded or bunched, so that when a shuttle 72 is axially translated along the track 70, it could get caught in the drape 22 if the shape of the shuttle 72 has flat faces or sharp edges, thereby causing interference motion and/or an undesirable amount of force being placed on the track 70. If this occurs, the track 70 could be pulled out of the sheath anchor 80, or worse yet, pull the arterial access sheath 78 out of the patient 20. As such, it is preferable that the sled 90 and outer casing 100 have smooth edges, such that the drape 22 deflects off, instead of catching on, the sled 90 and outer casing 100.

In contrast to the proximal-most shuttle 72c to which the guidewire 26c is affixed, which generally will not have an exposed length between the proximal-most shuttle 72c and medial shuttle 72b that is great enough to prolapse in response to an axially compressive force (and in fact, may have no exposed length, since the proximal-most shuttle 72c may be plugged into the medial shuttle 72b via mating of the plug 108 and loading/unloading port 88), the exposed length of the guide sheath 26a between the distal-most shuttle 72a and the sheath anchor 80 at any given time or the exposed length of the working catheter 26b between the medial shuttle 72b and the distal-most shuttle 72a at any given time, may be great enough that the guide sheath 26a or working catheter 26b prolapse in response to an axially compressive force.

Thus, each of the shuttles 72a-72b includes an anti-buckling mechanism 132 carried by the sled 90 and configured for preventing the respective one of the guide sheath 26a and working catheter 26b from prolapsing in response to axial compression applied to the respective one of the guide sheath 26a and working catheter 26b (e.g., preventing the guide sheath 26a and working catheter 26b from prolapsing when the respective shuttles 72a-72b are axially translated in the distal direction or preventing the working catheter 26b from prolapsing when the shuttle 72a is axially translated in the proximal direction). The anti-buckling mechanism 132 may be hard fixed or detachably fixed to the sled 90 or outer casing 100 of the respective shuttle 72a-72b. Significantly, in contrast to fixed length supports for preventing guide sheaths or working catheters from prolapsing, the anti-buckling mechanism 132 provides variable length support for the guide sheath 26a or working catheter 26b attached to the respective shuttle 72a-72b to which the anti-buckling mechanism 132 is affixed, which may minimize unused length of the guide sheath 26a or working catheter 26b and avoid catheter stack-up issues.

In the illustrated embodiment, the anti-buckling mechanism 132 comprises a stowage housing 134 and an axially split sheath 136 configured for varying its linear dimension by being alternately furled (rolled up) within the stowage housing 134 and unfurled (rolled out) from the stowage housing 134 to prevent the guide sheath 26a or working catheter 26b from buckling under a compressive load. The stowage housing 134 is configured for storing the axially split sheath 136 to prevent tangling or interference with other elements of the endovascular management and tracking system 14 or the hospital room environment.

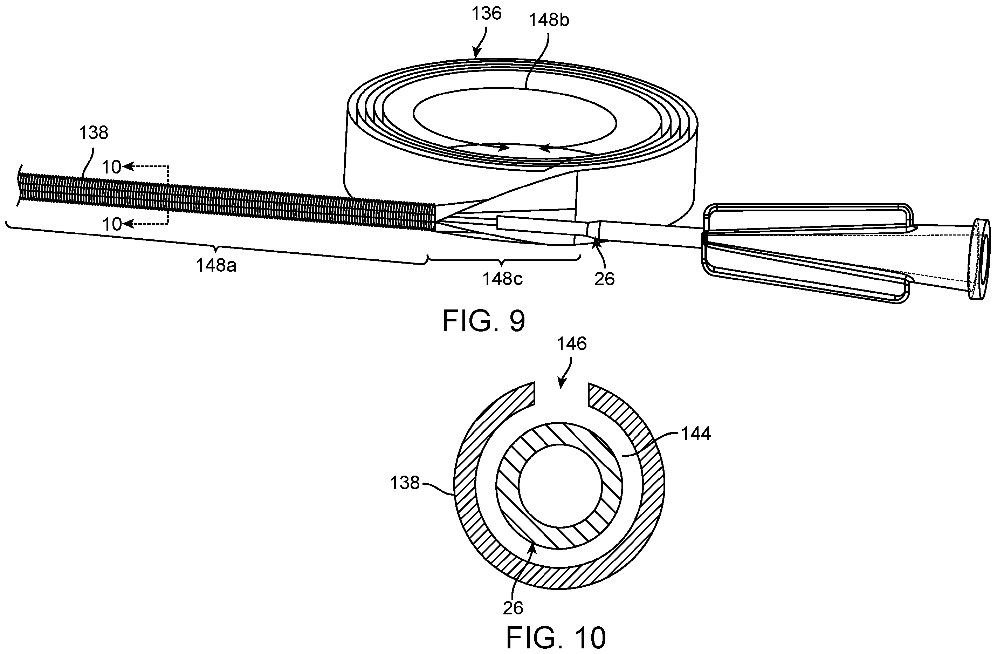

Referring further to **Figs. 9-10****,** the axially split sheath 136 comprises an elongate tubular body 138 having a proximal end (not shown) and a distal end 142 (shown in **Fig. 6****),** a central lumen 144 extending between the proximal end and distal end 142 of the tubular body 138, and a split 146 axially extending between the proximal end 140 and distal end 142 of the tubular body 138.

The axially split sheath 136 is capable of transitioning between a tubular state and a flattened state. Thus, at any given time, a distal portion 148a of the axially split sheath 136 will be in the tubular state, and a proximal portion 148b of the axially split sheath 136 will be in the flattened state. The axially split sheath 136 will also have a transition portion 148c between the tubular distal portion 148a and the flattened proximal portion 148b, which is neither fully in the tubular state nor fully in the flattened state. It should be appreciated that, because the length of the axially split sheath 136 is constant, the tubular distal portion 148a lengthens while the flattened proximal portion 148b shortens as the axially split sheath 136 is unfurled from the stowage housing 134, and shortens while the flattened proximal portion 148b lengthens as the axially split sheath 136 is furled within the stowage housing 134.

In one embodiment, the axially split sheath 136 is a composed of an elastomeric or shape recoverable material that is pre-shaped in the tubular state when unfurled from the stowage housing 134, but assumes a flattened state when furled within the stowage housing 134. As examples, the structure of the axial split sheath 136 may be a simple elastomeric tube, a wire/braid reinforced tube (especially those having a wire with a shape recovery to a cylindrical shape), a laser cut tube (especially those using stiffer materials ranging from stiff polymers, polyether-ether ketone (PEEK), PI, thick polytetrafluoroethylene (PTFE), etc., to metals with yield strains greater than 1%, such as spring steel, nitinol, etc.). In this manner, the flattened proximal portion 148b only assumes the flattened state in the presence of an external force, such as that applied by a flattening mechanism described in further detail below.

In another embodiment, the axially split sheath 136 may be pre-shaped to have both a tubular state and flattened state, such that the axially split sheath 136 has a bi-stable structure. The axially split sheath 136 may be composed of a suitable material, e.g., a carbon fiber composite laminated or impregnated with a polymer, that can be pre-shaped to have a bi-stable structure. Thus, the axially split sheath 136 will remain in a tubular shape until an application of an external force transitions the axially split sheath 136 to the flattened state, and likewise, the axially split sheath will remain in a flattened state until an application of an external force transitions the axially split sheath 136 from the flattened state to the tubular state. In this manner, the flattened proximal portion 148b of the axially split sheath 136 will not tend to transition to the tubular state, and will thus, fully remain in the flattened state. Thus, the axially split sheath 136 may be more tightly rolled up within the stowage housing 134 into a more compact form merely by pushing the axially split sheath 136 toward and within the stowage housing 134. In this manner, the axially split sheath 136 may be more consistently unfurled from and furled within the stowage housing 134 without the need for a motorized take-up mechanism that otherwise tension the axially split sheath 136, such that it remains in the flattened state when being furled.

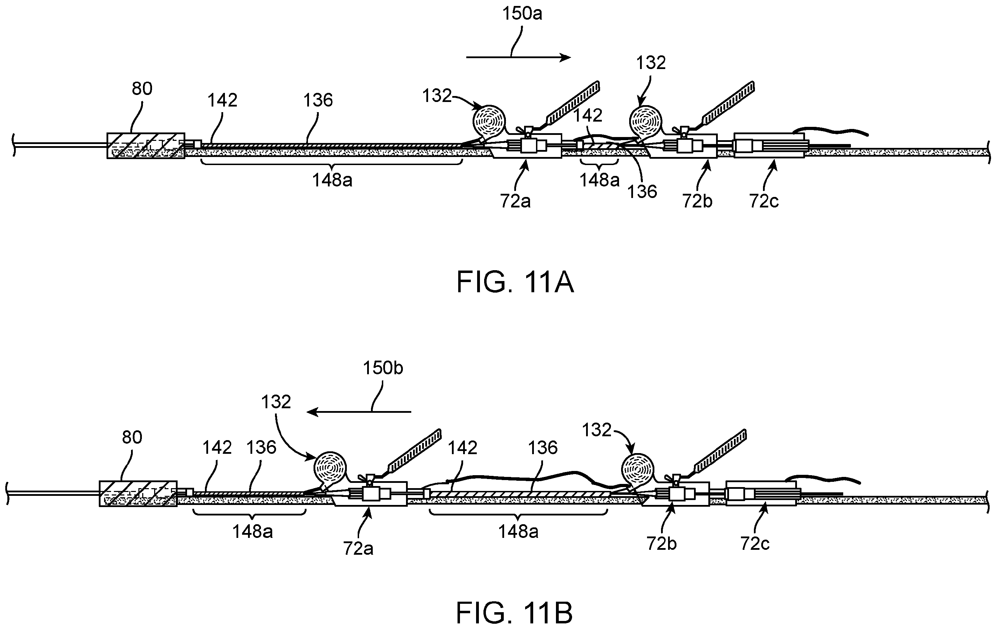

Referring to **Fig. 6** and **11A****-11D,** the distal end 142 of the tubular body 138 is affixed to a location, such that the tubular distal portion 148a of the axially split sheath 136 lengthens when the distance between the respective shuttle 72 and the location increases (i.e., the respective shuttle 72 is axially translated away from the affixation location or the affixation location is axially translated away from the respective shuttle 72), and shortens when the distance between the respective shuttle 72 and the location decreases (e.g., the respective shuttle 72 is axially translated toward the affixation location or the affixation location is axially translated toward the respective shuttle 72).

In the illustrated embodiment, the location to which the distal end 142 of the tubular body 138 associated with the distal-most shuttle 72a is affixed is a location relative to the patient 20, e.g., to the sheath anchor 80 used to affix the distal end 74 of the track 70 at or near the access site of the patient 20, and the location to which the distal end 142 of the tubular body 138 associated with the medial shuttle 72b (the "preceding shuttle") is affixed is the distal-most shuttle 72a (the "subsequent shuttle"). To this end, the anti-buckling mechanism 132 further comprises a male connector 150 (e.g., a clip or a plug) affixed to the distal end 142 of the tubular body 138 for removably mating to the loading/unloading port 82 disposed within the proximal end of the sheath anchor 80, thereby providing a firm attachment point to the sheath anchor 80 (in the case where the anti-buckling mechanism 132 provides support for the guide sheath 26a affixed to the distal-most shuttle 72a) or for removably coupling mating to the loading/unloading port 88 formed in the proximal end of the outer casing 100 of the distal-most shuttle 72a, thereby providing a firm attachment point to the distal-most shuttle 72a (in the case where the anti-buckling mechanism 132 provides support for the working catheter 26b affixed to the medial shuttle 72b). Not only does the anti-buckling mechanism 132 provide an affixation means for the distal end 142 of the tubular body 138, the anti-buckling mechanism 132 of the distal-most shuttle 72a positions the guide sheath 26a for smooth entry into the loading/unloading port 82 of the sheath anchor 80, and the anti-buckling mechanism 132 of the medial shuttle 72b positions the working catheter 26b for smooth entry into the loading/unloading port 88 of the distal-most shuttle 72a.

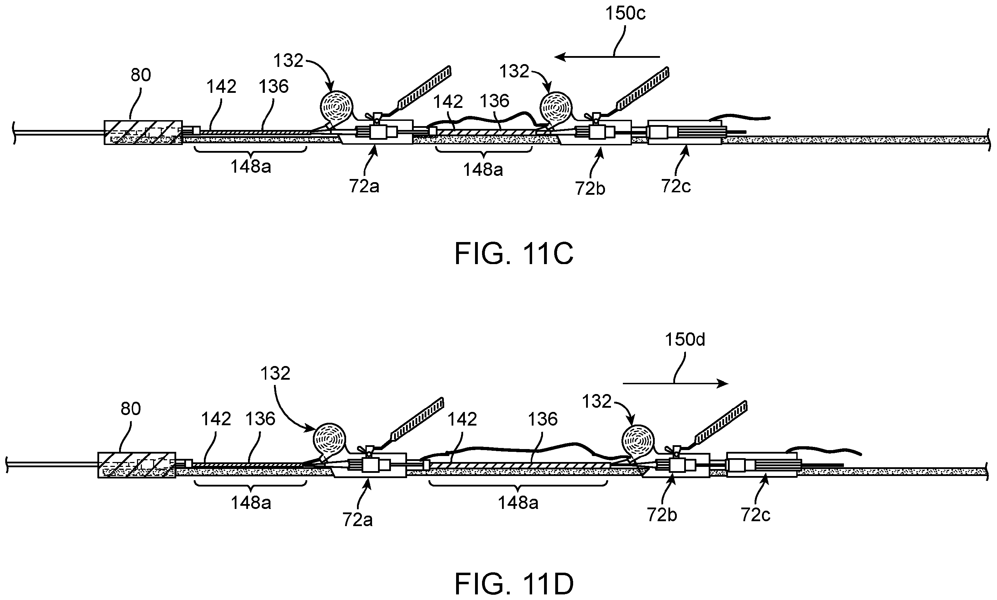

Thus, when the distal-most shuttle 72a is axially translated in the proximal direction 150a and toward the medial shuttle 72b, the tubular distal portion 148a of the axially split sheath 136 associated with the distal-most shuttle 72a lengthens and the tubular distal portion 148a of the axially split sheath 136 associated with the medial shuttle 72b shortens (see **Fig. 11A****).** In contrast, when the distal-most shuttle 72a is axially translated in the distal direction 150b and away from the medial shuttle 72b, the tubular distal portion 148a of the axially split sheath 136 associated with the distal-most shuttle 72a shortens and the tubular distal portion 148a of the axially split sheath 136 associated with the medial shuttle 72b lengthens (see **Fig. 11B**). When the medial shuttle 72b is axially translated in the distal direction 150c and toward the distal-most shuttle 72a, the tubular distal portion 148a of the axially split sheath 136 associated with the medial shuttle 72b shortens (see **Fig. 11C****).** In contrast, when the medial shuttle 72b is axially translated in the proximal direction 150d and away from the distal-most shuttle 72a, the tubular distal portion 148a of the axially split sheath 136 associated with the medial shuttle 72b lengths (see **Fig. 11D****).**

It should be appreciated that the split 146 in the tubular body 138 serves to facilitate placement of the axially split sheath 136 in the flattened state by transversely expanding to allow the tubular body 138 to open up into a ribbon. The split 146 in the tubular body 138 also serves to expose the central lumen 144, thereby allowing the guide sheath 26a or working catheter 26b to be transversely loaded into the exposed central lumen 144 at the interface between the tubular distal portion 148a and transition portion 148c of the axially split sheath 136. The central lumen 144 is sized to slidably receive the guide sheath 26a or working catheter 26b therein, such that the axially split sheath 136 provides support to the guide sheath 26a or working catheter 26b. Although the axially split sheath 136 may be transversely flexible, the axially split sheath 136 preferably has a sufficient columnar strength, such that the tubular distal portion 148a prevents the guide sheath 26a or working catheter 26b from prolapsing when an axial compression force caused by axial displacement of the shuttles 72a-72b is applied to the guide sheath 26a or working catheter 26b.

When the respective one of the shuttles 72a-72b axially translates the respective one of the guide sheath 26a and working catheter 26b in the distal direction, the axially split sheath 136, the tubular distal portion 148a of the axially split sheath 136 lengthens to envelope the respective guide sheath 26a or working catheter 26b at the transition portion 148c. In contrast, when the respective one of the shuttles 72a-72b axially translates the respective one of the guide sheath 26a and working catheter 26b in the distal direction, the tubular distal portion 148a of the axially split sheath 136 lengthens to peel away from the respective guide sheath 26a or working catheter 26b at the transition portion 148c.

In various embodiments, the anti-buckling mechanism 132 may have any suitable arrangement of a flattening mechanism (e.g., rollers, pins, rectangular orifices, etc.) for transitioning the axially split sheath 136 from the tubular state into the flattened state as it is furled into a stowage housing; a tube forming mechanism (e.g., an arcuate bearing surface) for transitioning the axially split sheath 136 from the flattened state to the tubular state as it is unfurled from the stowage housing; and a take-up spool for furling the axially split sheath 136.

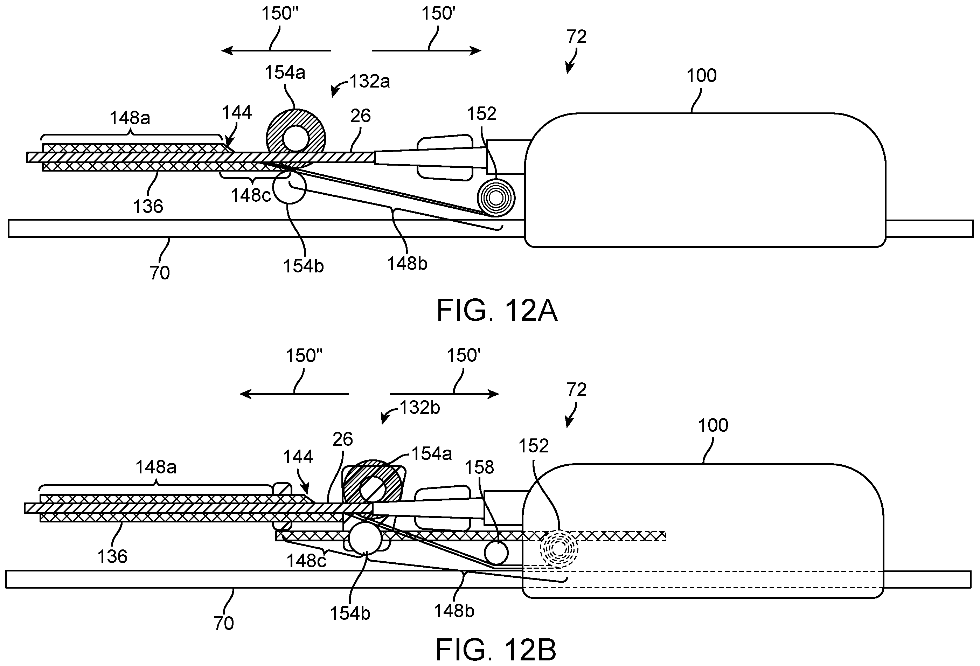

For example, with reference to **Fig. 12A****,** one embodiment of an anti-buckling mechanism 132a comprises a take-up reel 152 configured for alternately rolling and unrolling the flattened proximal portion 148b of the axially split sheath 136. In this embodiment, the take-up reel 152 is located outside of the casing 100 and has an axis of rotation (coming out from the drawing) that is parallel to the plane in which the track 70 lies. The anti-buckling mechanism 132a further comprises a flattening mechanism in the form of a pair of pinch rollers 154a, 154b between which the axially split sheath 136 is tightly disposed. In this manner, the axially split sheath 136 will transition from the tubular state to the flattened state at the point of contact between the pinch rollers 154a, 154b when the axially split sheath 136 axially translates in the proximal direction 150' relative to the shuttle 72 (i.e., during furling of the axially split sheath 136), and will transition from the flattened state to the tubular state at the point of contact between the pinch rollers 154a, 154b when the axially split sheath 136 axially translates in the distal direction 150" relative to the shuttle 72 (i.e., during unfurling of the axially split sheath 136). Thus, the flattened proximal portion 148b of the axially split sheath 136 extends proximally from the pinch rollers 154a, 154b, while the transition portion 148c, and then the tubular distal portion 148a, of the axially split sheath 136 extends distally from the pinch rollers 154a, 154b.

In this embodiment, the axial split sheath 136 may be pre-shaped to assume a tubular shape in the absence of an external force, so that it naturally transitions from the flattened state to the tubular state when the axially split sheath 136 axially translated in the distal direction 150" relative to the shuttle 72. The elongate medical device 26 (e.g., the guide sheath 26a and working catheter 26b) may be loaded into the exposed central lumen 144 at the interface between the tubular distal portion 148a and transition portion 148c of the axially split sheath 136.

With reference to **Fig. 12B****,** another embodiment of an anti-buckling mechanism 132b is similar to the anti-buckling mechanism 132a of **Fig. 12A****,** with the exception that the take-up reel 152 is located inside of the casing 100. The anti-buckling mechanism 132b further comprises a tube forming mechanism 156 distal to the pinch rollers 154a, 154b and through which the axially split sheath 136 is disposed. In the same manner described above with respect to **Fig. 12A****,** the axially split sheath 136 will transition from the tubular state to the flattened state at the point of contact between the pinch rollers 154a, 154b when the axially split sheath 136 axially translates in the proximal direction 150' relative to the shuttle 72 (i.e., during furling of the axially split sheath 136). However, instead of transitioning from the flattened state to the tubular state at the point of contact between the pinch rollers 154a, 154b, the axial split sheath 136 will transition from the flattened state to the tubular state at the tube forming mechanism 156 when the axially split sheath 136 axially translates in the distal direction 150" relative to the shuttle 72 (i.e., during unfurling of the axially split sheath 136). Thus, the flattened proximal portion 148b of the axially split sheath 136 extends proximally from the pinch rollers 154a, 154b, while the transition portion 148c of the axially split sheath 136 extends between the pinch rollers 154a, 154b and the tube forming mechanism 156, and the tubular distal portion 148a of the axially split sheath 136 extends distally from the tube forming mechanism 156.

In this embodiment, the axial split sheath 136 may be bi-stable, so that it transitions from the tubular state to the flattened state only in response to the external force applied by the pinch rollers 154a, 154b when the axially split sheath 136 axially translated in the proximal direction 150' relative to the shuttle 72, and transitions from the flattened state to the tubular state only in response to the external force applied by the tube forming mechanism 156 when the axially split sheath 136 is axially translated in the distal direction 150" relative to the shuttle 72. The anti-buckling mechanism 132b further comprises an idler roller 158 against which the which the flattened proximal portion 148b of the axial split sheath 136 bears, so that it can be directed toward the take-up reel 152 is located inside of the casing 100.

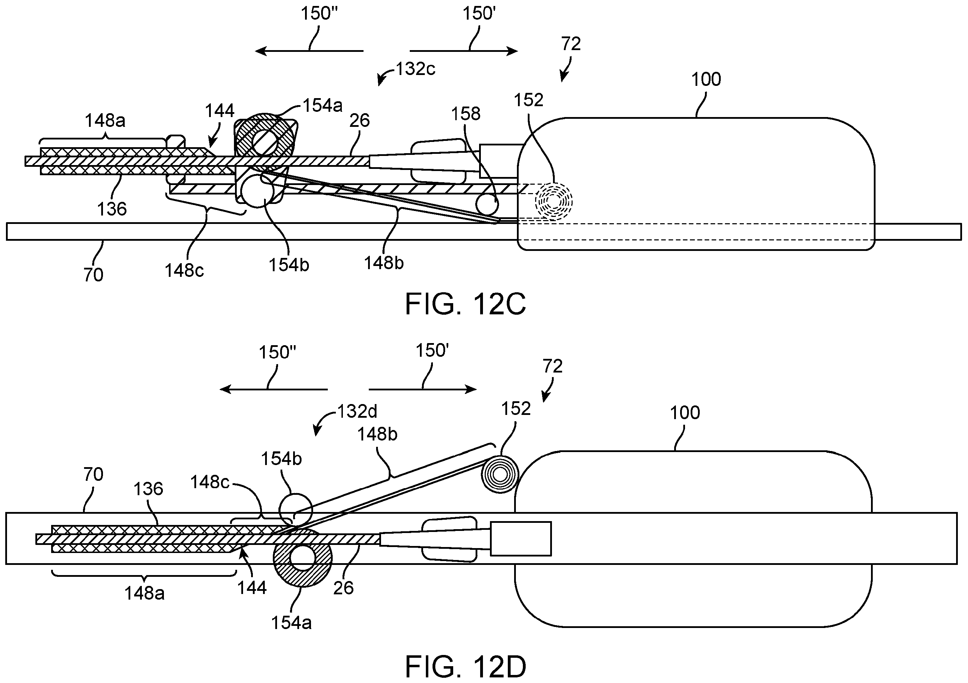

With reference to **Fig. 12C****,** still another embodiment of an anti-buckling mechanism 132c is similar to the anti-buckling mechanism 132b of **Fig. 12B****,** with the exception that the distance between the pinch rollers 154a, 154b and the take-up reel 152 is greater.

With reference to **Fig. 12D****,** yet another embodiment of an anti-buckling mechanism 132d is similar to the anti-buckling mechanism 132a of **Fig. 12A****,** with the exception that the take-up reel 152 is located outside of the casing 100 and has an axis of rotation (coming out from the drawing) that is orthogonal o the plane in which the track 70 lies.

In the illustrated embodiment, the axially split sheath 136 is configured for furling within the stowage housing 134 associated with each of the shuttle 72a-72b in a passive manner. That is, the anti-buckling mechanism 132 has no mechanism within the stowage housing 134 that actively pulls on the axially split sheath 136 (i.e., reels in) to furl it within the stowage housing 134. Rather, the external compression force applied to the axially split sheath 136 associated with the distal-most shuttle 72a when the distal-most shuttle 72a is axially translated in the distal direction 150", and the compression force applied to the axially split sheath 136 associated with the medial shuttle 72b when the medial shuttle 72b and the distal shuttle 72a are axially displaced toward each other causes the respective axially split sheath 136 to furl within the stowage housing 134.

In an alternative embodiment, the stowage housing 134 may include a take-up biasing mechanism (not shown) for applying a passive stowing force that ensures the flattened proximal portion 148b of the axially split sheath 136 remains in a contact and tight form during repeated furling and unfurling of the axially split sheath 136 within and out of the stowage housing 134 in response to back and forth movement of the distal shuttle 72a and medial shuttle 72b (in the illustrated embodiment, when the distal-most shuttle 72a is axially translated in the distal direction or the medial shuttle 72b and distal shuttle 72a are axially displaced toward each other). Without such passive stowing force, the size (i.e., diameter) of the rolled flattened proximal portion 148b of the axially split sheath 136 may outgrow the allotted space within the stowage housing 136, thereby preventing unencumbered furling or unfurling of the axially split sheath 136 within or out of the stowage housing 134. Such passive stowing force should be biased to enable unencumbered deflection and opening of the axially split sheath 136, preferably without biasing unconstrained furling of the axially split sheath 136 within the stowage housing 134 (i.e., shortening of the tubular distal portion 148a) so as to prevent un-commanded movement of the guide sheath 26a or working catheter 26b into the patient 20.

As will be described in further detail below, some embodiments of anti-buckling mechanisms may apply such a passive stowing force on the axially split sheath 136. In other more complicated embodiments, take-up biasing mechanism may comprise a tension spring (not shown) or a motor (not shown) that applies constant tension on the flattened proximal portion 148b of the axially split sheath 136. If a motor is used to apply constant tension on the respective axially split sheath 136, and the axial actuator 94 used to actuate the axial drive mechanism 92 that axially translates the distal shuttle 72a or medial shuttle 72b along the track 70 is a motor, both motors may be indexed to match each other, such that the speed at which the axially split sheath 32 furls or unfurls matches the speed of the respective shuttle 72 when axially translated along the track 70.

In an optional embodiment, the anti-buckling mechanism 132 associated with either or both of the shuttles 72a-72b may additionally serve as an actuator and axial drive mechanism for axially translating the shuttles 72a-72b. In this case, the axially split sheath 136 of the anti-buckling mechanism 132 may replace the axial drive mechanism 92 described above, while a motor (not shown) within the stowage housing 134 of the anti-buckling mechanism 132 may replace the motorized actuator 94 described above. Thus, the axially split sheath 136 is configured for being actuated to furl the axially split sheath 136 into the stowage housing 134 and unfurl the axially split sheath 136 from the stowage housing 134 while axially translated the respective one of the shuttles 72a-72b, whereas the motor within the stowage housing 134, in response to control signals generated by the master input device 194, is configured for actuating the axially split sheath 136. When used as the axial drive mechanism, the axially split sheath 136 preferably has the necessary columnar strength, such that it does not prolapse under compression when the axial split sheath 136 is unfurled from the stowage housing 134 for each of the shuttles 72a-72b.

In the case of the distal-most shuttle 72a, active furling of the axially split sheath 136 into the stowage housing 134 causes axial tension within the axially split sheath 136 between the distal-most shuttle 72a and the location that is fixed relative to the patient 20 (e.g., the sheath anchor 80 used to affix the distal end 74 of the track 70 at or near the access site of the patient 20), thereby, in turn, causing the distal-most shuttle 72a to axially translate in the distal direction. In contrast, active unfurling of the axially split sheath 136 from the stowage housing 134 causes axial compression within the axially split sheath 136 between the distal-most shuttle 72a and the location that is fixed relative to the patient 20 (e.g., the sheath anchor 80 used to affix the distal end 74 of the track 70 at or near the access site of the patient 20), thereby, in turn, causing the distal-most shuttle 72a to axially translate in the proximal direction 150'.

In the case of the medial shuttle 72b, active furling of the axially split sheath 136 into the stowage housing 134 causes tension within the axially split sheath 136 between the medial shuttle 72b and the distal-most shuttle 72a, thereby, in turn, causing the medial shuttle 72b to axially translate in the distal direction. In contrast, active unfurling of the axially split sheath 136 from the stowage housing 134 causes axial compression within the axially split sheath 136 between the medial shuttle 72b and the distal-most shuttle 72a, thereby, in turn, causing the medial shuttle 72b to axially translate in the proximal direction 150'.

It should be appreciated that, although the use of anti-buckling mechanisms 132 illustrated in **Figs. 12A-12D** lends itself well to preventing buckling of the guide sheath 26a and working catheter 26b in the endovascular management and tracking system 14 illustrated in **Figs. 1-2****,** such anti-buckling mechanisms 132 can be used with other types of catheter driver systems, including conventional driver systems that do not utilize shuttles that axially translate on a track.

Furthermore, although the use of an axially split sheath that alternately furls within and unfurled from a stowage housing provides an elegant and simple solution for supporting and preventing prolapse of the guide sheath 26a and working catheter 26b when they undergo axial compression in response to axial displacement of the shuttles 72a-72b along the track 70, alternative embodiments of anti-buckling mechanisms may use other means for providing variable length support for the guide sheath 26a or working catheter 26b when affixed to the endovascular management and tracking system 14 illustrated in **Figs. 1-2****.** In much the same manner as the anti-buckling mechanism 132 described above, the distal end of each of these scissor mechanisms may be affixed to a location (e.g., to the sheath anchor 80 if associated with the distal-most shuttle 72a or to the distal most-shuttle 72a if associated with the medial shuttle 72b), such that the these anti-buckling mechanisms lengthen when the respective shuttle 72 is axially translated away from the location, and shortens when the respective shuttle 72 is axially translated toward the location.

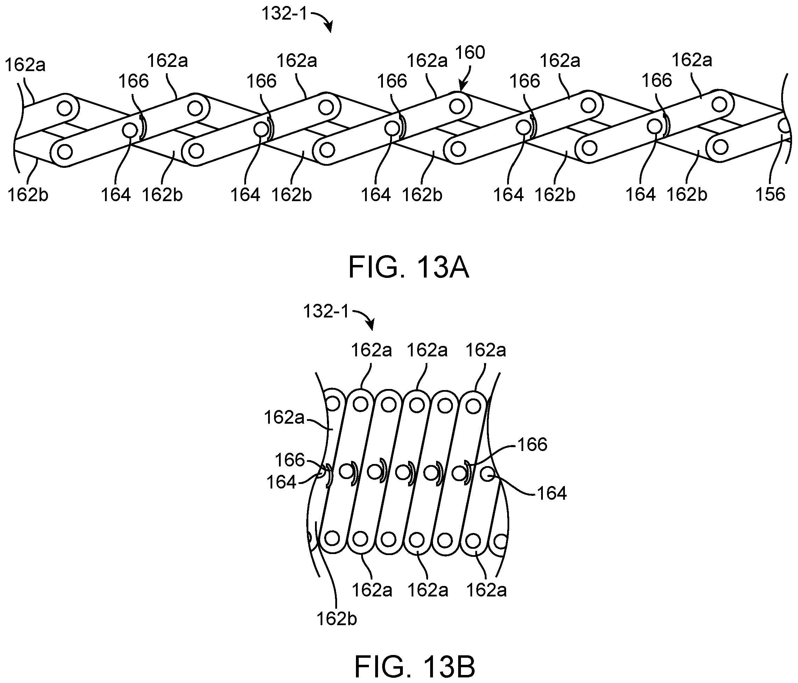

One alternative embodiment of an anti-buckling mechanism 132-1 comprises a scissor mechanism 160 capable of lengthening **(****Fig. 13A****)** or shortening **(****Fig. 13B****).** The scissor mechanism 160 comprises two series of hinged links 162a, 162b that interconnect with each other at the midpoints 164 of the hinged links 162a, 162b, and hinge away from each other to lengthen the scissor mechanism 160 or toward each other to shorten the scissor mechanism 160. The anti-buckling mechanism 132-1 may comprise a series of loops 166 affixed along the length of the scissor mechanism 160 through which the guide sheath 26a or working catheter 26b may be slidably disposed.

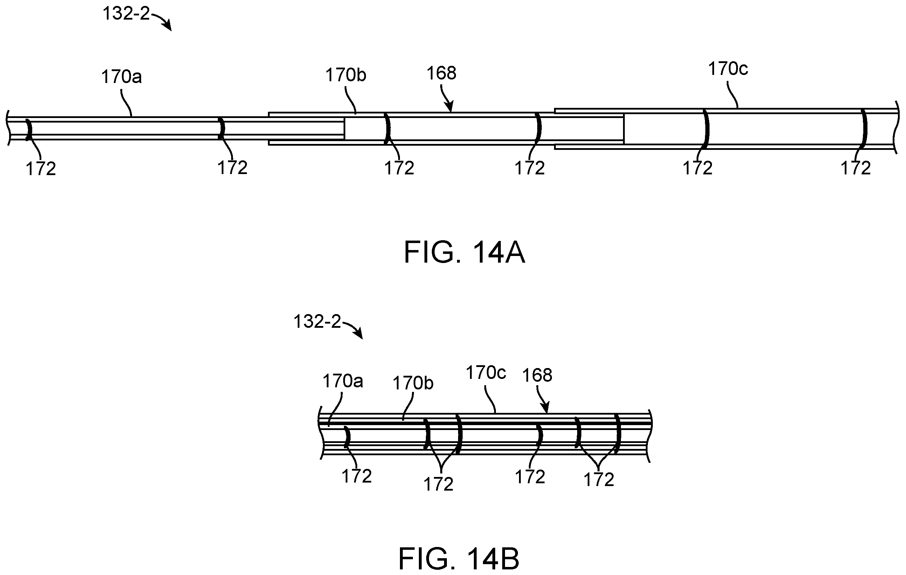

Another alternative embodiment of an anti-buckling mechanism 132-2 comprises a linear slide mechanism 168 capable of lengthening **(****Fig. 14A****)** and shortening **(****Fig. 14B****).** The linear slide mechanism 168 comprises a plurality of rigid linear slides 170a-170c that slide relative to each other to lengthen or shorten the linear slide mechanism 168. The anti-buckling mechanism 132-2 may comprise a series of loops 172 affixed along the length of the linear slide mechanism 168 through which the guide sheath 26a or working catheter 26b may be slidably disposed.

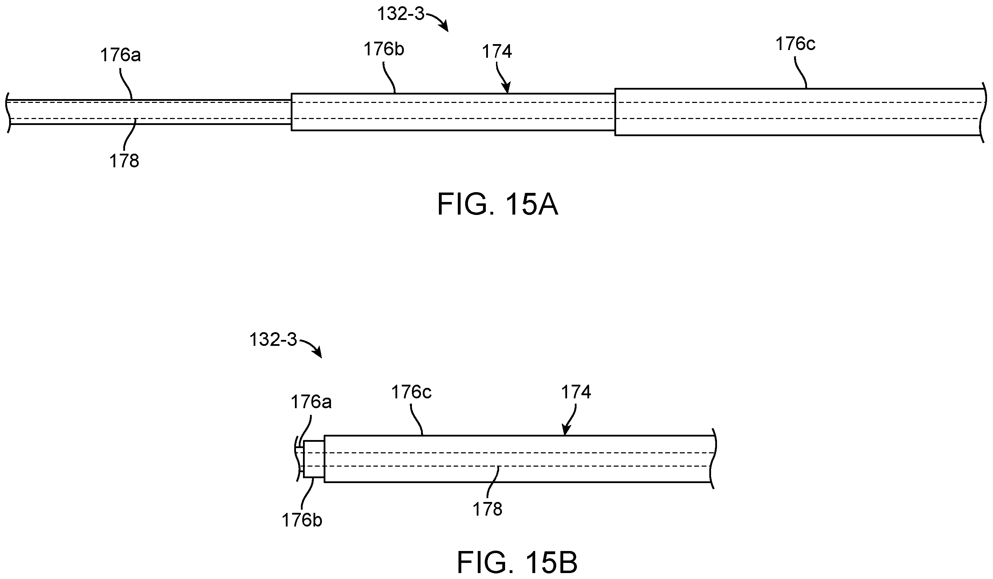

Still another alternative embodiment of an anti-buckling mechanism 132-3 comprises a telescoping mechanism 174 capable of lengthening **(****Fig. 15A****)** and shortening **(****Fig. 15B****).** The telescoping mechanism 174 comprises a plurality of hollow cylinders 176a-176c that slidably fit within one another to lengthen or shorten the telescoping mechanism 174. The telescoping mechanism 174 comprises a central lumen 178 (shown in phantom) through which the guide sheath 26a or working catheter 26b may be slidably disposed.

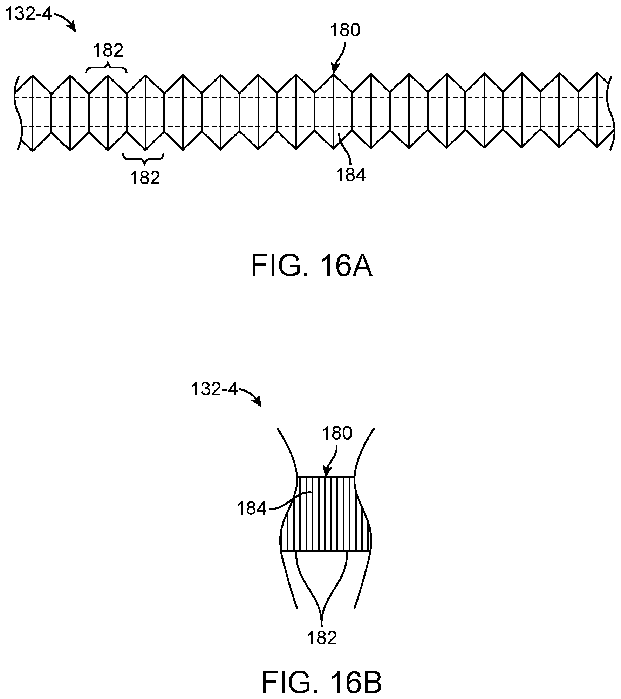

Yet another alternative embodiment of an anti-buckling mechanism 132-4 comprises an accordion mechanism 180 capable of lengthening **(****Fig. 16A****)** and shortening **(****Fig. 16B****).** The accordion mechanism 180 comprises a plurality of sections 182 that fold away from each other to lengthen the accordion mechanism 180 or toward each other to shorten the accordion mechanism 180. The accordion mechanism 180 comprises a central lumen 184 through which the guide sheath 26a or working catheter 26b may be slidably disposed.

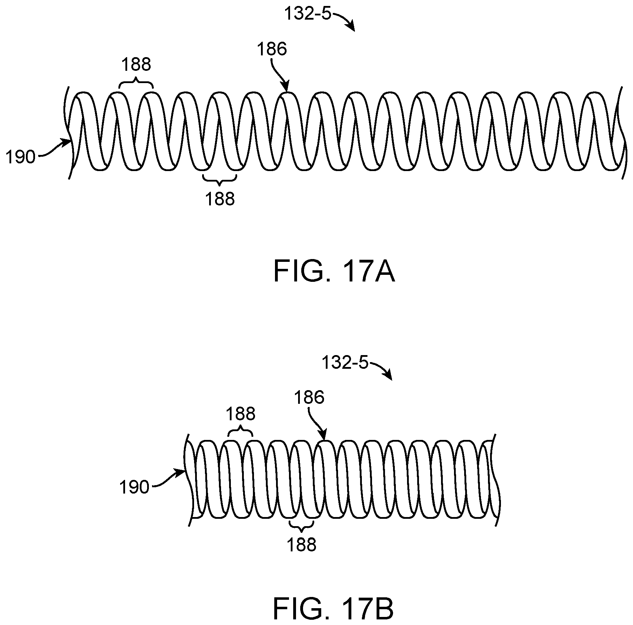

Yet another alternative embodiment of an anti-buckling mechanism 132-5 comprises a linear spring mechanism 186 capable of lengthening **(****Fig. 17A****)** and shortening **(****Fig. 17B****).** The linear spring mechanism 186 comprises a plurality of coils 188 that can be displaced away from each other to lengthen the linear spring mechanism 186 or toward each other to shorten the linear spring mechanism 186. The linear spring mechanism 186 comprises a central lumen 190 through which the guide sheath 26a or working catheter 26b may be slidably disposed.

In an alternative embodiment of the coaxial endovascular assembly 12, the working catheter 26b and/or guidewire 26c may be reinforced, such that the coaxial endovascular assembly 12 has a low tendency of buckling. In this case, one or both of the shuttles 72a-72b may forego the use of an anti-buckling mechanism 132.

In one embodiment, if the actuators 94, 98 are motorized, each of the shuttles 72a-72c may comprise sensors that measure forces or torques imparted by the actuators 94, 98 on the drive mechanisms 92, 96, thereby enabling a sensing of resistance to motion that can be communicated back to the haptic interface, which as described in further detail below, communicates tactile feedback to the operator 24 via control station 16 as input commands are provided via the control station 16.

Communications between and within the endovascular management and tracking system 14 and control station 16 may occur through wired or wireless connections or a combination thereof. All standard communication architectures (e.g., ethernet, USB, Wi-Fi, Bluetooth, FireWire, etc.) and all data transfer protocols are envisioned to form either a centralized control like where the control station 16 manages all communications (point-to-point) or a micro-local network where the endovascular management and tracking system 14 and control station 16 communicate with each other in a coordinated manner. Data streams between the endovascular management and tracking system 14 and control station 16 may include information, such as, e.g., motor/actuator commands, shuttle-based user input commands, flow switch commands, axial track location sensing output, force/torque sensor outputs, fluid flow outputs, pressure sensor outputs, etc.

As will be described in further detail below, the control station 16 may be connected to the endovascular management and tracking system 14 via a cable, thereby providing one or more communication links capable of transferring signals between the control station 16 and the endovascular management and tracking system 14. Alternatively, the control station 16 may be wirelessly connected to the endovascular management and tracking system 14. For example, the control station 16 may be located in a geographically remote location and communication is accomplished, at least in part, over a wide area network such as the Internet. The control station 16 may also be connected to the endovascular management and tracking system 14 via a local area network or even wireless network that is not located at a geographically remote location.

Referring back to **Fig. 6****,** in one embodiment, a power and communications cable 192a couples the control station 16 to one of the shuttles 72a-72c, and power and communications cables 192b-192c couple that shuttle to the remaining shuttles in a daisy chain fashion. For example, the power and communications cable 192a may couple the control station 16 to the distal-most shuttle 72a, so that power and communications can be maintained between the control station 16 and endovascular management and tracking system 14 as preceding shuttles (e.g., the medial shuttle 72b or proximal-most shuttle 72c) are loaded onto and unloaded from the track 70. In other embodiments, separate power and communication wires may individually couple the control station 16 to the shuttles 72a-72c. In an alternative embodiment, rather than using the power and communications cable 192c, power and communications can be supplied to the proximal-most shuttle 72c from the medical shuttle 72b via electrically coupling between the male plug 108 of the proximal-most shuttle 72c and the loading/unloading port 88 of the medial shuttle 72b.

In another embodiment, the track 70 is at least composed of an electrically conductive material, and is coupled to a power source, such that the track 70 may be electrified. In this manner, power can be transmitted from the electrified track 70 to the shuttles 72a-72c (e.g., via direct sliding contacts or inductive coupling). In still another embodiment, the shuttles 72a-72c may be respectively powered by reusable or single-use batteries. In these cases, data can be transferred to or from the respective shuttles 72a-72c individual wireless communication links (e.g., via radio frequency (RF), microwave, infrared (IR), inductive coupling via the track 70, or other wireless transmission links) between the respective shuttles 72a-72c and the control station 16 and/or via wireless communications links between the shuttles 72a-72c.

Referring back to **Figs. 1** and **2****,** the control station 16 comprises a master input device 194 operatively coupled to the endovascular management and tracking system 14. The operator 24 may interact with the master input device 194 to operate the endovascular management and tracking system 14 in a master-slave arrangement. To this end, the master input device 194 is configured for allowing the operator 24 to remotely control the endovascular management and tracking system 14 to perform real-time catheter-based medical procedures on the patient 20. For example, the master input device 194 may be configured for causing the endovascular management and tracking system 14 to perform various tasks using the coaxial endovascular assembly 12 (e.g., to advancing, retracting, rotating, optional steering of the guide sheath 26a, working catheter 26b, and guidewire 26c relative to each other, etc.).

The control station 16 further comprises a control panel 196 (e.g., a pad or on-screen touch control) configured for displaying status and/or non-physical or non-motion input, such as, e.g., text or numeric or on/off information. For example, the control panel 196 may be configured for allowing the physician to change general settings, set-up a library for each of the shuttles 72 including associating each of the shuttles 72 with a particular one of the elongate medical devices 26, and to link any of the shuttles 72 with each other to provide synchronization motion between the elongate medical devices 26. Optionally, the control panel 196 may allow the operator 24 to perform non-real-time functions, such as locking individual shuttles (described in further detail below), synchronizing the shuttles 72, changing synchronization parameters, such as direction and ratio of motion, performance of interventional/diagnostic functions using the working catheter 26b (e.g., stent delivery, balloon inflation, ablation/mapping of endocardial tissue, ultrasound imaging, atherectomy of a blood vessel, delivery of vaso-occlusive devices into an aneurysm, delivery of contrast/medicine, or to perform any other medical function for which the working catheter 26b is designed), etc.

The control panel 196, or alternatively a separate graphical display (not shown), may optionally display various aspects of the robotic endovascular system 10. For example, an image of the distal end of the coaxial endovascular assembly 12 may be displayed in real time on the control panel 196 to provide the operator 24 with the current orientation of the elongate medical devices 26 as they are positioned within the vasculature of the patient 20. The graphical display(s) may also be configured for displaying patient-specific information to the operator 24 (e.g., image data (e.g., x-ray images, MRI images, CT images, ultrasound images, etc.), hemodynamic data (e.g., blood pressure, heart rate, etc.), and patient record information (e.g., medical history, age, weight, etc.). For example, the control station 16 may be interfaced with an angiographic system (not shown) or its display components, such that the control station 16 (e.g., the control panel 196) may display information or images from the angiographic system, or vice versa. Furthermore, the control station 16 (e.g., the control panel 196) may be configured to receive input to send commands to the angiographic system to move the procedure table 18 or change the position of the x-ray camera/source. The control station 16 (e.g., the control panel 196) may also be configured for controlling or triggering other ancillary devices, such as power injectors and infusion pumps.

In the illustrated embodiment, the control station 16 is affixed to the procedure table 18 via a clamp mechanism 198. The clamp mechanism 198, or alternatively a different clamp mechanism (not shown), can be used to affix the proximal end 76 of the track 70 (shown in **Fig. 6****).** Having only a single point of attachment allows the control station 16 to be clamped onto a table or similar surface as needed without taking up valuable space in the operating room environment. In an alternative embodiment, the control station 16 can be setup remotely from the endovascular management and tracking system 14 outside of the radiation field with communications, processing, and interventionalist interfacing with the endovascular management and tracking system 14.

The master input device 194 may comprise multiple controllers for respectively controlling the motions of the respective elongate medical devices 26 via the shuttles 72. The motions of all of the respective elongate medical devices 26 may be independently controlled by the multiple controllers of the master input device 194, or the motions of at least two of the respective elongate medical devices 26 may be linked together to provide synchronized motion (e.g., 1:1 linked motion), such that control of one of the elongate medical devices 26 by one of the multiple controllers of the master input device 194 controls another one of the elongate medical devices 26 in accordance with the linked motion (either axial translation or rotational translation).

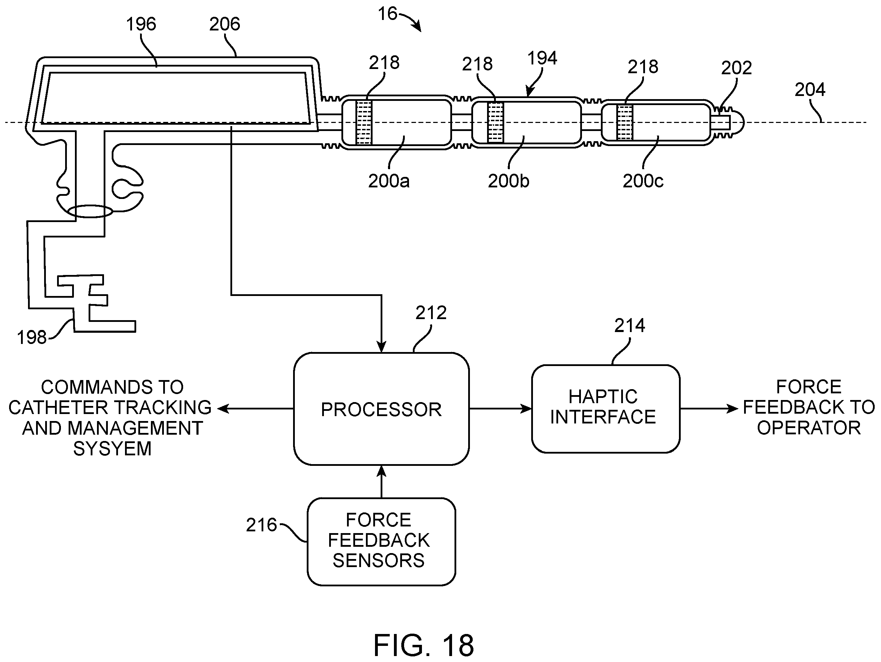

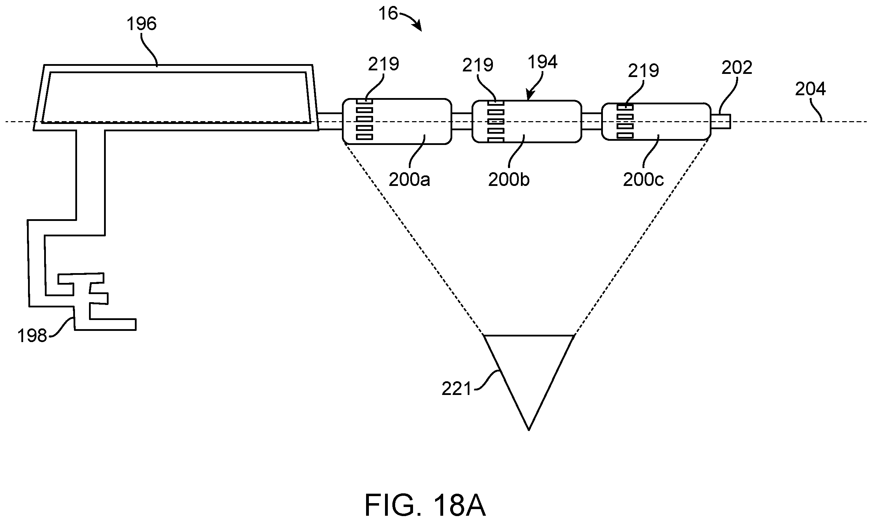

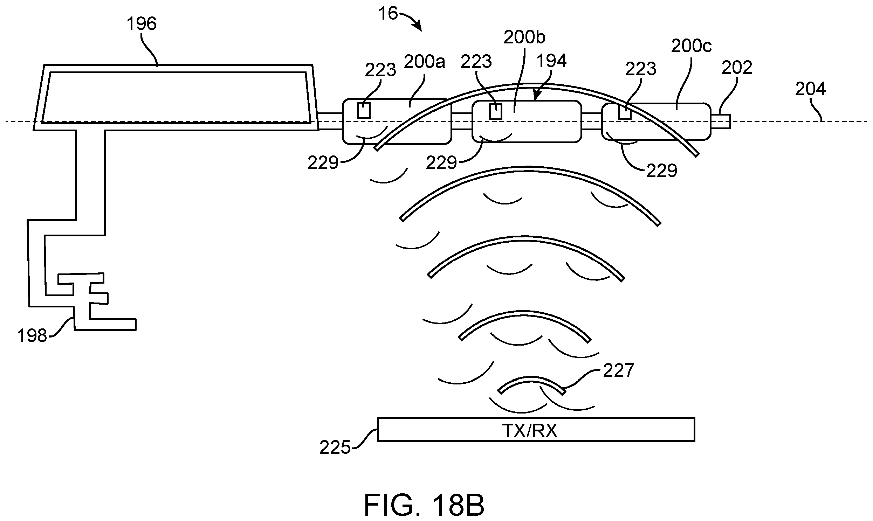

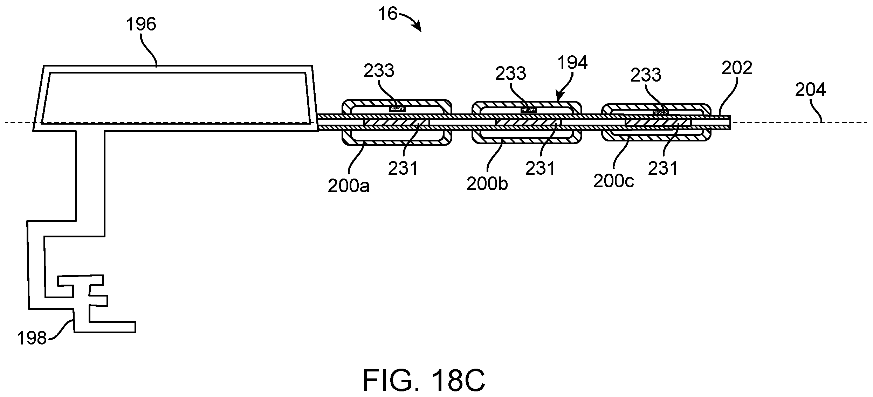

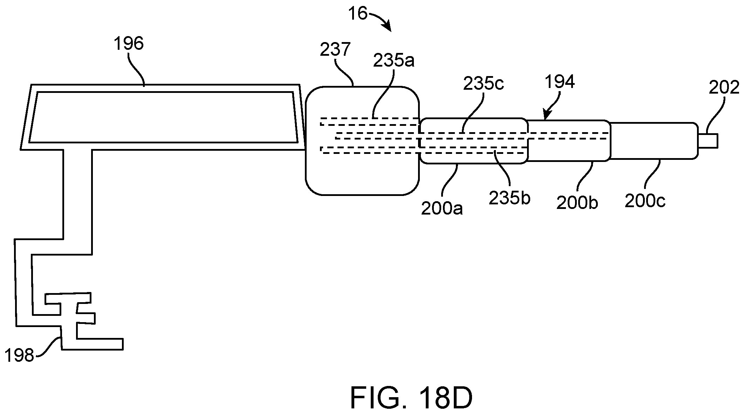

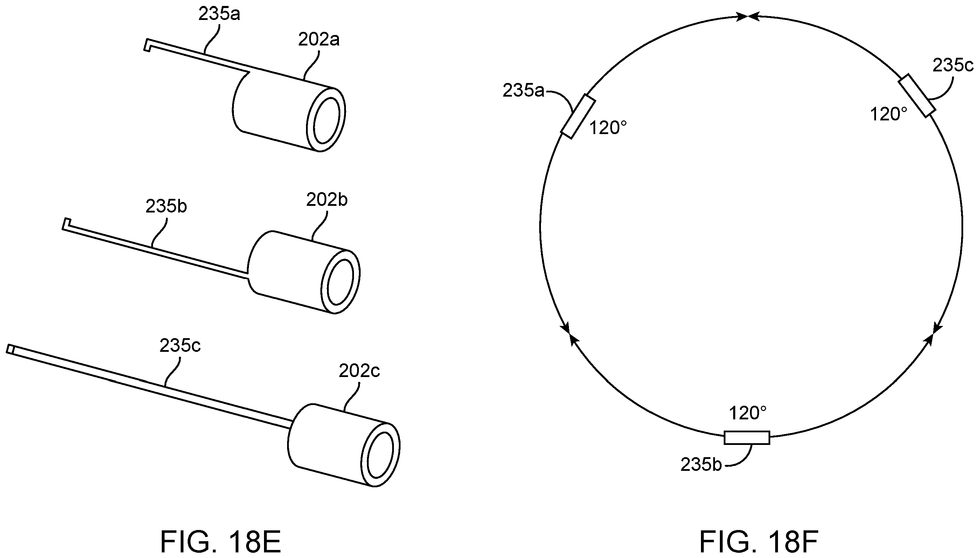

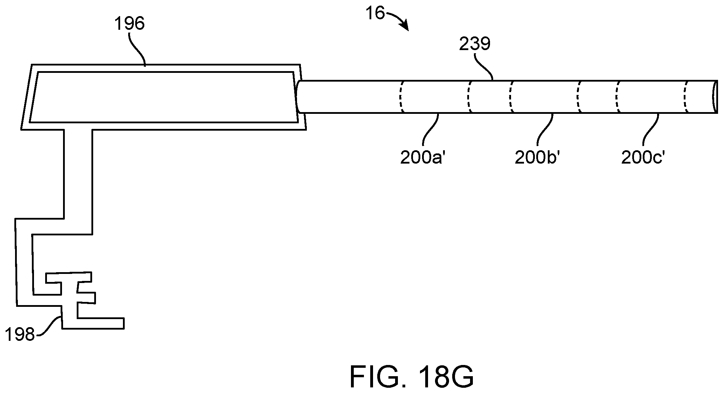

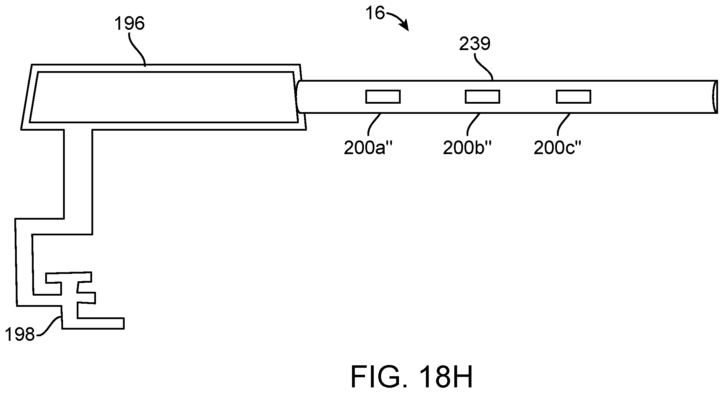

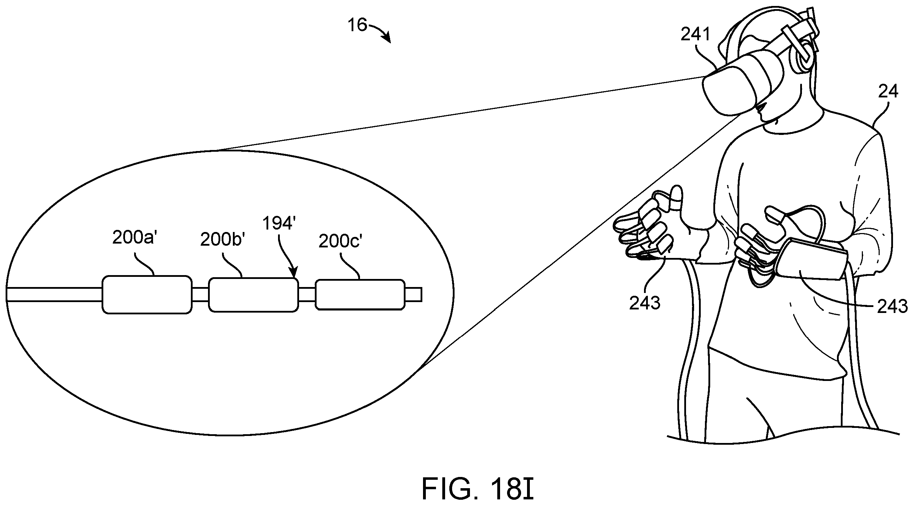

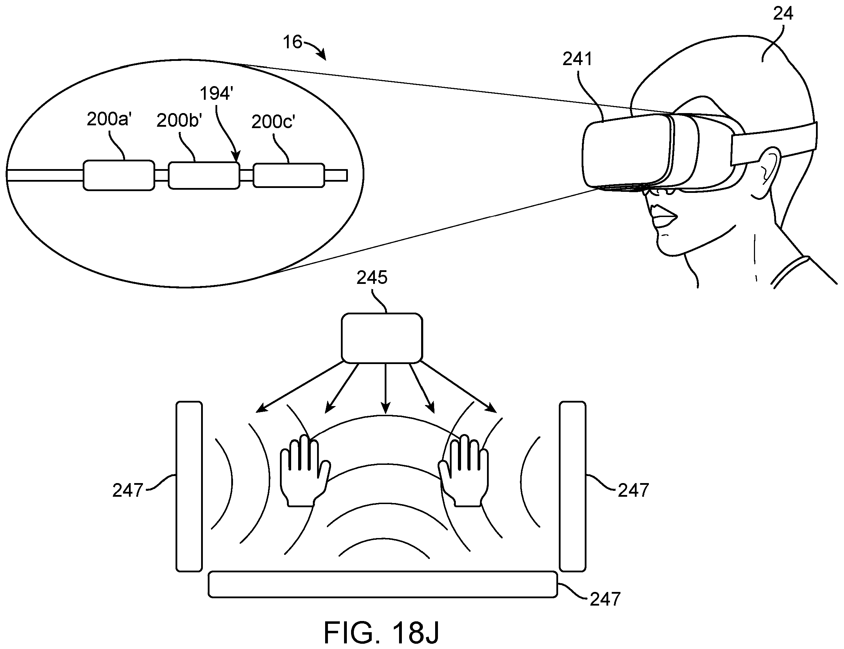

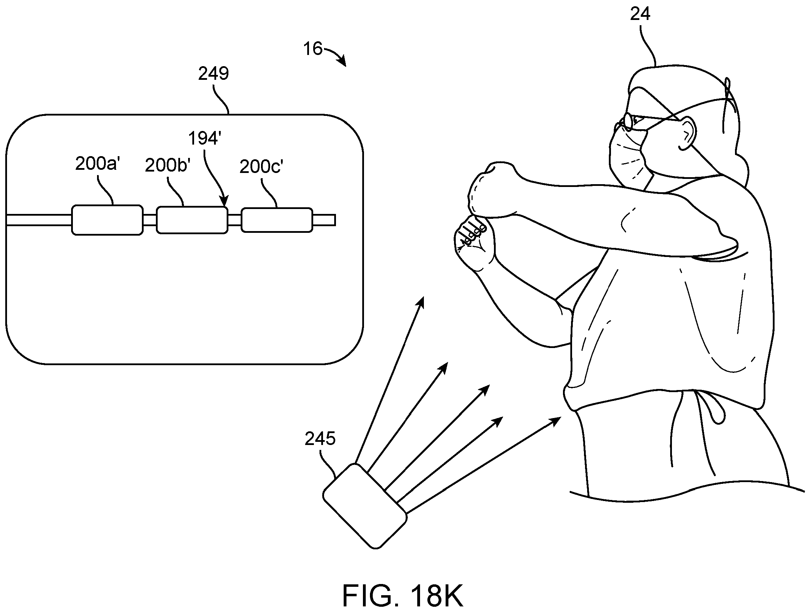

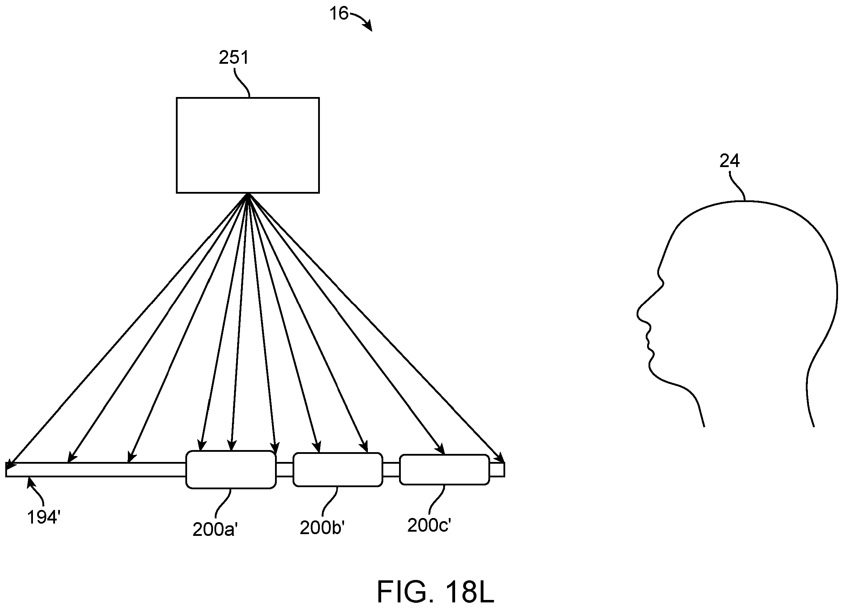

In one advantageous embodiment illustrated in **Fig. 18****,** the master input device 194 comprises a linear array of individual control elements 200a-200c and a rail 202 on which the control elements 200a-200c are axially and rotationally slidable (i.e., they may both axially translate along and rotationally translate about a longitudinal axis 204 of the rail 202. The use of the linear array of individual control elements 200a-200c for independently manipulating the elongate medical devices 26a-26c uses similar motions to those used in the context of manual catheter lab device manipulation, and thus, would allow physicians to perform familiar and intuitive endovascular procedures. That is, the operator 24 may grasp one of the control elements 200a-200c and move the grasped control element along the longitudinal axis 204 of the rail 202 (either proximally or distally) to axially translate the elongated medical device 26 (either proximally or distally) that is controlled by the grasped control element 200, or rotate the grasped control element about the longitudinal axis 204 of the rail 202 (either clockwise or counterclockwise) to rotationally translate the elongated medical device 26 (either clockwise or counterclockwise) that is controlled by the grasped control element 200.

The rail 202 may be rigid to provide the control elements 200a-200c with only two degrees-of-freedom (DOF) (i.e., linear translation along the longitudinal axis 204 of the rail 202 (z-direction) and rotational translation about the longitudinal axis 204 of the rail 202 (roll), or may be flexible to provide the control elements 200a-200c with additional four DOFs (i.e., linear translation along the x- and y-directions and pitch and yaw relative to the longitudinal axis 204 of the rail 202). In the illustrated embodiment, the rail 200 is straight, such that the array of control elements 200a-200c arranged on the rail is rectilinear, although in alternative embodiment, the rail 200 may be slightly curved, such that the array of control elements 200a-200c arranged on the rail are slightly curvilinear. Although the control elements 200a-200c are illustrated as being cylindrical in nature to emulate the proximal ends of the elongate medical devices 26a-26c, it should be appreciated that the control elements 200a-200c may have any suitable cross-section other than circular, including oval, ellipsoidal, polygonal (e.g., triangular, rectangular, hexagonal, octagonal, etc.), etc.

The individual control elements 200a-200c may be operatively associated with the respective shuttles 72a-72c, such that each control element controls movement (axial and rotational) of the guide sheath 26a, working catheter 26b, or guidewire 26c affixed to the respective one of the shuttles 72a-72c. In one embodiment, the control elements 200a-200c are axially and rotationally spring-loaded, such that they return to their nominal axial translation and rotational translation positions when released by the operator 24. These nominal axial translation and rotational translation positions are preferably in the centers of the axial translation ranges and rotational translation ranges, such that the control elements 200a-200c may have positive and negative travel ranges from the nominal positions (i.e., a forward axial translation range, a reverse axial translation range, a clockwise rotational translation range, and a counterclockwise rotational translation range).

Notably, the rail 202 has an open end by virtue of clamping the control station 16 at only one point via the clamping mechanism 196, thereby facilitating quick exchange of the control elements 200a-200c. Each of the control elements 200a-200c may be removably affixed to the rail 202, such that control elements 200 may interchangeable in that they can be added to or removed from the master input device 194, as needed. Furthermore, while the rail 202 may be reusable, it is desired that the control elements 200a-200c be single-use and disposable. Thus, the used control elements 200a-200c may be easily removed from the rail 202 and discarded and replaced with new and sterilized control elements 200a-200c.

Although three control elements 200a-200c respectively corresponding to the three shuttles 72a-72c are illustrated in **Fig. 18****,** it should be appreciated that the master input device 194 may comprise any suitable number of control elements 200, ultimately depending on the number of shuttles 72 used. For example, the master input device 194 may comprise less three control elements 200, e.g., if less than three shuttles 72 are used, or more than three control elements 200, e.g., if more than three shuttles 72 are used. Although not as advantageous, instead of an array of control elements 200, the master input device 194 may comprise joy-sticks, keypads or keyboards, sliders, roller balls, touch sensors, touch screens, mouses, etc.

In the illustrated embodiment, the control elements 200a-200c are progressively larger or smaller in diameter to visually or tactilely indicate to the physician which of control elements 200a-200c are associated to the respective one of the elongate medical devices 26a-26c (e.g., the distal-most control element 200a may be operatively associated with the guide sheath 26a and may have a relatively large diameter, the medial control element 200b may be operatively associated with the working catheter 26b and have a relatively moderate diameter, and the proximal-most element 200c may be operatively associated with the guidewire 26c, and may have a relatively small diameter).

In an optional embodiment, the control elements 200a-200c are capable of being slid within each other in a telescoping fashion. In another optional embodiment, the control elements 200a-200c may illuminate with different colors (such as by red-green-blue (RGB) light emitting diodes (LEDs)) to indicate which of the control elements 200 are active and operatively associated with a respective one of the elongate medical devices 26a-26c. Any of the control elements 200 that are inactive may be illuminated with a white, clear-frosted color, or some similarly less-descript color. If the motions of two or more of the elongate medical devices 26a-26c are linked together, the control elements 200 that are associated with these linked devices can have a visual indication, such as flashing illumination.

The control station 16 may optionally comprises a sterile sleeve 206 configured for covering the master input device 194, thereby providing a sterile barrier between the operator 24 and the non-sterile components of the control station 16. To accommodate the sterile sleeve 206, the rail 202 is cantilevered, so that the sterile sleeve 206 can be easily slid over the array of control elements 200 from the free end of the rail 202 to the fixed end of the rail 202. The sterile sleeve 206, or a separate sterile sleeve (not shown), may also cover the control panel 196. The sterile sleeve 206 should allow unrestricted motion of each of the control elements 200. The sterile sleeve 206 may be configured to allow variations in the diameter of the control elements 200. The portion of the sterile sleeve 206 over each of the control elements 200a-200c may be smooth and in close or near contact with the respective control element 200, or the sterile sleeve 206 may be loose to allow bunched sections between each adjacent pair of control elements 200.

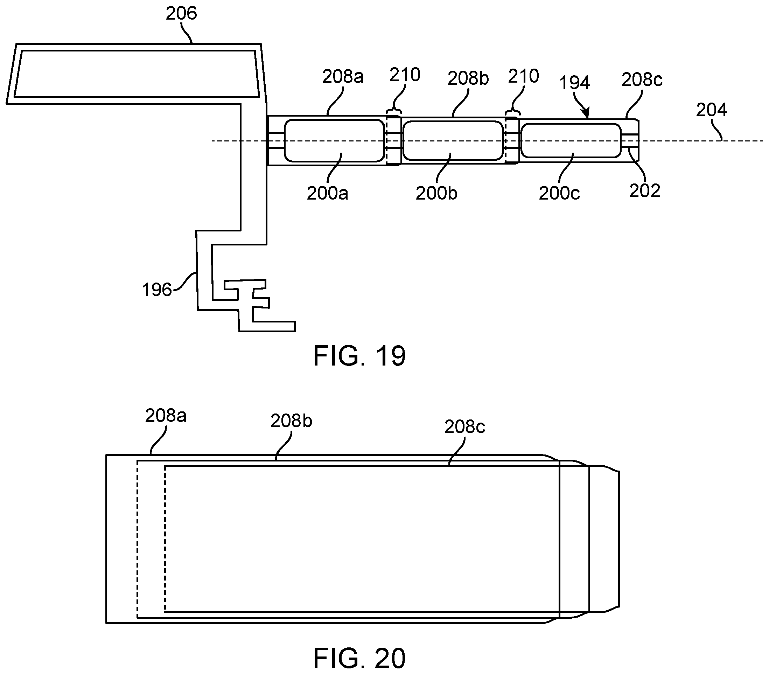

In one embodiment illustrated in **Fig. 19****,** instead of a single sterile sleeve that covers the entire master input device 194, the control station 16 comprises a set of single-use overlapping sterile control element covers 208a-208c, which are configured for being slid into place respectively over the control elements 200a-200c, thereby providing a sterile surface for the operator 24 to touch. When disposed over the respective control elements 200a-200c, the sterile control element covers 208a-208c may form slidable fluid seals 210 between adjacent control elements 200a-200c to prevent contaminated fluid transfer between any adjacent control elements 200a-200c. The slidable fluid seals 210 may be formed by a slip fit between the control elements 200a-200c or a sealing member, such as, e.g., an O-ring. As illustrated in **Fig. 20****,** the sterile control element covers 208a-208c may be packaged as a set of collapsed stacked "cups" that can be slid over the control elements 200a-200c.

Referring back to **Fig. 18****,** the control station 16 further comprises a processor 212, which may comprise, e.g., a personal computer or other type of computer work station for issuing commands to accurately coordinate and control actuations of various motors (described in further detail below) within the endovascular management and tracking system 14. As will be described in further detail below, the processor 212 may issue commands to the endovascular management and tracking system 14 in accordance with a mapping algorithm that maps the sensed axial translation and rotational translation of the control elements 200a-200c to the desired axial translation and rotational translation of the elongate medical devices 26a-26c. The control station 16 may optionally include various indicators in the form of lights, colored/non-colored segments of mechanical interfaces or sound cues, which may communicate power, manual mode/automated mode, fluid flow or fluid switch state, motion, or errors (e.g., excessive force or torque, controller errors, communication errors, low battery, etc.).

The control station 16 further comprises one or more devices for providing feedback (e.g., visual, audible, haptic, etc.) to the operator 24. For example, the control station 16 may have a haptic interface 214 that connects the operator 24 and the coaxial endovascular assembly 14 through a sense of touch, also known as kinesthetic stimulation or tactile feedback. The haptic interface 214 communicates tactile feedback to the operator 24 via, e.g., the master input device 194, as input commands are provided via the master input device 194, so that the operator 24 feels resistance representing the direction and magnitude of forces exerted on the elongate medical devices 26 as they are moved within the vasculature of the patient 20. Under control of the processor 212, the haptic interface 214 creates and transfers the haptic feedback to the operator 24 using actuators (not shown). For example, motors capable of translating motor torque to a resistance (counterforces and vibrations) of controller motion to allow the operator 24 to feel when the elongate medical devices 26 are become difficult to move. Other examples of translating tactile feedback to the operator 24 include using weight, pneumatic, magnetic, electrical artificial muscles (electroactive polymers), piezo electric, ultrasound, pressure actuators, etc.

The actual forces exerted on the elongate medical devices 26 may be sensed via any arrangement of tactile feedback sensors 216 associated with the catheter tracking and management system 14, and provided as tactile feedback to the processor 212, which will accordingly control the haptic interface 214 to provide the tactile feedback to the operator 14.

For example, a change of current can be detected in the motors of the respective shuttles 72a-72c to which the elongate medical devices 26a-26c are mechanically coupled. That is, as forces are applied to the distal ends of the elongate medical devices 26a-26c, the electrical current in the motors that drive the elongate medical devices 26a-26c will accordingly change. As another example, active force sensors (e.g., electrical, piezoelectrical, magnetic, resistive, capacitive, pressure, hydraulic, and the like) may be incorporated into the distal tips and/or along the lengths of the elongate medical devices 26a-26b. As still another example, forces exerted on the distal ends of the elongate medical devices 26a-26b and mechanically transmitted to the respective anti-buckling mechanisms 132 (illustrated in **Fig. 6****)** may be sensed within the axial split sheaths 136 via deflection detectors that measure deflections of the axial split sheaths 136 or force or pressure detectors that directly measure force or pressure in the axial split sheaths 136 in response to compressive forces exerted at the distal ends of the elongate medical devices 26a-26b, or by force detectors or pressure detectors. In an alternative embodiment, a medical imager (e.g., a fluoroscopic imager) may provide visual feedback of forces exerted on the elongate medical devices 26a-26b to the processor 212, which will accordingly control the haptic interface 214 to provide the tactile feedback to the operator 14. For example, the medical imager may detect contact between the distal tips of the elongate medical devices 26a-26c and a vessel wall or lesion, or may detect bending of the elongated medical devices 26a-26c. The processor 212 may then use this visual feedback to estimate the forces exerted on the elongate medical devices 26a-26c.

As will be discussed in further detail below, rather than being incorporated into the master input device 194, the haptic interface 214 may alternatively have a wearable form factor, such as rings, gloves, finger tip pads, wrist bands, etc., or may even generate non-contact force fields using, e.g., ultrasound pads, air pressure-based feedback, fluid viscosity change-based feedback, etc. In conjunction with, or instead of, the haptic interface 214, the control panel 196 or other graphical displays may also display force build up in the elongate medical devices 26 as they are being moved via the master input device 194.

The master input device 194 optionally comprises an identifier (ID) 218 (e.g., a barcode, QR code, or radiofrequency identifier (RFID)) affixed to each of the control elements 200a-200c (or alternatively, each of the sterile covers 208a-208c disposed over the control elements 200a-200c) for allowing quick identification of the type of elongate medical device 26 (e.g., either guide sheath 26a, a working catheter 26b, or a guidewire 26c) with which the respective control element 200 is associated. In another embodiment, each control element 200 may be identification (ID)-coupled with a corresponding shuttle 72 (i.e., the control element 200a and shuttle 72a are paired, the control element 200b and shuttle 72b are paired, and the control element 200c and shuttle 72c are paired) via the ID 218 and may be packaged together. In other embodiments, if the motions of a pair of elongate medical devices 26 are to be linked together to provide synchronized motion, the corresponding two of the control elements 200 may be pre-paired and packaged together with a label that identifies the ratio of the linked motion (e.g., 1:1, 2:1, 1:2, etc.) between the pair of elongate medical devices 26 that the pre-paired control elements 200 are designed to effect.

The axial translation and rotational translation (and optionally axial speed and rotational speed) of the control elements 200a-200c may be detected via a sensor assembly, such that the elongate medical devices 26a-26c may be axially translated and rotationally translated in accordance with the mapping algorithm briefly described above. The sensor assembly outputs signals indicative of the detected axial translation of each of the control elements 200a-200c along the longitudinal axis 204, and indicative of the detected rotational translation of each of the control elements 200a-200c about the longitudinal axis 204. The processor 212 may then command the endovascular management and tracking system 14 to axially translate and rotationally translate each of the elongate medical devices 26a-26c in response to the control signals output by the sensor assembly.

Notably, as discussed above, the control elements 200a-200c may be designed to be single-use and disposable. In this regard, it is desirable for the relatively inexpensive components of the sensor assembly (e.g., the passive components (i.e., components that are not powered)) reside on or within the control elements 200a-200c (or alternatively, each of the sterile covers 208a-208c disposed over the control elements 200a-200c), while the relatively expensive components (e.g., the active components (i.e., components that are powered)) of the sensor assembly reside in or on the reusable rail 202 or another location remote from the control elements 200a-200c. In this manner, only the less expensive components of the sensor assembly will be replaced, thereby decreasing the operational cost of the robotic endovascular system 10.

For example, in one embodiment illustrated in **Fig. 18A****,** the master input device 194 comprises a plurality of encoders in the form of fiducial elements 219 circumferentially disposed around each of the control elements 200a-200c (or alternatively, each of the sterile covers 208a-208c disposed over the control elements 200a-200c), and a vision tracking system 221 (e.g., one or more cameras) configured for visually sensing the fiducial elements 219 disposed on the control elements 200a-200c, and thus, the axial translation and rotational translation (and optionally axial speed and rotational speed) of each of the control elements 200a-200c.

In another embodiment illustrated in **Fig. 18B****,** the master input device 194 comprises a plurality of electromagnetic passive transponders 223, each of which is incorporated into a respective one of the control elements 200a-200c (or alternatively, each of the sterile covers 208a-208c disposed over the control elements 200a-200c), and a stationary electromagnetic transceiver 225 configured for transmitting electromagnetic energy 227 that impinges on the electromagnetic passive transponders 223, which encode the electromagnetic energy with positional information, and in particular, with the axial translations (z-locations) and rotational translations (rolls) of the electromagnetic passive transponders 223, and thus, the control elements 200a-200c. The electromagnetic transceiver 225 then receives the encoded electromagnetic energy 229 emitted by the electromagnetic passive transponders 219, which electromagnetic energy 229 can then be decoded to determine the axial translations and rotational translations of the control elements 200a-200c. Optionally, in the case where the rail 202 is configured for flexing, such that each of the control elements 200a-200c may linearly translated along the x- and y-directions, in addition to the z-direction, and can be pitched or yawed, in addition to be rolled, the positional information encoded into the emitted electromagnetic energy 229 by the electromagnetic passive transponders 223 may additionally comprise the x- and y-locations, as well as the pitch and yaw, of the electromagnetic passive transponders 219, and thus, the control elements 200a-200c, such that six degrees of freedom (DOF) (i.e., x-, y-, and z-locations and roll, pitch, and yaw) of the control elements 200a-200c can be determined.

In still another embodiment illustrated in **Fig. 18C****,** the master input device 194 comprises an active array of sensors 231 disposed within, and along the longitudinal axis 204 of, the rail 202, and a plurality of complementary passive (inactive) elements 233, each of which is disposed within a respective one of the control elements 200a-200c (or alternatively, each of the sterile covers 208a-208c disposed over the control elements 200a-200c). Each of the sensors 231 are located adjacent a respective one of the passive elements 233, such that the sensors 231 may sense the locations of the respective passive elements 233, and thus, the axial translation and rotational translation of the control elements 200a-200c.

In one specific embodiment, the control elements 200a-200c may be composed of an optically transparent material, and the passive elements 233 may take the form of a series of engravings (e.g., a grating) embedded around the control elements 200a-200c. A laser light may be passed through the gratings within the control elements 200a-200c, e.g., from a laser source (not shown) disposed within the rail 202. The gratings encode the laser light with positional information via diffraction, and in particular, with the axial translations (z-locations) and rotational translations (rolls) of the control elements 200a-200c. The sensors 231 may be optical detectors that measure the encoded laser light diffracted by the gratings, which diffracted laser light can then be decoded to determine the axial translations and rotational translations of the control elements 200a-200c.

In other specific embodiments, the passive elements 233 may be resistors, in which case, the sensors 231 may electrically sense the locations of the respective passive elements 233, and thus, the axial translation and rotational translation of the control elements 200a-200c; or may be ferric elements, in which case, the sensors 231 may magnetically sense the locations of the respective passive elements 233; or may be coils, in which case, the sensors 231 may inductively sense the locations of the respective passive elements 233; or may be capacitive plates, in which case, the sensors 231 may capacitively sense the locations of the respective passive elements 233; or may be ultrasonically reflective elements, in which case, the sensors 231 may ultrasonically sense the locations of the respective passive elements 233; and so forth.

In yet another embodiment illustrated in **Figs. 18D-18F****,** the master input device 194 comprises a plurality of mechanical arms 235a-235c, each of which is cantilevered to an end of a respective one of the control elements 200a-200c, and a sensor box 237 comprising sensors (not shown) in which the free ends of the mechanical arms 235a-235c operatively interact (e.g., optically, electrically, magnetically, inductively, capacitively, ultrasonically, etc.) within the sensor box 237. The respective lengths of the mechanical arms 235a-235c depend upon the relative locations of the control elements 200a-200c on the rail 202. That is, the length of the mechanical arm 235a cantilevered to the distal-most control element 200a will be relatively short, the length of the mechanical arm 235c cantilevered to the proximal-most control element 200c will be relatively long, and the length of the mechanical arm 235b cantilevered to the medial control element 200b will be relatively moderate. So that the mechanical arms 235a-235c do not mechanically interfere with each other when any of the control elements 200a-200c are rotated about the longitudinal axis 204 of the rail 202, the mechanical arms 235a-235c are clocked from each other (e.g., 120 degrees apart from each other) and have a limited rotational range (e.g., 120 degrees), as best illustrated in **Fig. 18F****.** In the case where the maximum number of control elements 200 that can be located on the rail 202 are more or less than three, the mechanical arms 231 may be clocked from each other a different amount and may have a different limited range (e.g., 180 degrees if the maximum number of control elements 200 that can be located on the rail 202 is two, and 90 degrees if the maximum number of control elements 200 that can be located on the rail 202 is four).

In any of the embodiments illustrated in **Figs. 18A-18F****,** relatively inexpensive accelerometers (not shown) may optionally be incorporated into each of the control elements 200a-200c, such that the linear speeds and/or angular speeds of the control elements 200a-200c may be determined.

In yet another embodiment illustrated in **Fig. 18G****,** instead of having physical control elements 200a-200c, the master input device 194 comprises a three-dimensional (3D) touchscreen 239 that displays interactive 3D representations of a linear array of virtual control elements 200a'-200c'. In the illustrated embodiment, the 3D touchscreen 239 is cylindrical or semi-cylindrical on which arcuate portions of the linear array of virtual control elements 200a'-200c' are displayed. In alternative embodiments, the 3D touchscreen 239 may have cross-sectional shapes other than circular or arcuate, including, polygonal, such as, e.g., triangular, rectangular, hexagonal, octagonal, etc.

In the illustrated embodiment, the touchscreen 239 is cylindrical or semi-cylindrical that displays at least arcuate portions of a linear array of control icons 200a'-200c'. The arcuate touchscreen 239 may be, e.g., resistive or capacitive, and may have, e.g., an organic light emitting diode (OLED) touch sensitive surface. The surface of the 3D touchscreen 239 is separated into distinct illuminated regions corresponding to the control icons 200a'-200c'. One of the control icons 200a'-200c' may be touched to allow the operator 24 to manipulate that control icon. In the illustrated embodiment, the distinct illuminated regions having outlines, such that the operator 24 can easily distinguish the control icons 200a'-200c' from each other. In other embodiments, the distinct regions may be illuminated with different color or patterns.

Thus, while grasping a distinct region of the 3D touchscreen 239 corresponding to one of the control icons 200a'-200c' in a similar manner as grasping one of the physical control elements 200a-200c illustrated in **Fig. 18****,** the operator 24 may move their hand along the longitudinal axis of the 3D touchscreen 239 and/or around the circumference of the 3D touchscreen 239, which emulates the axial translation and/or rotational translation of a physical control element. As the operator 24 moves their hand along the longitudinal axis of the 3D touchscreen 239, the selected one of the control elements 200a'-200c' may be displayed statically on the 3D touchscreen 239 or may be dynamically displayed as linearly moving along the 3D touchscreen 239 with the hand of the operator 24, thereby providing a better indication of the axial locations of the elongate medical devices 26a-26c relative to each other.

The axial and rotational hand movements of the operator 24 may be sensed by the 3D touchscreen 239, such that the elongate medical devices 26a-26c may be axially translated and rotationally translated in accordance with a mapping algorithm having the sensed axial translation and rotational hand movements of the operator 24 as inputs, and the operation of the motors (not shown) contained with the respective shuttles 72a-72c as outputs, as will be described in further detail below.

In an alternative embodiment illustrated in **Fig. 18H****,** rather than displaying arcuate control icons 200a'-200c' that can be effectively grasped by the operator 24, the 3D touchscreen 239 may display control icons 200a"-200c" (e.g., rectangles) that are designed to be moved using a fingertip of the operator 24. For example, any of the control icons 200a"-200c" may be moved along the axis of the 3D touchscreen 239. For example, while touching one of the control icons 200a"-200c" with a fingertip, the operator 24 may move their hand along the longitudinal axis of the 3D touchscreen 239 and/or around the circumference of the 3D touchscreen 239, which emulates the axial translation and/or rotational translation of a physical control element.

Although the control station 16, including the master input device 194 and control panel 196, have been described and illustrated as being physical devices that exists in the real world, and thus, can be physically touched by the operator 24, in alternative embodiments, one or both of a master input device and control panel of the control station 16 may be virtual. As such, the operator 24 need not have to touch a physical master input device or control panel, thereby mitigating sanitation and spatial issues.

For example, with reference to **Fig. 18I****,** the control station 16 may comprise a head-mounted extended reality (XR) device (e.g., a virtual reality (VR), augmented reality (AR), or mixed reality (MR) device) 241 configured for presenting digitally reproduced images to the operator 24 in a three-dimensional (3D) environment, such that the operator 24 perceives the digitally reproduced images as real. In particular, the XR device 241 is configured for displaying a virtual master input device 194' having virtual control elements 200a'-200c' in the 3D environment of the operator 24. Thus, the operator 24 may virtually interact with the virtual control elements 200a'-200c' of the virtual master input device 194' as if the operator 24 were physically interacting with the control elements 200a-200c of the master input device 194 illustrated in **Fig. 18****.**

The XR device 241 may also display a control panel (not shown) as a two-dimensional (2D) image, as well as real-time medical imaging (e.g., a fluoroscopic image) of the vasculature target site of interest in the patient 20 and video of the hospital room environment, including the endovascular management and tracking system 14', e.g., in the corner of the screen of the XR device 241. Alternatively, the XR device 241 may display a virtual control panel (not shown) in the 3D environment of the operator 24. In other embodiments, the XR device has an "optical see-through" display through which the operator 24 can directly view the hospital room environment, or alternatively, has a "video see-through" display on which video of the hospital room environment intermixed with virtual content, including the virtual master input device 194' (with or without the rail), is presented.

The control station 16 further comprises haptic feedback gloves 243 (e.g., HaptX^{®} gloves) that can be worn on the hands of the operator 24 for capturing the hand gestures made by the operator 24 while virtually interacting with the virtual control elements 200a'-200c' of the virtual master input device 194'. Such hand gestures may include, e.g., grasping one of the virtual control elements 200a'-200c' with the hand of the operator 24, and moving the grasping hand along the axis of the virtual master input device 194' or rotating the grasping hand about the axis of the master input device 194' in the same manner as the operator 24 may physically grasp one of the physical control elements 200a-200c and move the physical control element along the axis of the physical master input device 194 or rotating the grasped hand about the axis of the physical master input device 194 of **Fig. 18****.** The captured hand gestures can be output by the MR device 241 to the processor 212, which may interpret the hand gestures and issue commands to the endovascular management and tracking system 14 in accordance with a mapping algorithm that maps the interpreted hand gestures to the desired axial translation and rotational translation of the elongate medical devices 26a-26c. The haptic feedback gloves 243 also serves as the haptic interface 214 (illustrated in **Fig. 18****)** to provide tactile feedback to the operator 24 as the operator 24 virtually interacts with the virtual control elements 200a'-200c' of the virtual master input device 194', so that the operator 24 feels resistance representing the direction and magnitude of forces exerted on the elongate medical devices 26 as they are moved within the vasculature of the patient 20.

As another example, with reference to **Fig. 18J****,** the control station 16 comprises, instead of haptic feedback gloves 243, a stationary gesture monitoring system 245 (e.g., a device similar to the sensor of the Xbox Kinect^{®} or a light detection and ranging (LIDAR) sensor assembly), and one or more haptic ultrasound pads 247. The gesture monitoring system 245 is configured for capturing the hand gestures made by the operator 24 while virtually interacting with the virtual control elements 200a'-200c' of the virtual master input device 194', and for providing the captured hand gestures to the processor 212 for interpretation. In an alternative embodiment, the XR device 241 comprises forward-facing cameras (not shown) configured for capturing images of the hand gestures made by the operator 24 while virtually interacting with the virtual control elements 200a'-200c' of the virtual master input device 194', and for providing the captured images of the hand gestures to the processor 212 for interpretation. In an optional embodiment, the operator 24 may wear gloves (not shown) covered with fiducial elements that can be more easily sensed by the stationary gesture monitoring system 245 or alternatively the forward-facing cameras of the XR device 241 to capture the hand gestures made by the operator 24.

The haptic ultrasound pad(s) 247 serve as the haptic interface 214 (illustrated in **Fig. 18****)** by emitting ultrasound pressure waves that exert force on the hands of the operator 24, thereby providing tactile feedback to the operator 24 as the operator 24 virtually interacts with the virtual control elements 200a'-200c' of the virtual master input device 194', so that the operator 24 feels resistance representing the direction and magnitude of forces exerted on the elongate medical devices 26 as they are moved within the vasculature of the patient 20. In the illustrated embodiment, multiple ultrasound pads 247 are oriented in different orientations to provide directionally for the tactile feedback provided to the operator 24.

As still another example, with reference to **Fig. 18K****,** the control station 16 comprises, instead of an XR system, a two-dimensional (2D) display screen 249 that displays a virtual master input device 194' and optionally a virtual control panel (not shown). The stationary gesture monitoring system 245 captures the hand gestures made by the operator 24 while the operator 24 views the master input device 194 displayed on the display screen 249, and provides the captured hand gestures to the processor 212 for interpretation. In addition to issuing commands to the endovascular management and tracking system 14, the processor 212 may augment the master input device 194 in response to the captured hand gestures made by the operator 24, e.g., by moving the displayed control elements 200a-200c in accordance with the hand gestors or otherwise augmenting the displayed control elements 200a-200c, e.g., with different colors.

As yet another example, with reference to **Fig. 18L****,** the control station 16 comprises, instead of an XR system, a holographic machine 251 configured for displaying a virtual master input device 194' having virtual control elements 200a'-200c', and optionally a virtual control panel, in the 3D environment of the operator 24. Instead of displaying the virtual master input device 194' on a head-mounted display like the XR system, the holographic machine 251 projects the virtual master input device 194' into the 3D environment of the operator 24. The operator 24 may interact with the virtual control elements 200a'-200c' of the virtual master input device 194' in the same manner discussed above with respect to the XR device 241 of **Fig. 18I****.** Hand gestures may be captured by, e.g., haptic feedback gloves 243 (shown in **Fig. 18I****)** worn on the hands of the operator 24 or the stationary gesture monitoring system 245 (shown in **Fig. 18J****).** Tactile feedback can be provided to the operator 24 by, e.g., haptic feedback gloves 243 (shown in **Fig. 18I****)** worn on the hands of the operator 24 or the haptic ultrasound pad(s) 247 (shown in **Fig. 18J****).**

These nominal axial translation and rotational translation positions are preferably in the centers of the axial translation ranges and rotational translation ranges, such that the control elements 200a-200c may have positive and negative travel ranges from the nominal positions (i.e., a forward axial translation range, a reverse axial translation range, a clockwise rotational translation range, and a counterclockwise rotational translation range).

In much the same manner as the physical control elements 200a-200c described above, each of the virtual control elements 200a'-200c' in any of the embodiments described above may be virtually axially and rotationally spring-loaded, such that they return to their nominal axial translation and rotational translation positions when virtually released by the operator 24. These nominal axial translation and rotational translation positions are preferably in the centers of the axial translation ranges and rotational translation ranges, such that the control elements 200a'-200c' may have positive and negative travel ranges from the nominal positions (i.e., a forward axial translation range, a reverse axial translation range, a clockwise rotational translation range, and a counterclockwise rotational translation range).

It should be appreciated that although the master input devices 194, 194' illustrated in **Figs. 18A-18L** have been described as controlling the catheter tracking and management system 14 illustrated in **Figs. 1-17****,** the master input devices 194, 194' illustrated in **Figs. 18A-18L** may be employed to control any robotic catheter system, wherein the elongate medical devices are coaxially arranged and may axially and rotationally translate relative to each other, including those that do not have shuttles that translate along a track.

As briefly discussed above, the processor 212 may issue commands to the endovascular management and tracking system 14 in accordance with a mapping algorithm that maps the sensed axial translation and rotational translation of the control elements 200a-200c (or virtual control elements 200a'-200c') to the desired axial translation and rotational translation of the elongate medical devices 26a-26c. That is, in response to an input comprising the axial translation or rotational translation of the control elements 200a-200c (or virtual control elements 200a'-200c'), the mapping algorithm outputs the desired axial translation or rotational translation of the elongate medical devices 26a-26c. The processor 212 may then translate the output of the mapping algorithm into commands that are sent to the endovascular management and tracking system 14, and in particular, the motors of the endovascular management and tracking system 14 to facilitate these desired axial translations or rotational translations.

As also briefly discussed above, the control elements 200a-200c may be axially and rotationally spring-loaded, such that they return to their nominal axial translation and rotational translation positions when physically released by the operator 24, or the virtual control elements 200a'-200c' may be virtually axially and rotationally spring-loaded, such that they return to their nominal axial translation and rotational translation positions when virtually released by the operator 24. In this embodiment, the mapping algorithm will only axially translate (proximally or distally) or rotationally translate (clockwise or counterclockwise) each of the elongate medical devices 26a-26b in response to axial translation or rotationally translation of the corresponding control element 200 away from their neutral positions (i.e., against the physical spring force or virtual spring force). As such, axially translation or rotational translation of the elongate medical device 26a-26c will cease to occur in response to the operator 24 physically releasing the control element 200a-200c or virtually releasing the virtual control elements 200a'-200c'. By repeatedly axially translating or rotationally translating and then releasing a control element 200 or by repeatedly virtually axially translating or rotationally translating and then virtually releasing a virtual control element 200', the corresponding elongate medical device 26 may be incrementally axially translated or rotationally translated.

In one embodiment, the mapping algorithm outputs an axial translation for each elongate medical device 26 in response to an input of an axial translation of the corresponding control element 200 (or virtual control element 200') in accordance with an axial translation ratio, and likewise outputs a rotational translation for each elongate medical device 26 in response to an input of a rotational translation of the corresponding control element 200 (or virtual control element 200') in accordance with a rotational translation ratio. In general, the axial translation ratio and the rotational translation ratio will be positive. That is, the mapping algorithm will output an axial translation for each elongate medical device 26 in the distal direction in response to an input of an axial translation of the corresponding control element 200 (or virtual control element 200') in the distal direction, and will output an axial translation for each elongate medical device 26 in the proximal direction in response to an input of an axial translation of the corresponding control element 200 (or virtual control element 200') in the proximal direction. Likewise, the mapping algorithm will output a rotational translation for each elongate medical device 26 in the clockwise direction in response to an input of a rotational translation of the corresponding control element 200 (or virtual control element 200') in the clockwise direction, and will output a rotational translation of each elongate medical device 26 in the counterclockwise direction in response to an input of a rotational translation of the corresponding control element 200 (or virtual control element 200') in the counterclockwise direction.

It is desirable that the rotational translation ratio be 1:1 or unity (i.e., rotational translation for each elongate medical device 26 will exactly track the rotational translation of the corresponding control element 200 (or virtual control element 200'). In contrast, it is desirable that the axial translation ratio be >1:1 or less than unity (i.e., axial translation for each elongate medical device 26 will be less the axial translation of the corresponding control element 200 (or virtual control element 200'). For example, if the corresponding control element 200 (or virtual control element 200') is axially translated 1 cm, the elongate medical device 26 may axially translate 1 mm (i.e., 10:1 ratio). In alternative embodiments, however, the rotational translation ratio may be different than unity, while the axial translation ratio may be 1:1 or unity.

Either or both of the axial translation ratio and rotational translation ratio may be constant, such that there is a linear relationship between the input of the axial translation or rotational translation of a control element 200 or virtual control element 200' into the mapping algorithm and the respective axial translation or rotational translation of the corresponding elongate medical device 26 output by the mapping algorithm. Alternatively, either or both of the axial translation ratio and rotational translation ratio may vary over time, such that there is non-linear relationship between the axial translation or rotational translation of a control element 200 or virtual control element 200' input into the mapping algorithm and the respective axial translation or rotational translation of the corresponding elongate medical device 26 output by the mapping algorithm. For example, it may be desirable for the axial translation ratio or rotational translation ratio to be initially higher (e.g., to quickly overcome in static frictional forces between the elongate medical device 26 and the vasculature) and then decrease. As another example, the axial translation ratio or rotational translation ratio may vary in accordance with a custom profile that is designed for the particular operator 24. Such custom profile may be manually generated, or alternatively, such custom profile may be generated via a calibration procedure or may be dynamically adapted to the particular operator 24 over a period of time.

The mapping algorithm may modulate either or both of the axial translation ratio and rotational translation ratio, e.g., to ensure that the elongate medical devices 26a-26c are axially translated or rotationally translated in a smooth manner. For example, any tremor in the hands of the operator 24 may cause a control element 200 or virtual control element 200' to axially translate or rotationally translate in a disjointed manner. The mapping algorithm may smooth out (e.g., via integration) the input of the disjointed axial translation or rotational translation of the control element 200 or virtual control element 200', such that the output of the desired axial translation or rotational translation of the corresponding elongate medical device 26 is smooth. As another example, the mapping algorithm may introduce small amplitude axial or rotational vibrations into the input of the axial translation or rotational translation of the control element 200 or virtual control element 200', such that the output of the desired axial translation or rotational translation of the corresponding elongate medical device 26 has small amplitude axial or rotational vibrations, e.g., to avoid static frictional forces between the elongate medical device 26 and the vasculature of the patient 20, thereby facilitating distal advancement of the elongate medical device 26.

In another embodiment, rather than using an axial translation ratio or a rotational translation ratio, the mapping algorithm may output an axial velocity for each elongate medical device 26 in response to an input of an axial translation of the corresponding control element 200 (or virtual control element 200'), and likewise outputs a rotational velocity for each elongate medical device 26 in response to an input of a rotational translation of the corresponding control element 200 (or virtual control element 200'). In general, the axial velocity will be in the same direction as the axial translation of the corresponding control element 200 (or virtual control element 200'), while the rotational velocity will be in the same direction as the rotational translation of the corresponding control element 200 (or virtual control element 200'). That is, the mapping algorithm will output an axial velocity for each elongate medical device 26 in the distal direction in response to an input of an axial translation of the corresponding control element 200 (or virtual control element 200') in the distal direction, and will output an axial velocity for each elongate medical device 26 in the proximal direction in response to an input of an axial translation of the corresponding control element 200 (or virtual control element 200') in the proximal direction. Likewise, the mapping algorithm will output a rotational velocity for each elongate medical device 26 in the clockwise direction in response to an input of a rotational translation of the corresponding control element 200 (or virtual control element 200') in the clockwise direction, and will output a rotational velocity for each elongate medical device 26 in the counterclockwise direction in response to an input of a rotational translation of the corresponding control element 200 (or virtual control element 200') in the counterclockwise direction.

In one embodiment, the mapping algorithm outputs a uniform axial velocity or uniform rotational velocity for each elongate medical device 26 in response to any axial translation or rotational translation of the corresponding control element 200 (or virtual control element 200'). In another embodiment, the mapping algorithm outputs an axial velocity or rotational velocity for each elongate medical device 26 that varies depending on the extent or the speed at which the corresponding control element 200 (or virtual control element 200') is axially translated or rotationally translated. For example, if the control element 200 (or virtual control element 200') is axially translated or rotationally translated a relative long distance or relatively quickly, the mapping algorithm may output an axial velocity or rotational velocity of the corresponding elongate medical device 26 that is relatively high. In contrast, if the control element 200 (or virtual control element 200') is axially translated or rotationally translated a relative short distance or relatively slow, the mapping algorithm may output an axial velocity or rotational velocity of the corresponding elongate medical device 26 that is relatively low. In still another embodiment, the mapping algorithm outputs an axial velocity or rotational velocity for each elongate medical device 26 that varies in accordance with a custom profile that is designed for the particular operator 24. Such custom profile may be manually generated, or alternatively, such custom profile may be generated via a calibration procedure or may be dynamically adapted to the particular operator 24 over a period of time.

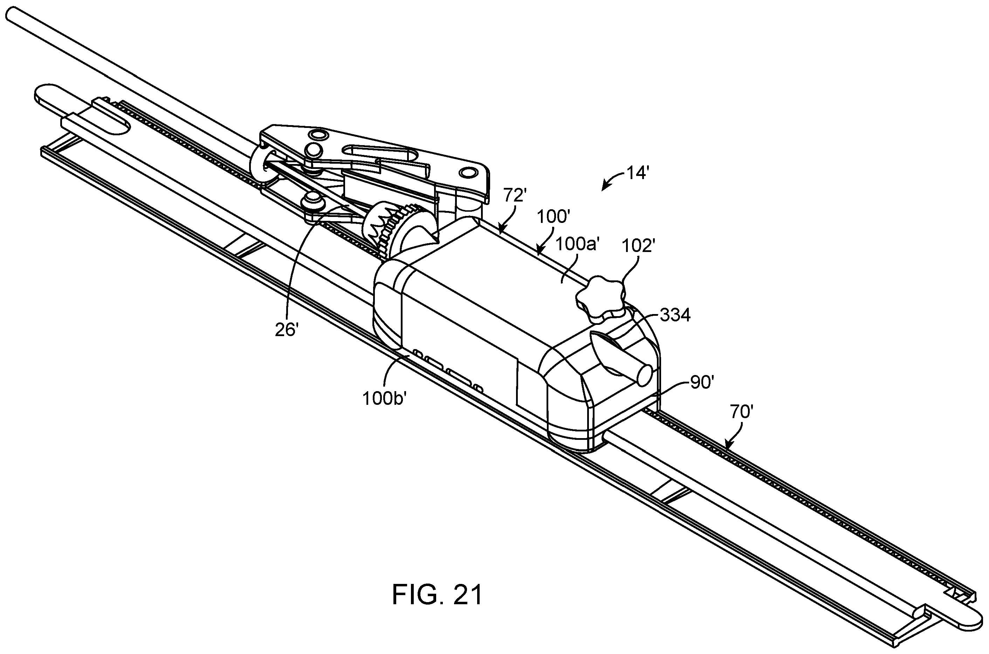

Referring to **Figs. 21-24****,** one detailed embodiment of the endovascular management and tracking system 14' will be described. As with the catheter and tracking system 14 illustrated in **Fig. 6****,** the endovascular management and tracking system 14 generally comprises a plurality of track segments 70' (only one shown) and a plurality of shuttles 72' (only one shown) configured for being mechanically coupled to and axially translated along the track segments 70'.

Although only one track segment 70' is illustrated in **Figs. 21-24****,** it should be appreciated that each track segment 70' is specifically designed, such that multiple track segments 70' may be removably coupled to each other to create a track having a variable length. Thus, a track may be lengthened by adding one or more track segments 70', and shortened by subtracting one or more track segments 70'. As such, the track may be better conformed to the hospital room environment. For example, for the particular hospital environment in which the endovascular management and tracking system 14' is to be set up, the total length of the track may be customized to the contoured distance between the control station 16 (shown in **Figs. 1-2****)** (or otherwise an anchor to which the proximal end of the track is to be affixed) and the sheath anchor 80 (shown in **Fig. 6****)** directly or indirectly affixed to the patient 20 and to which the distal end of the track is to be affixed.

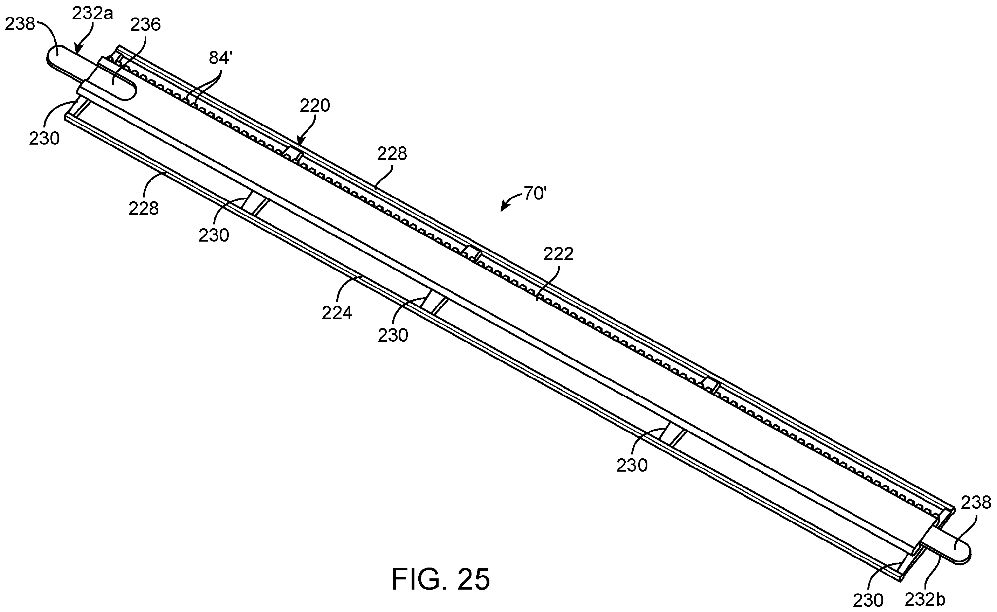

Referring further to **Figs. 25-29****,** in contrast to the track 70 illustrated in **Fig. 6****,** which is described as taking the form of a simple rail, each track segment 70' comprises a track rack 220 and a track rail 222 affixed to the track rack 220.

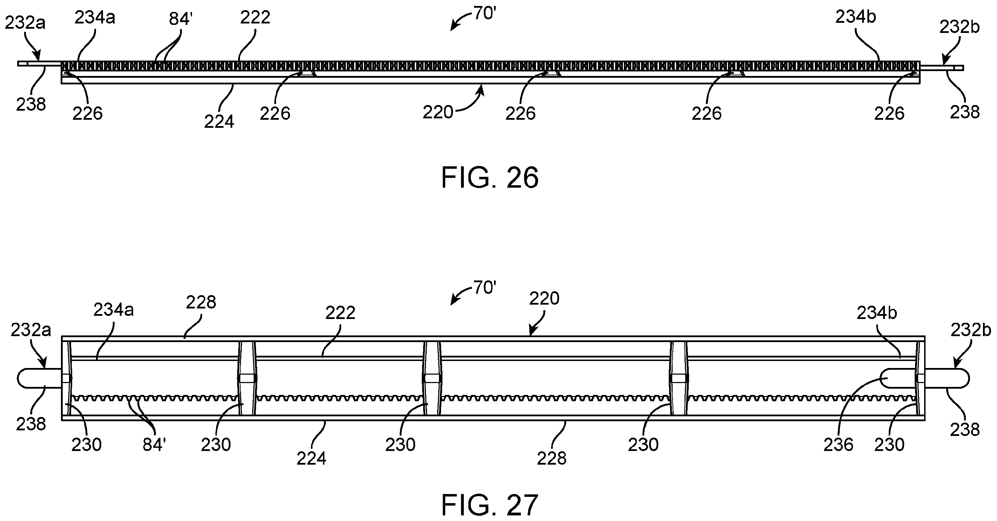

The track rack 220 comprises an elongated base 224 configured for being stably rested on the drape 22 (shown in **Figs. 1-2****)** and one or more pedestals 226 (shown best in **Figs. 26** and **28****-29)** affixed on top of the base 224 and to which the track rail 222 is affixed. In this manner, the track rail 222 can be raised above the drape 22, such that the shuttle 72' can freely ride along the track rail 222 without impingement by the hospital room environment, and in particular, the drape 22 upon which the track segment 70' will likely lay.

In the illustrated embodiment, the base 224 takes the form of a rectangular frame having a pair of elongated frame members 228 and a series of crossbars 230 affixing the pair of elongated frame members 228 together (best shown in **Figs. 25** and **27****),** and in particular, spanning across the width of the base 224 and periodically spaced along the length of the base 224. The base 224 is planar, such that the track segment 70', like the track 70 illustrated in **Fig. 6****,** has a relatively high top-to-bottom (vertical) flexibility, but a relatively low side-to-side (horizontal) and torsional flexibility (i.e., high horizontal and torsional rigidity). In this manner, the track segment 70' may better conform to the contours of the hospital room environment solely in response to gravity (e.g., when laid down on the procedure table 18, patient 20, and/or drape 22), while allowing the shuttle 72' to apply the required drive forces to the track 70' in order to axially translate the shuttle 72' along the coupled track segment 70'. Notably, the horizontal and torsional rigidity of the track segment 70' may be adjusted without significantly modifying the vertical flexibility of the track segment 70' by varying the number of crossbars 230 that affix the elongate frame members 228 together. That is, increasing the number of crossbars 230 used to affix the elongate frame members 228 together, accordingly increases the horizontal and torsional rigidity of the track segment 70', while decreasing the number of crossbars 230 used to affix the elongate frame members 228 together, accordingly decreases the horizontal and torsional rigidity of the track segment 70'. In the illustrated embodiment, a plurality of pedestals 226 are arranged along the centers of the crossbars 230.

Like the track 70, the track rail 222 takes the form of an open monorail on which the shuttles 72', and may be a ribbon-like element (wide and thin rectangular extrusion) with a rectangular cross-section, as best illustrated in **Fig. 27****.** Like the track 70, each track segment 70' comprises at least one row of teeth 84', and in the illustrated embodiment a single row of teeth 84', extending along the length of the track rail 222 with which corresponding drive mechanisms of the shuttle 72' engage to facilitate axial translation of the shuttle 72' along the track segment 70'. As will be described in further detail below, the track rail 222 on which the teeth 84' are disposed serves as the rack in a rack-and-pinion arrangement for translation of the shuttle 72' along the track segment 70'.

As briefly discussed above, multiple track segments 70' may be removably coupled to each other. To this end, the track segment 70' comprises a pair of track couplers 232 disposed at opposite ends of each track segment 70', and in the illustrated embodiment, at opposite ends of the track rail 222 of each track segment 70'. The track couplers 232 are complementary to each other, such track segments 70' may be removably coupled to each other by engagement of corresponding couplers 232 of adjacent track segments 70'.

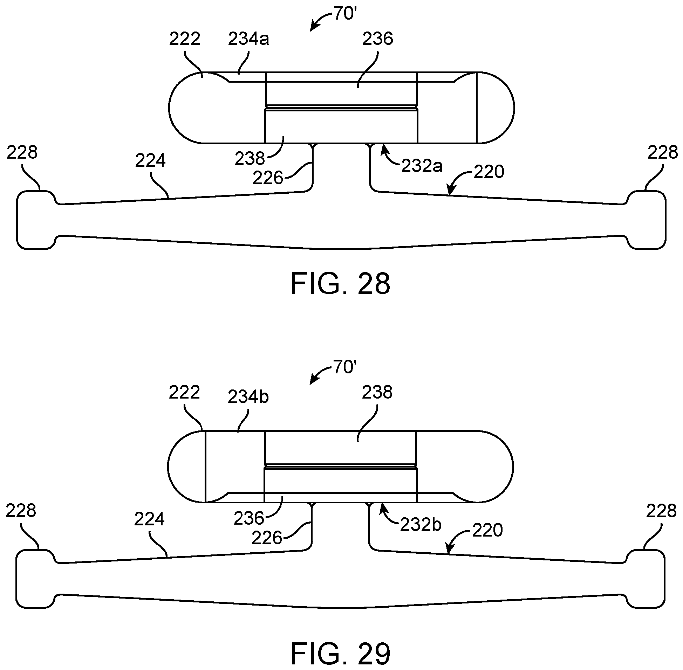

For example, in the illustrated embodiment, a track coupler 232a disposed on a proximal end 234a of the track rail 222 comprises a recess 236 (best shown in **Figs. 25** and **28****)** formed in the bottom surface of the track rail 222, and a tab 238 extending from the proximal end 234a of the track rail 222. In contrast, a track coupler 232b disposed on a distal end 234b of the track rail 222 comprises a recess 236 (best shown in **Fig. 27** and **29****)** formed in the top surface of the track rail 222, and a tab 238 extending from the distal end 234b of the track rail 222. The respective shapes of the recess 236 and tab 238 are identical. In this manner, track segments 70' may be removably coupled to each other by engagement of a corresponding recess 236 and tab 238 of adjacent track segments 70' (i.e., the tab 238 at the proximal end 234a of the track rail 222 of one track segment 70' can be engaged with (e.g., snapped into) the corresponding recess 236 at the distal end 234b of the track rail 222 of another track segment 70', as illustrated in **Fig. 28****),** thereby coupling the track segments 70' together. The track segments 70' may be decoupled from each other by disengagement of the corresponding recess 236 and tab 238 of adjacent track segments 70' (i.e., the tab 238 at the proximal end 234a of the track rail 222 of one track segment 70' can be disengaged from (e.g., snapped out of) the corresponding recess 236 at the distal end 238b of the track rail 222 of the other track segment 70', thereby decoupling the track segments 70' from each other. Alternatively, other types of corresponding track couplers 232 may be utilized to removably coupled multiple track segments together.

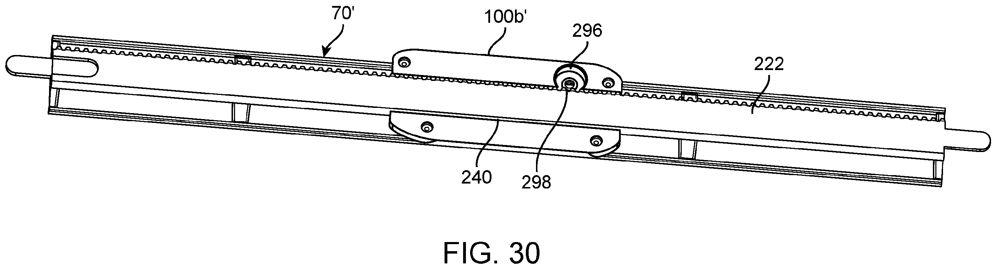

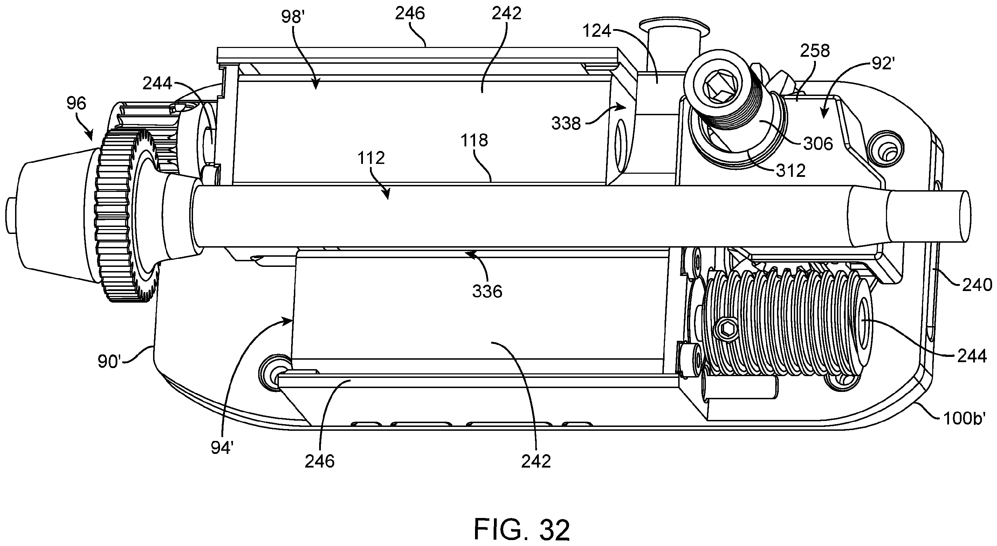

Referring to **Figs. 30-32****,** like each of the shuttles 72a-72c illustrated in **Figs. 6** and **7A****-7D,** the shuttle 72' includes a sled 90' (shown also in **Fig. 21****)** that is configured for riding on the track segment 70'; an axial drive mechanism 92' (e.g., a geared or frictional coupling) carried by the sled 90' and configured for being actuated to axially translate the shuttle 72' (and thus, the elongate medical device 26 affixed to the respective shuttle 72') along the track segment 70'; an axial actuator 94' (in this case, a motor) carried by the sled 90' and configured for actuating the axial drive mechanism 92'; a rotary drive mechanism 96' carried by the sled 90' and configured for being actuated to rotate the elongate medical device 26 affixed to the shuttle 72' about its longitudinal axis; a rotary actuator 98' (in this case, a motor) carried by the sled 90' and configured for actuating the rotary drive mechanism 96'; an anti-buckling mechanism 132' affixed to the sled 90' and configured for preventing the elongate medical device 26 from prolapsing in response to axial compression applied to the elongate medical device 26 (e.g., preventing the elongate medical device 26 from prolapsing when the shuttle 72' is axially translated in the distal direction or when a subsequent shuttle (not shown) through which the elongate medical device 26 extends is axially translated in the proximal direction); and an outer casing 100' (best shown in **Figs. 21-23****)** mounted to the sled 90' and containing one or more of the axial drive mechanism 92', axial motor 94', a rotary drive mechanism 96', a rotary motor 94' (and in this case, the axial drive mechanism 92', axial motor 94', and a rotary motor 94').

In the illustrated embodiment, the outer casing 100' comprises an upper casing portion 100a' affixed to the top of the sled 90' and a bottom casing portion 100b' affixed to the bottom of the sled 90'. As best shown in **Figs. 30-32****,** the bottom casing portion 100b' comprises an open channel 240 through which the track segment 70' is slidably received. The cross-section of the open channel 240 is closely toleranced to the cross-section of the track rail 222 of the other track segment 70', such that shuttle 72' may stably ride along the track segment 70'.

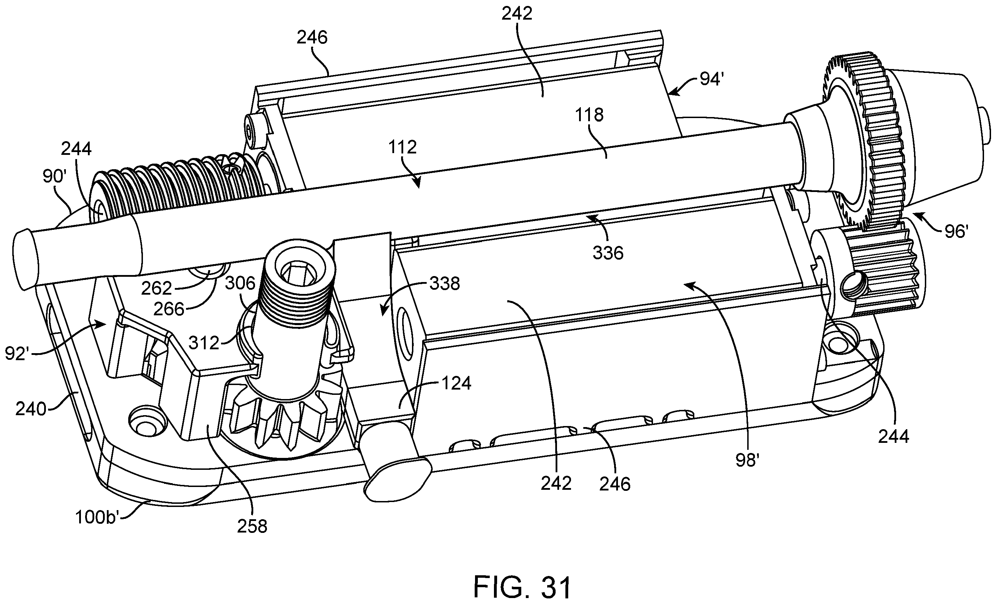

In the illustrated embodiment, each of the motors 94', 98' takes the form of a stepper motor, although in alternative embodiments, the motors 94', 98' may take the form of any motor that can be any motor that can be controlled in response to electrical control signals, such as those delivered by the control station 16 (shown in **Figs. 1** and **2****).** As best shown in **Figs. 31-32****,** each of the motors 94', 98' comprises a motor box 242, a stator, rotor, and motor electronics (not shown) contained within the motor box 242, and a motor shaft 244 affixed to the rotor within the motor box 242. The shuttle 72' further comprises motor mount brackets 246 that affix the motors 94', 98' to the sled 90'. In the illustrated embodiment, the motor mount brackets 246 and sled 90' have a unibody design, although in alternative embodiments, the motor mount brackets 246 may be directly affixed to the sled 90' via appropriate fasteners (e.g., screws) for stably supporting the respective motors 94', 98' relative to the sled 90'.

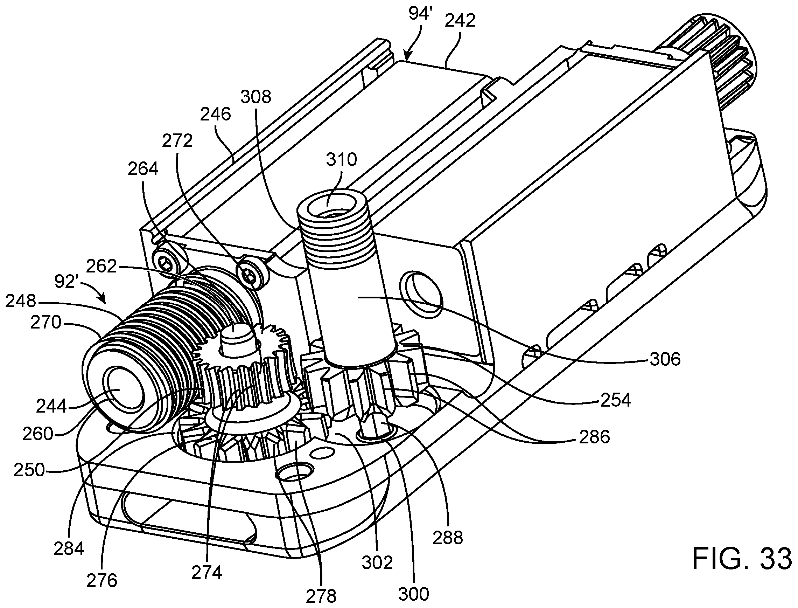

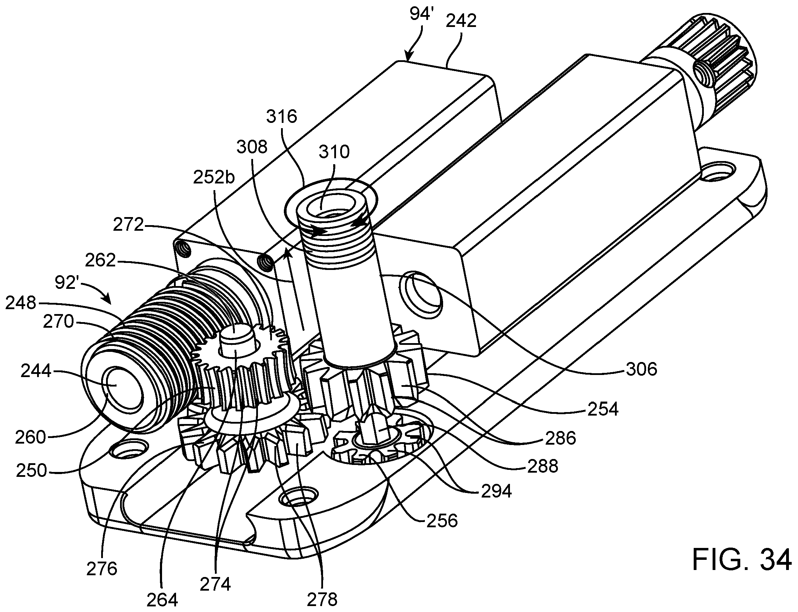

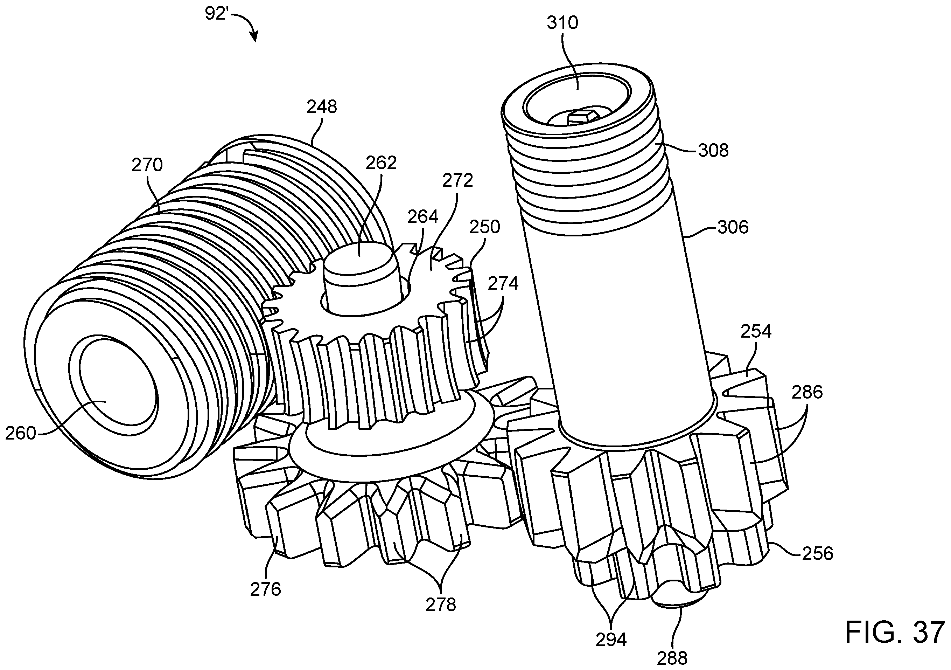

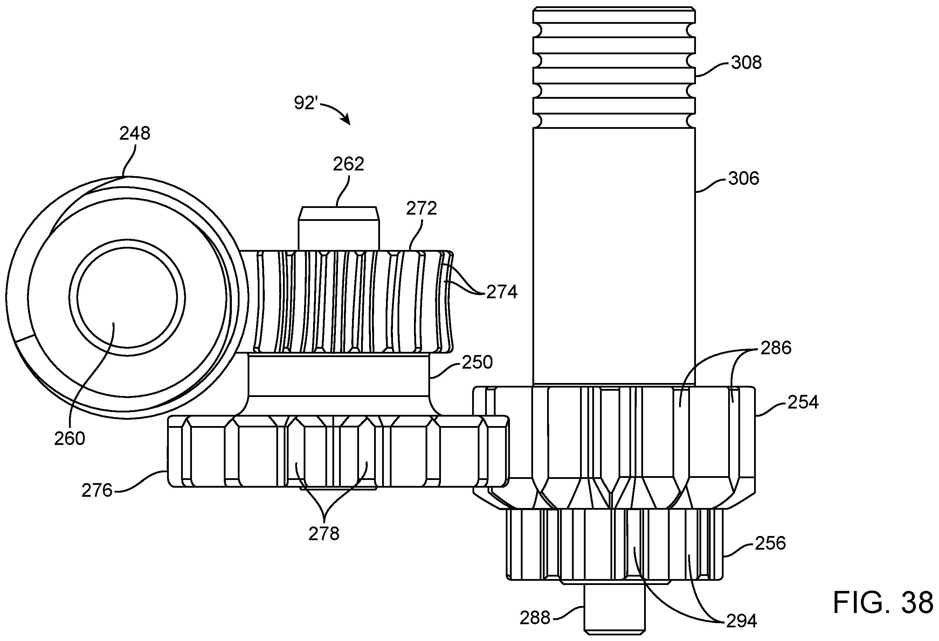

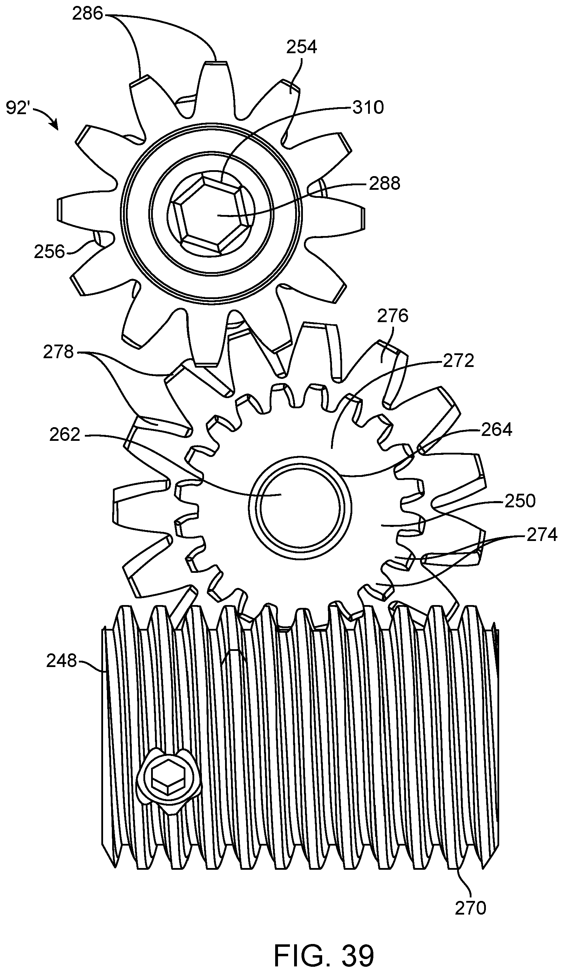

Referring now to **Figs. 33-39****,** the axial drive mechanism 92' will now be described in further detail. In this embodiment, the axial drive mechanism 92' takes the form of a drive train comprising a worm screw 248 affixed to the motor shaft 244 of the axial motor 94', a worm-to-cog gear 250 operably coupled to the worm screw 248, a cog gears 254 operably coupled to the worm-to-cog gear 250, and a pinion gear 256 (shown in **Figs. 34** and **36****-39)** operably coupled between the cog gear 254 and the teeth 84' of the track segment 70' (shown in **Figs. 25-27****)** in a rack-and-pinion arrangement. The worm screw 248 drives the worm-to-cog gear 250, which, in turn, drives the cog gear 254, which, in turn, drive the pinion gear 256, which in turn, engages the teeth 84' of the track segment 70' to axially translate the shuttle 72' along the track segment 70'. The shuttle 72' further comprises a gear support bracket 258 (shown in **Figs. 31-32** and **36)** suitably mounted to the sled 90' via fasteners (not shown) for stably integrating the axial drive mechanism 92' together.

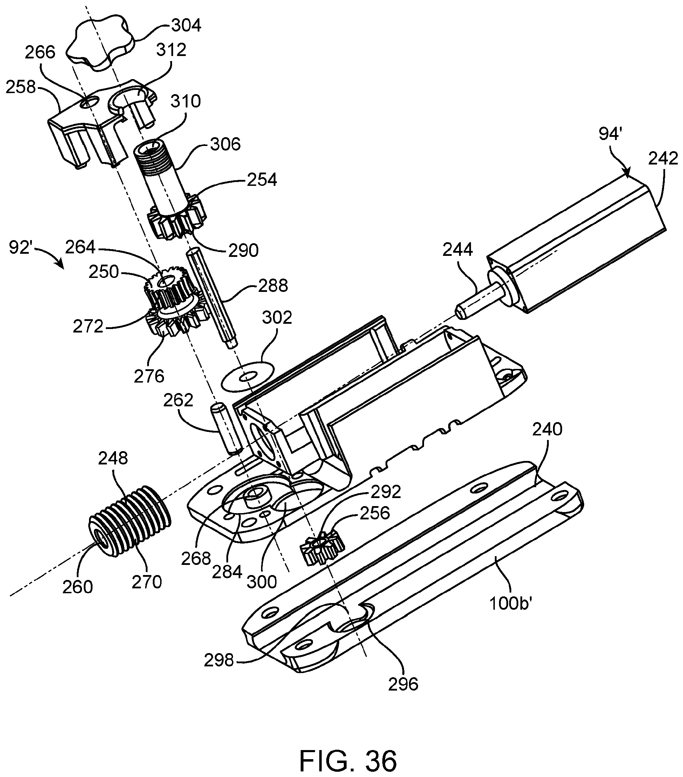

The worm screw 248 comprises a bore 260 through which the motor shaft 244 of the axial motor 94' is affixed, such that the worm screw 248 rotates with the motor shaft 244. The worm-to-cog gear 250 is affixed by the gear support bracket 258 in a manner that it rotates about an axis that is ninety degrees from the axis of the motor shaft 244. In particular, the worm-to-cog gear 250 comprises an axial shaft 262 and a bore 264 through which the axial shaft 262 is affixed. One end of the axial shaft 262 is rotatably disposed in a through-hole 266 within the gear support bracket 258 (shown in **Fig. 31** and **36****),** while the other end of the axial shaft 262 is rotatably disposed within an annular flange 268 formed on the sled 90' (shown in **Fig. 36****),** thereby providing lateral support for the worm-to-cog gear 250.

The worm screw 248 comprises a spiral thread 270 and the worm-to-cog gear 250 comprises a worm gear 272 having an arrangement of angled teeth 274 that meshes with the spiral thread 270 of the worm screw 248, such that rotation of the worm screw 248 causes rotation of the worm-to-cog gear 250 about an axis that is ninety degrees from the axis of the motor shaft 244. The worm-to-cog gear 250 further comprises a cog wheel 276 that is integrated with the worm gear 272, such that the cog wheel 276 rotates in unison with the worm gear 272. The worm gear 272 is partially seated within, and may rotate within, a circular recess 284 (shown in **Figs 33** and **36****)** formed within the sled 90' around the annular flange 268 to provide additional axial stability to the worm-to-cog gear 250.

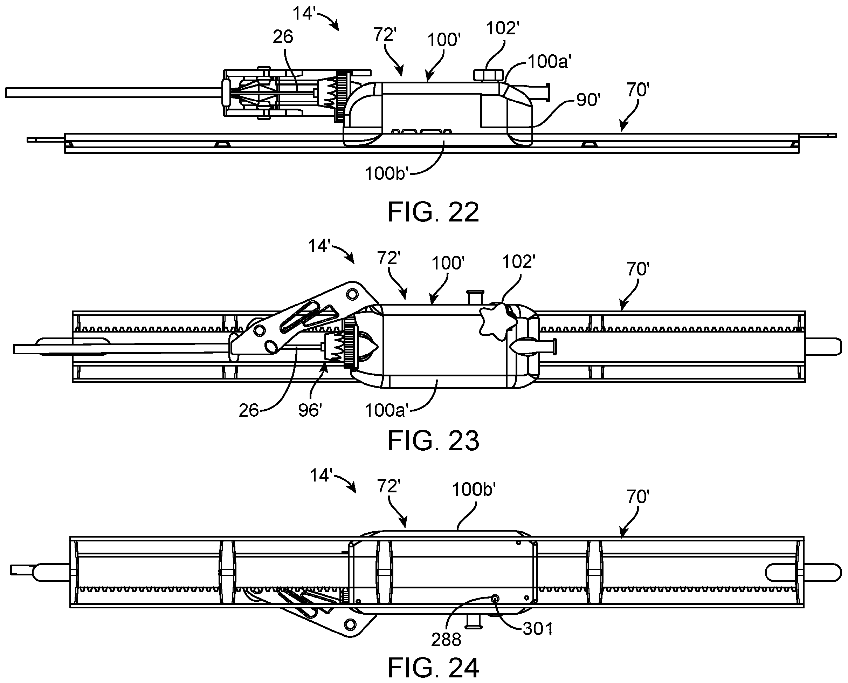

The cog gear 254 comprises an arrangement of teeth 286 that match and mesh with the teeth 278 of the cog wheel 276, such that rotation of the cog wheel 276 in one direction rotates the cog gear 254 in the opposite direction. Rotation of the cog gear 254, in turn, rotates the pinion gear 256. In particular, the cog gear 254 is mechanically coupled to the pinion gear 256 via a shaft 288 coaxially affixed to the cog gear 254 and pinion gear 256. The cog gear 254 and pinion gear 256 have respective bores 290, 292 (best shown in **Fig. 36****)** that slidably receive the shaft 288. The shaft 288 and bores 290, 292 have non-circular cross-sections, and in this case, hexagonal, to provide an interference fit between the gears 254, 256 and the shaft 288, such that rotation of the cog gear 254 correspondingly rotates the pinion gear 256. The pinion gear 256 comprises an arrangement of teeth 294 that match and mesh with the teeth 84' of the track segment 70' (shown in **Figs. 25-27****),** such that rotation of the pinion gear 256 translates the shuttle 72' in a linear direction along the track segment 70'. The pinion gear 256 is located between the sled 90' and a recess 296 formed within the bottom casing portion 100b' in alignment with the teeth 84' of the track segment 70'. An opening 298 (shown best in **Figs. 30** and **36****)** is formed between the recess 296 and the open channel 240 of the bottom casing portion 100b', thereby exposing the teeth 84' of the track segment 70' for the teeth 274 of the pinion gear 256 to engage. One end of the shaft 288 extends through a through-hole 300 (shown best in **Figs. 33** and **36****)** formed in the sled 90', and then through a through-hole (shown in **Fig. 24****)** at the bottom casing portion 100b'. The shuttle 72' comprises a thrust plate 302 (shown best in **Figs. 33** and **36****)** that resides within the through-hole 300 of the sled 90 for securely retaining the pinion gear 256 within the recess 296 of the bottom casing portion 100b'.

Like the endovascular management and tracking system 14 illustrated in Fig. 6, the endovascular management and tracking system 14' may be advantageously designed to be dynamically switched between the fully automated modality and a fully manual modality. To this end, the shuttle 72 comprises a manual clutched axial actuator 102' (shown in **Figs. 21-23****)** that is similar to the manual axial actuator 102 of each of the shuttles 72a-72c illustrated in **Figs. 7A-7D****,** in that the manual axial actuator 102' may be manually operated to axially index the shuttle 72', such that axial movement of the shuttle 72' along the track segment 70' may be finely controlled. However, the manual clutched axial actuator 102' is additionally configured for being operated to alternately engage and disengage at least a portion of the axial drive mechanism 92' to and from the track 70, and in this case, by alternately engaging and disengaging the complementary gears 254, 256 from the each other.

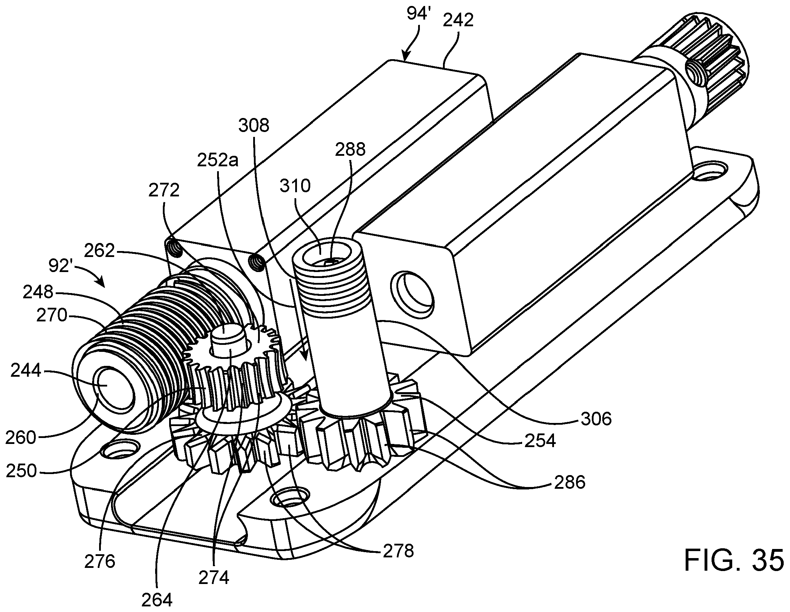

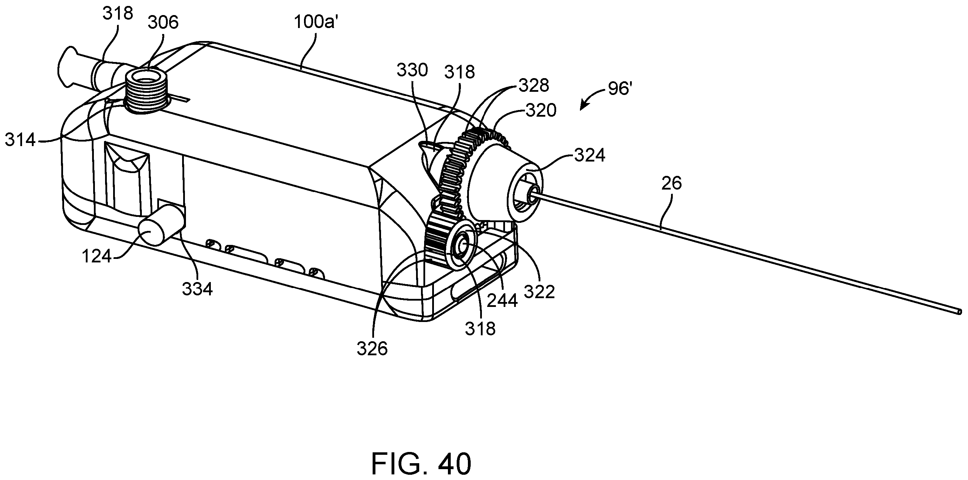

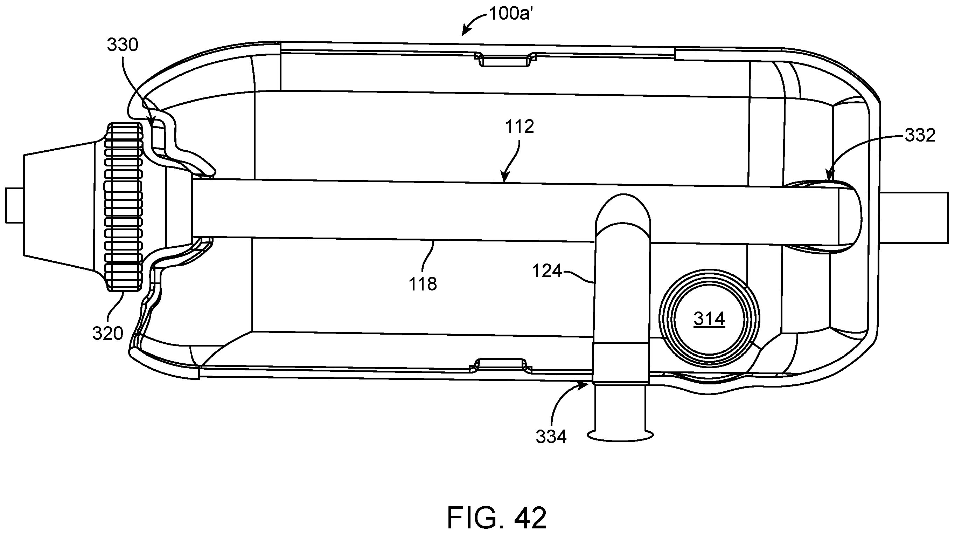

In particular, the manual clutched axial actuator 102' comprises a knob 304 (shown best in **Fig. 36****)** and a sleeve 306 comprises a bore 310 that is extensive with the bore 290 of the cog gear 254, and through which the shaft 288 extends, such that the sleeve 306 may be slidably disposed over the shaft 288. The sleeve 306 is mechanically coupled between the knob 304 and the cog gear 254. For example, the sleeve 306 and cog gear 254 may be formed as a unibody design, whereas the knob 304 may be affixed to the sleeve 306 via a threaded arrangement 308. As best shown in **Figs. 31-32****,** the sleeve 306 may extend through a through-opening 312 formed in the gear support bracket 258 and then, a through-opening 314 (shown in **Figs. 40** and **42****-43)** formed in the upper casing portion 100a', thereby providing lateral support to the sleeve 306, and by extension, to the shaft 288. In this manner, the knob 304 may be axially displaced to alternately engage (see **Fig. 35****)** and disengage (see **Fig. 34****)** the cog gear 254 with and from the cog gear 254. In the illustrated embodiment, the knob 304 can be pushed to engage the cog gear 254 with the cog gear 254 and pulled to disengage the cog gear 254 from the cog gear 254.

Thus, as illustrated in **Fig. 35****,** when the cog gear 254 is engaged with the cog wheel 276 by axially displacing the knob 304 (shown in **Fig. 36****),** and thus the sleeve 306, in the axial direction 252a, the endovascular management and tracking system 14 is placed in an automated modality, in which case, the axial motor 94' may be operated to automatically translate the shuttle 72' along the track segment 70'. In this state, the shuttle 72' may also be considered to be parked or locked in place on the track segment 70' to the extent that the shuttle 72' cannot be translated along the track segment 72 without commanded operation of the axial motor 94'.

In contrast, as illustrated in **Fig. 34****,** when the cog gear 254 is disengaged from the cog wheel 276 by axially displacing the knob 304 (shown in **Fig. 36****),** and thus the sleeve 306, in the axial direction 252b, the endovascular management and tracking system 14 is placed in a manual modality, in which case, operation of the axial motor 94' cannot automatically translate the shuttle 72' along the track segment 70'. However, the knob 304, and thus the sleeve 306, may be rotated in a clockwise or counterclockwise direction (see arrow 316 shown in **Fig. 34****)** to manually translate the shuttle 72' along the track segment 70'. In particular, the bore 310 of the sleeve 306 has a non-circular cross-section that matches the cross-section of the shaft 288 (in this case, hexagonal), such that an interference fit is provided between the sleeve 306 and the shaft 288. In this manner, rotation of the knob 304 rotates the sleeve 306, which, in turn, rotates the shaft 288, which in turn, rotates the pinion gear 256, thereby translating the shuttle 72' in the linear direction along the track segment 70'. Because the axial motor 94' and a large portion of the axial drive mechanism 92' (i.e., every mechanism but the pinion gear 256) are decoupled from the track segment 70', the shuttle 72' may alternatively be manually translated along the track segment 70' directly by hand without manipulation of the knob 304; albeit not in a finely controlled manner otherwise enabled by rotation of the knob 304.

Referring to **Figs. 40-42****,** the rotary drive mechanism 96' will now be described in further detail. In this embodiment, the rotary drive mechanism 96' comprises a cog gear 318 affixed to the motor shaft 244 of the rotary motor 98' (shown in **Figs. 31-32****),** and a cog gear 320 operably coupled to the cog gear 318. The cog gear 318 comprises a bore 322 through which the motor shaft 244 of the rotary motor 98' is affixed, such that the cog gear 318 rotates with the motor shaft 244.

The cog gear 320 comprises a bore 324 in which a component (not shown in **Figs. 40-41****)** to which the elongate medical device 26 is affixed, such that the cog gear 320 rotates with the motor shaft 244. In the embodiment illustrated in **Fig. 40****,** the shuttle 72' is to be used with an elongate medical device 26 that has fluid capability, e.g., a guide sheath 26a or a working catheter 26b, in which case, such component will be a male Touhy-Borst connector 122 (shown in **Fig. 8****)** of the RHV 112 to which the respective elongate medical device 26 is mechanically and fluidly coupled. Alternatively, if the shuttle 72' is to be used with an elongate medical device 26 with no fluid capability, e.g., a guide wire 26c, such component would be a collet 56 (shown in **Fig. 3****)** at the proximal end of the respective elongate medical device 26.

The cog gear 318 drives the cog gear 320, which, in turn, rotates the elongate medical device 26 relative to the shuttle 72'. To this end, the cog gears 318, 320 respectively have arrangements of teeth 326, 328 that match and mesh with each other, such that rotation of the cog gear 318 in one direction rotates the cog gear 320 in the opposite direction. In the illustrated embodiment, the rotary drive mechanism 96' resides outside of the casing 100, and thus, the upper casing portion 100a has an opening 330 through which the cog gear 320, along with the elongate medical device 26, and through which the distal portion of the motor shaft 244 of the rotary motor 98', along with the cog gear 318, extend.

In the illustrated embodiment, the components of each of the axial drive mechanism 92' and rotary drive mechanism 96' may be composed of a suitably rigid material, such as metal (e.g., stainless steel or brass), plastic (e.g., acetal or nylon), or a combination thereof.

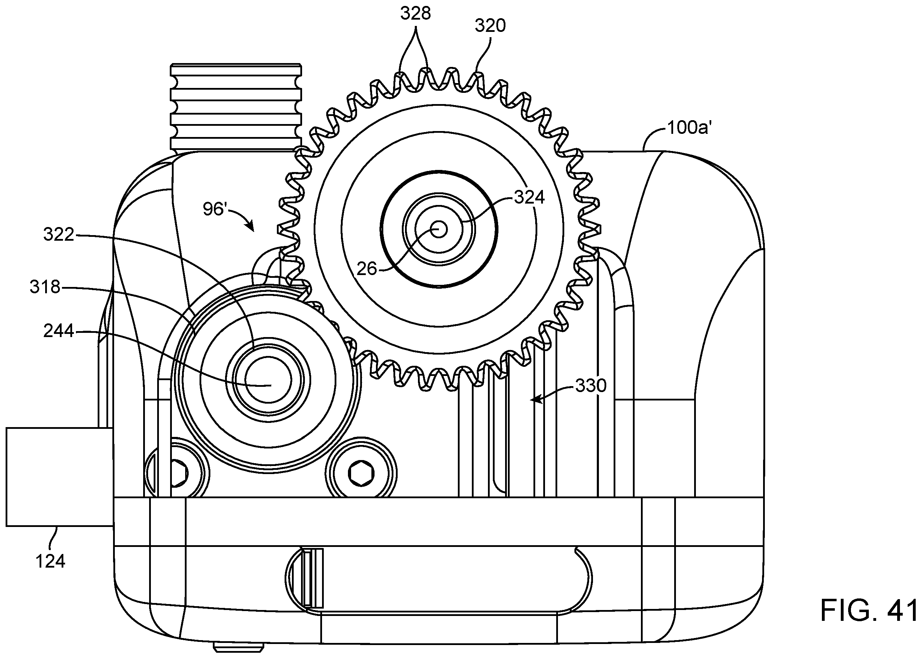

In the embodiment illustrated in **Figs. 40-42****,** the shuttle 72' is to be used with an elongate medical device 26 having fluid capability. In this case, the shuttle 72' further comprises the RHV 112, which in the illustrated embodiment, is removably mounted within the outer casing 100', such that it, along with the cog gear 320 mounted around the male Touhy-Borst connector of the RHV 112, can be replaced. As illustrated in Fig. 41, the distal end of the cylindrical tube 118 of the RHV 112 extends through the opening 330. The upper casing portion 100a' includes an additional opening 332 through which the proximal end of the cylindrical tube 118 of the RHV 112 extends, and an additional opening 334 through which the end of the side arm 124 of the RHV 112 extends.

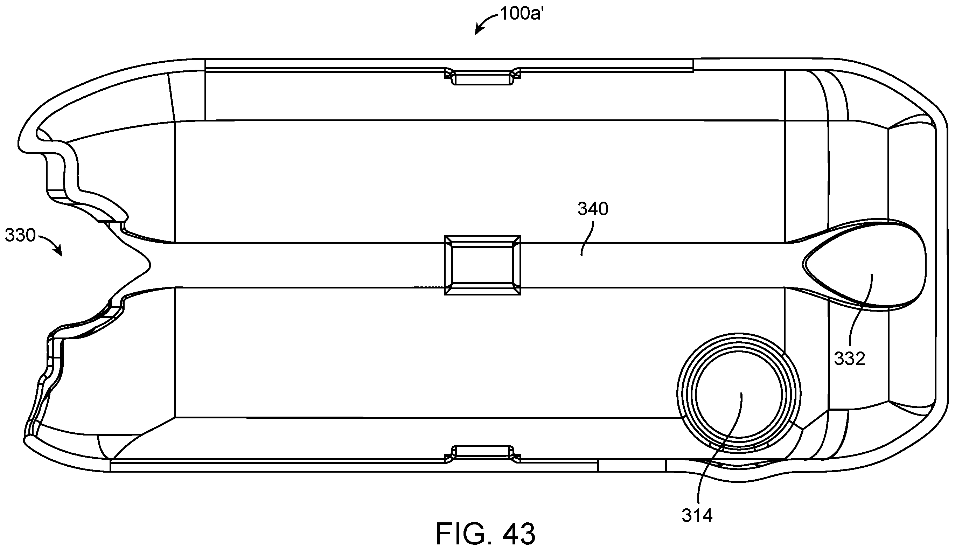

As best illustrated in **Figs. 31-32****,** the motor mount brackets 246 and gear support bracket 258 are arranged relative to each other, such that the RHV 112 may be stably held in place when the upper casing portion 100a' is mounted to the sled 90'. In particular, the RHV 112 may be nestled between the motor mounts 254 and gear support bracket 258, with the cylindrical tube 118 of the RHV 112 disposed within a space 336 between the motor mounts 254, and the side arm 124 of the RHV 112 disposed within a space 338 between the motor mount 254 associated with the axial motor 94' and the gear support bracket 258. The cylindrical tube 118 of the RHV 112 may also be sandwiched between the inner surface of the upper casing portion 100a' and the space 336 between the motor mounts 254 when the upper casing portion 100a' is affixed to the sled 90'. As illustrated in **Fig. 43****,** the inner surface of the upper casing portion 100a' comprises an open arcuate channel 340 for accommodating an arcuate segment of the cylindrical tube 118 when the upper casing portion 100a' is mounted to the sled 90', thereby preventing lateral movement of the RHV 112. The inner surface of the upper casing portion 100a' further comprises a raised arcuate portion 342 configured for firmly engaging the center of the cylindrical tube 118 when the upper casing portion 100a' is mounted to the sled 90'.

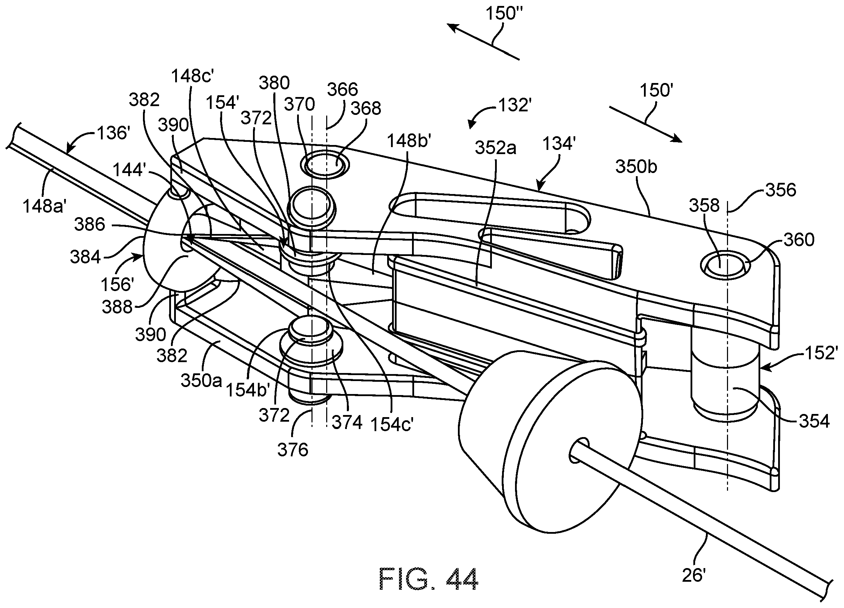

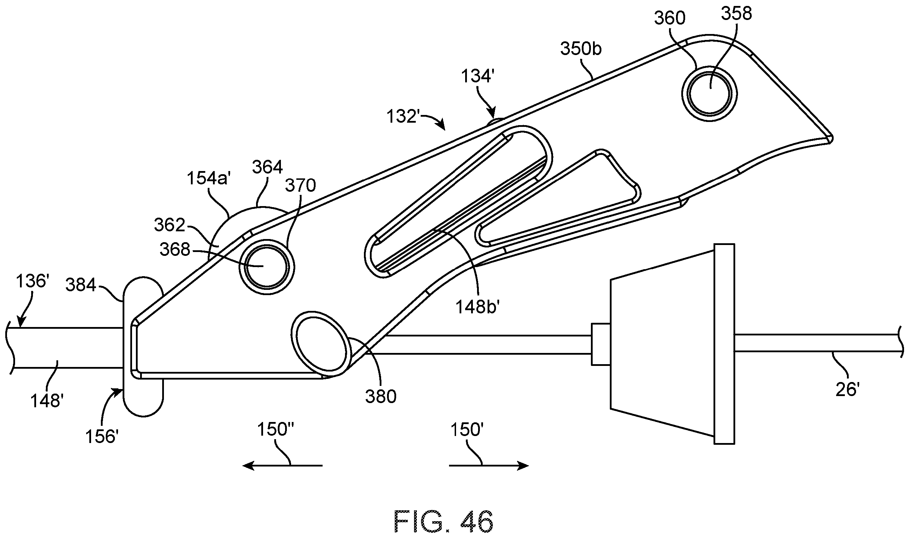

Referring now to **Figs. 44-49****,** the anti-buckling mechanism 132' will now be described in detail. The anti-buckling mechanism 132' comprises a housing 134' formed by a pair of parallel brackets 350a, 350b and a pair of cross-supports 352a, 352b, and an axially split sheath 136' that furls within the housing 134' and unfurls from the housing 134'.

The anti-buckling mechanism 132' further comprises a take-up reel 152' disposed at the proximal end of the housing 134', and in the illustrated embodiment, rotationally affixed between the brackets 350a, 350b. In particular, the take-up reel 152' comprises a spool 354, a rotational axis 356, and a shaft 358 extending through the rotational axis 356 of the take-up reel 152'. The proximal end of the housing 134' comprises a pair of holes 360 respectively formed through the brackets 350a, 350b and in which opposing ends of the shaft 358 of the take-up reel 152' is rotationally disposed, such that the spool 354 may rotate about the rotational axis 356 of the take-up reel 152' relative to the housing 134' to alternately roll the flattened proximal portion 148b' of the axially split sheath 136' when furled within the housing 134', and unrolling the flattened proximal portion 148b' of the axially split sheath 136' when unfurled from the housing 134'.

The anti-buckling mechanism 132' further comprises a flattening mechanism 154' disposed within the housing 134', and in the illustrated embodiment, rotationally affixed between the brackets 350a, 350b. In particular, the flattening mechanism 154' comprises pinch rollers 154a'-154c' between which the axially split sheath 136' is disposed.

The pinch roller 154a' comprises a drum 362 having a uniform diameter friction surface 364, a rotational axis 366, and a shaft 368 extending through the rotational axis 366 of the pinch roller 154a'. The drum 362 spans substantially the entire space between the brackets 350a, 350b, thereby maximizing the contact area between the friction surface 364 and the flattened proximal portion 148b' of the axially split sheath 136. The distal end of the housing 134' comprises a pair of holes 370 respectively formed through the brackets 350a, 350b and in which opposing ends of the shaft 368 of the pinch roller 154a' is rotationally disposed, such that the drum 362 may rotate about the rotational axis 366 of the pinch roller 154a' relative to the housing 134'.

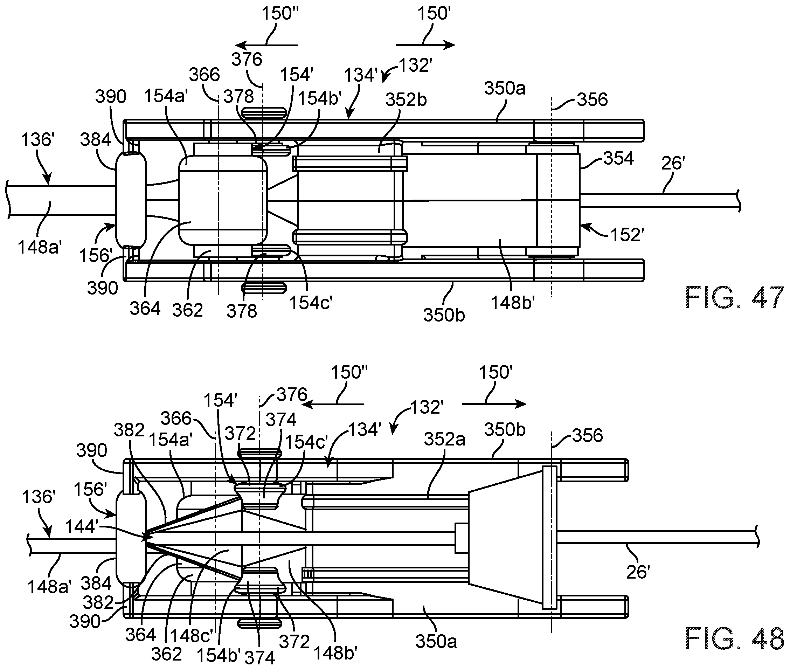

Each of the pinch rollers 154b', 154c' comprises a wheel 372 having a concave frictional surface 374, a rotational axis 376, and a shaft 378 (best shown in **Fig. 47****)** extending through the rotational axis 376 of the respective pinch roller 154b', 154c'. The distal end of the housing 134' comprises a pair of holes 380 respectively formed through the brackets 350a, 350b. The shafts 378 of the pinch rollers 154b', 154c' are respectively rotationally disposed in the holes 380. The holes 380 in the brackets 350a, 350b are aligned with each other, such that the rotational axes 376 of the pinch rollers 154b', 154c' are coaxial, and are offset from the holes 370 to which the shaft 368 of the pinch roller 154a' is rotationally affixed, such that the pinch rollers 154b', 154c' are offset from the pinch roller 154a' in a directional orthogonal to their rotational axes 366, 376 a distance that places the frictional surfaces 364, 374 of the drum 362 and wheels 372 in contact with each other, or otherwise in close proximity to each other, such that, as the axially split sheath 136' is axially translated in the proximal direction 150' between the pinch roller 154a' and pinch rollers 154b', 154c', its tubular distal portion 148a' is continually transformed into its transition portion 148c', and then into its flattened proximal portion 148b'.

Notably, the opposing edges 382 of the transition portion 148c' of the axially split sheath 136' are captured within the concave friction surfaces 374 of the wheels 372 of the pinch rollers 154b', 154c', thereby ensuring that the flattened proximal portion 148b' is properly aligned between the pinch roller 154a' and pinch rollers 154b', 154c', and that the flattening mechanism 154' robustly transforms the tubular distal portion 148a' into the flattened proximal portion 148b' while the axially split sheath 136' is axially translated in the proximal direction 150'. The cross-support 352a is disposed between the take-up reel 152' and the flattening mechanism 154', and has a flat bearing surface on which the flattened proximal portion 148b' of the axially split sheath 136' slides, thereby ensuring that the flattened proximal portion 148b' of the axially split sheath 136' remains in the flattened state between the take-up reel 152' and the flattening mechanism 154'.

The anti-buckling mechanism 132' further comprises a tube forming mechanism 156' disposed at the distal end of the housing 134' distal to the flattening mechanism 154'. The tube forming mechanism 156' comprises a ring 384 having an aperture 386 through which the axially split sheath 136' is disposed. The ring 384 has an arcuate bearing surface 388 surrounding the aperture 386 that contacts the outer surface of the axially split sheath 136'. The diameter of the aperture 386 is slightly greater than the diameter of the tubular distal portion 148a', but much smaller than the width of the flattened proximal portion 148b', of the axially split sheath 136', such that, as the axially split sheath 136' is axially translated in the distal direction 150" through the aperture 386 of the ring 384, its flattened proximal portion 148b' is continually transformed from its transition portion 148c', and then into its tubular distal portion 148a'. The ring 384 is affixed between the brackets 350a, 350b via rigid supports 390.

As best illustrated in **Figs. 44** and **48****,** the elongate medical device 26' may be loaded into the exposed central lumen 144' of the axially split sheath 136' at the interface between the tubular distal portion 148a' and transition portion 148c'.

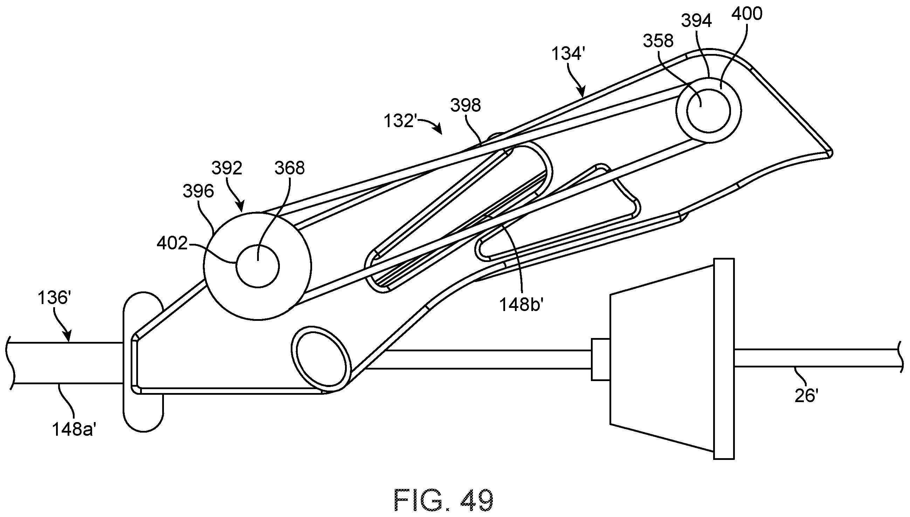

Referring to **Fig. 49****,** an optional embodiment of the anti-buckling mechanism 132' further comprises a take-up biasing mechanism 392 configured for biasing the take-up reel 152' (shown in **Figs. 44-47****)** to roll the flattened proximal portion 148b' of the axially split sheath 136' when being furled into the housing 134'.

In the illustrated embodiment, the take-up biasing mechanism 392 comprises an idler pulley 394 affixed to the take-up reel 152', such that the take-up reel 152' rotates in unison with the idler pulley 394. In particular, the idler pulley 394 has a bore 400 through which the shaft 358 of the take-up reel 152' is affixed, such that the idler pulley 394 rotates, along with the take-up reel 152', about the rotational axis 356 of the take-up reel 152'.

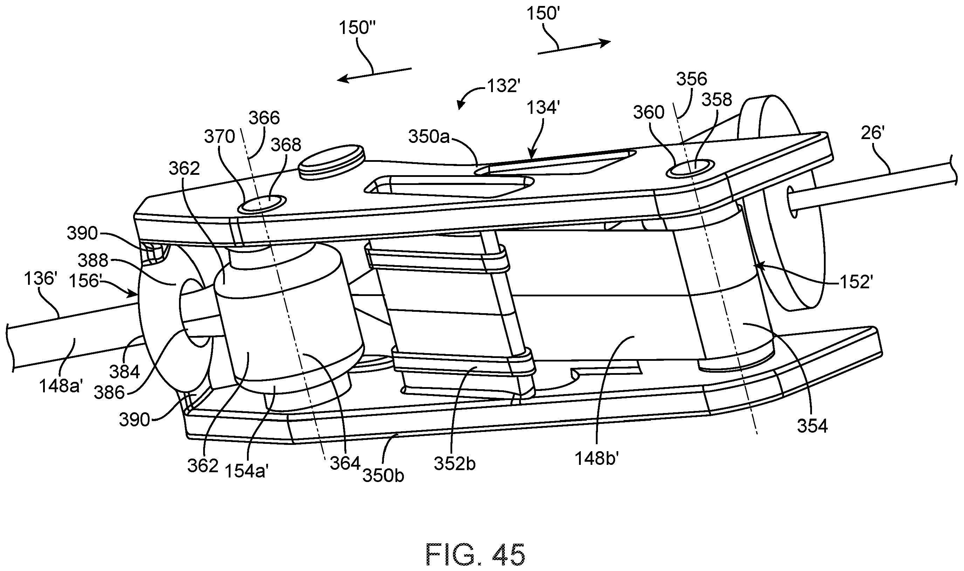

The take-up biasing mechanism 392 further comprises a driver pulley 396 configured for rotating when the flattened proximal portion 148b' of the axially split sheath 136' is furled within the housing 134'. In the illustrated embodiment, the driver pulley 396 is affixed to one of the pinch rollers 154a'-154c', and in particular, the pinch roller 154a' (shown in **Figs. 45-48****)** (which rotates as the axially split sheath 136' is furled into the housing 134'), such that driver pulley 396 rotates in unison with the pinch roller 154a'. The driver pulley 396 has a bore 402 through which the shaft 368 of the pinch roller 154a' is affixed, such that the driver pulley 396 rotates, along with the pinch roller 154a', about the rotational axis 366 of the pinch roller 154a'. In alternative embodiments, the driver pulley 396 may be directly or indirectly coupled to some other component (e.g., the track 70') that is registered with the axial translation of the axially split sheath 136' when being furled into the housing 134'.

The take-up biasing mechanism 392 further comprises a drive belt 398 coupling the idler pulley 394 to the driver pulley 396, such that the idler pulley 394 rotates in response to rotation of the driver pulley 396. Preferably, the driver pulley 396 drives the idler pulley 394 via the drive belt 398 at a rotational speed that maintains constant tension on the flattened proximal portion 148b' of the axially split sheath 136' as it is being furled within the housing 134'. To this end, the diameter of the idler pulley 394 is less than the diameter of the driver pulley 396, such that the driver pulley 396 over-drives the idler pulley 394.

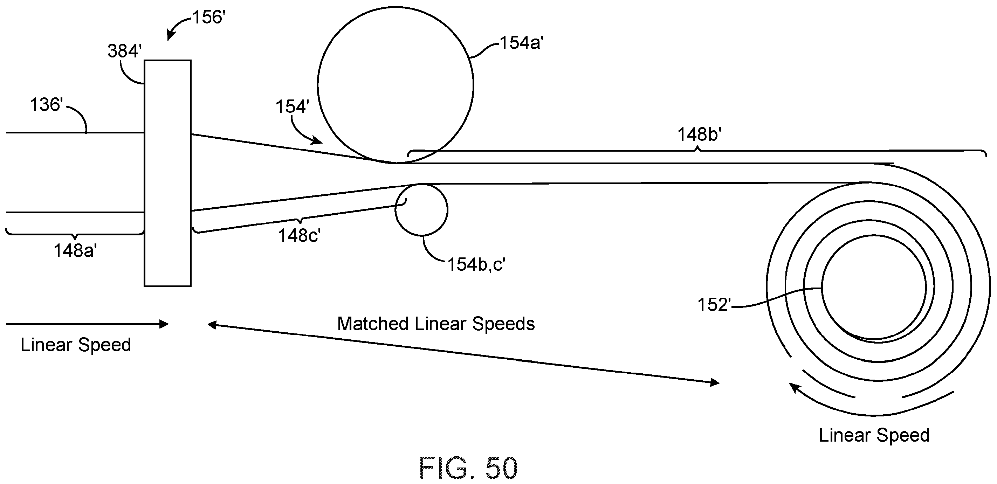

Referring to **Fig. 50****,** it should be appreciated that, without intervention, the linear speed of the furled flattened proximal portion 148b' of the axially split sheath 136' (i.e., the portion that is wrapped around the take-up reel 152') that is rotating with the take-up reel 152' will tend to increase as its diameter D grows, thereby potentially causing a mismatch with the linear speed of the tubular distal portion 148a' of the axially split sheath 136' relative to the housing 134', which may confound the anti-buckling mechanism 132'. Thus, it is preferred that the take-up biasing mechanism 392 include some type of clutching mechanism configured for matching the linear speed of the furled flattened proximal portion 148b' with the linear speed of the axially split sheath 136' relative to the housing 134'.

In the illustrated embodiment, the clutching mechanism takes the form of a frictional clutch between the idler pulley 394 and the drive belt 398. Thus, as the linear speed of the furled flattened proximal portion 148b' of the axially split sheath 136' tends to increase more than the linear speed of the tubular distal portion 148a' of the axially split sheath 136' relative to the housing 134', the resulting increased tension in the axially split sheath 136' will be translated to an increase in tension in the drive belt 398, thereby causing the drive belt 398 to slip relative to the idler pulley 394. Accordingly, such slippage will cause the rotational speed of the take-up reel 152' will decrease, and thus, the linear speed of the furled flattened proximal portion 148b' of the axially split sheath 136' will decrease to match the linear speed of the tubular distal portion 148a' of the axially split sheath 136' relative to the housing 134'. It should be appreciated that the diameter ratios between the , driver pulley 396 and the idler pulley 394 (preferably, greater than one in an over-driven arrangement) may be set, such that the minimum linear speed of the furled flattened proximal portion 148b' of the axially split sheath 136' will be at least slightly greater than the linear speed of the tubular distal portion 148a' of the axially split sheath 136' relative to the housing 134', so that constant tension is applied to the flattened proximal portion 148b' of the axially split sheath 136' as it is being furled within the housing 134', as described above.

In an optional embodiment, the take-up biasing mechanism 392 comprises a ratchet (not shown) associated with the idler pulley 394, such that the idler pulley 394 free spins as the flattened proximal portion 148b' of the axially split sheath 136' is unrolled from the take-up reel 152' when unfurled from the housing 132'.

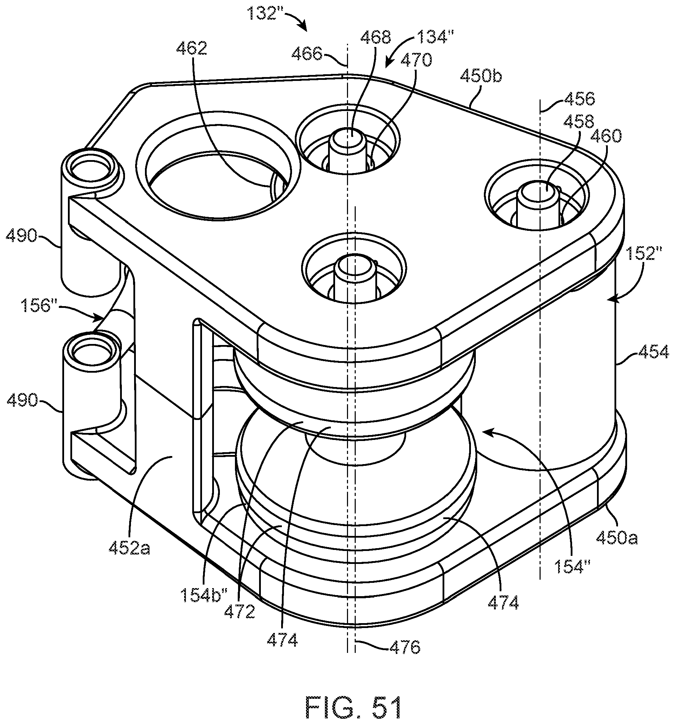

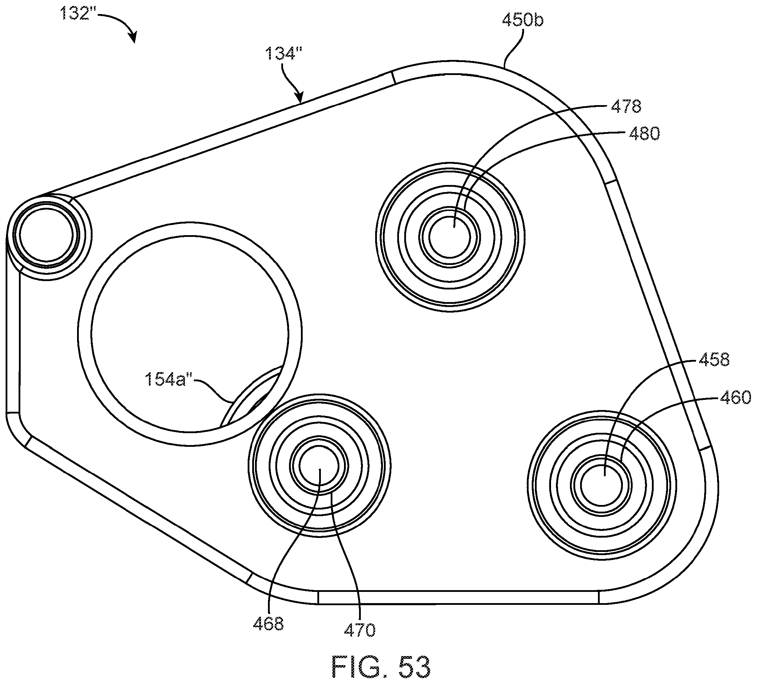

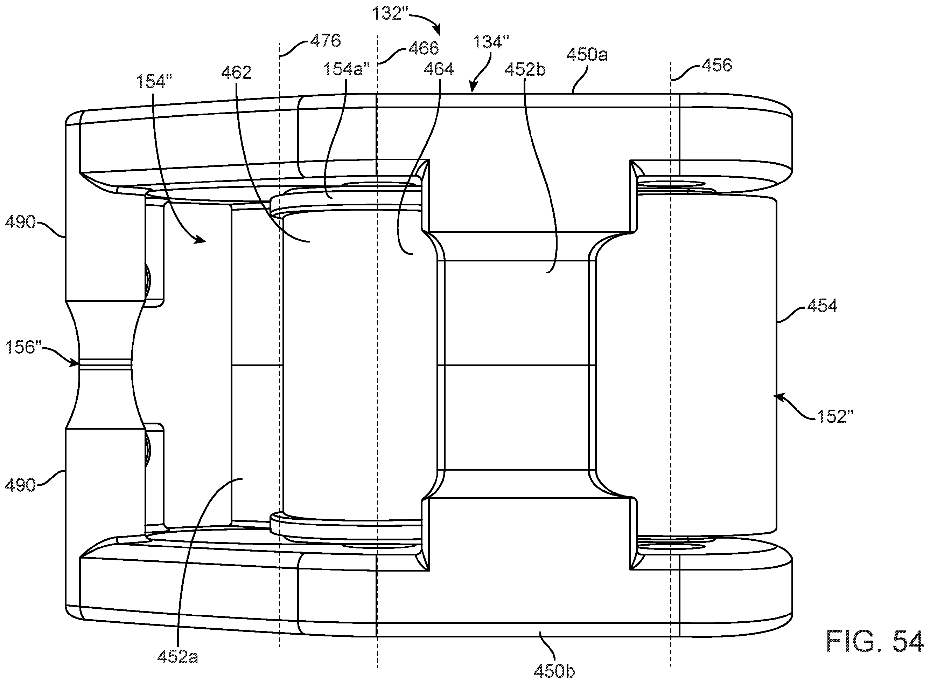

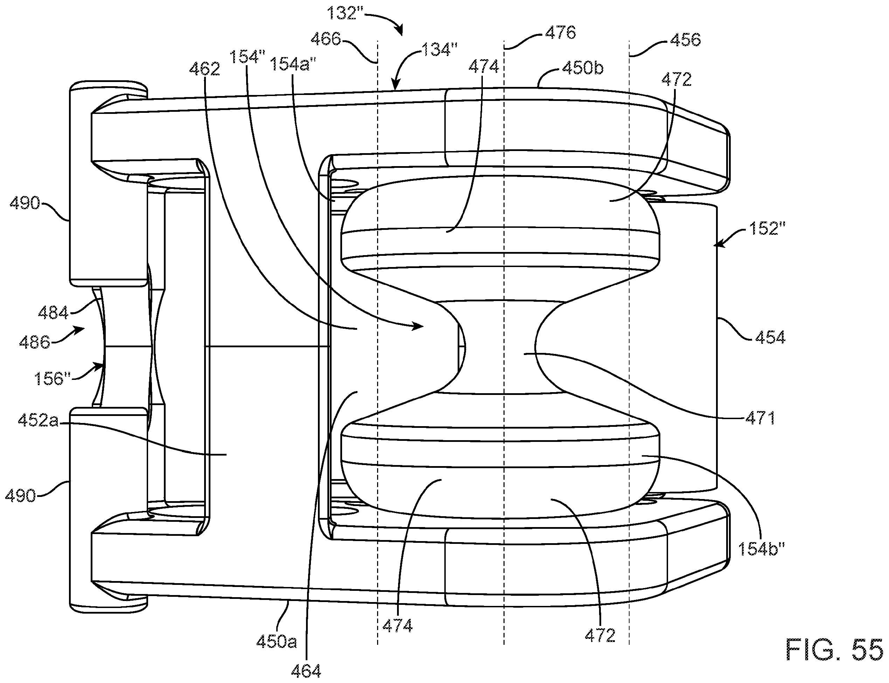

Referring now to **Figs. 51-55****,** an alternative embodiment of an anti-buckling mechanism 132" that can be used in the shuttle 72' illustrated in **Figs. 20-24** will be described. The anti-buckling mechanism 132" is similar to the anti-buckling mechanism 132' illustrated in **Figs. 44-49****,** with the exception that the anti-buckling mechanism 132" is more compact in that the distance between the flattening mechanism and the take-up reel is substantially shortened. As a result, the flattened proximal portion of the axially split sheath 136' between the flattening mechanism and the take-up reel is more likely to remain in a flattened state.

The anti-buckling mechanism 132" comprises a housing 134" formed by a pair of parallel brackets 450a, 450b and a pair of cross-supports 452a, 452b, and the axially split sheath 136' (shown in **Figs. 44-49****)** that furls within the housing 134" and unfurls from the housing 134".

The anti-buckling mechanism 132' further comprises a take-up reel 152" disposed at the proximal end of the housing 134", and in the illustrated embodiment, rotationally affixed between the brackets 450a, 450b. In particular, the take-up reel 152" comprises a spool 454, a rotational axis 456, and a shaft 458 extending through the rotational axis 456 of the take-up reel 152". The proximal end of the housing 134" comprises a pair of holes 460 respectively formed through the brackets 450a, 450b and in which opposing ends of the shaft 458 of the take-up reel 152" is rotationally disposed, such that the spool 454 may rotate about the rotational axis 456 of the take-up reel 152" relative to the housing 134" to alternately roll the flattened proximal portion 148b' of the axially split sheath 136' when furled within the housing 134", and unrolling the flattened proximal portion 148b' of the axially split sheath 136' when unfurled from the housing 134".

The anti-buckling mechanism 132' further comprises a flattening mechanism 154" disposed within the housing 134", and in the illustrated embodiment, rotationally affixed between the brackets 450a, 450b. In the illustrated embodiment, the flattening mechanism 154" comprises pinch rollers 154a", 154b" between which the axially split sheath 136' is disposed.

The pinch roller 154a" comprises a drum 462 having a uniform diameter friction surface 464, a rotational axis 466, and a shaft 468 extending through the rotational axis 466 of the pinch roller 154a". The drum 462 spans substantially the entire space between the brackets 450a, 450b, thereby maximizing the contact area between the friction surface 464 and the flattened proximal portion 148b' of the axially split sheath 136. The distal end of the housing 134" comprises a pair of holes 470 respectively formed through the brackets 450a, 450b and in which opposing ends of the shaft 468 of the pinch roller 154a" is rotationally disposed, such that the drum 462 may rotate about the rotational axis 466 of the pinch roller 154a" relative to the housing 134".

The pinch roller 154b" is dumbbell-shaped. In particular, the pinch roller 154b" comprises a center bar 471 and a pair of enlarged wheels 472 straddling the bar 471, each of which is has a convex frictional surface 474, a rotational axis 476, and a shaft 478 extending through the rotational axis 476 of the pinch roller 154b". The distal end of the housing 134" comprises a pair of holes 480 respectively formed through the brackets 450a, 450b. The shaft 478 of the pinch roller 154b" is rotationally disposed in the holes 480. The holes 480 in the brackets 450a, 450b are offset from the holes 470 to which the shaft 468 of the pinch roller 154a" is rotationally affixed, such that the pinch roller 154b" is offset from the pinch roller 154a" in a directional orthogonal to their rotational axes 466, 476 a distance that places the frictional surfaces 464, 474 of the drum 462 and wheels 472 in contact with each other, or otherwise in close proximity to each other, such that, as the axially split sheath 136' is axially translated in the proximal direction 150' between the pinch roller 154a" and pinch roller 154b", its tubular distal portion 148a' is continually transformed into its transition portion 148c', and then into its flattened proximal portion 148b'.

Notably, the opposing edges 382 of the transition portion 148c' of the axially split sheath 136' wrap around the convex friction surfaces 474 of the wheels 472 of the pinch roller 154b", thereby ensuring that the flattened proximal portion 148b' is properly aligned between the pinch roller 154a" and pinch rollers 154b", and that the flattening mechanism 154" robustly transforms the tubular distal portion 148a' into the flattened proximal portion 148b' while the axially split sheath 136' is axially translated in the proximal direction 150'.

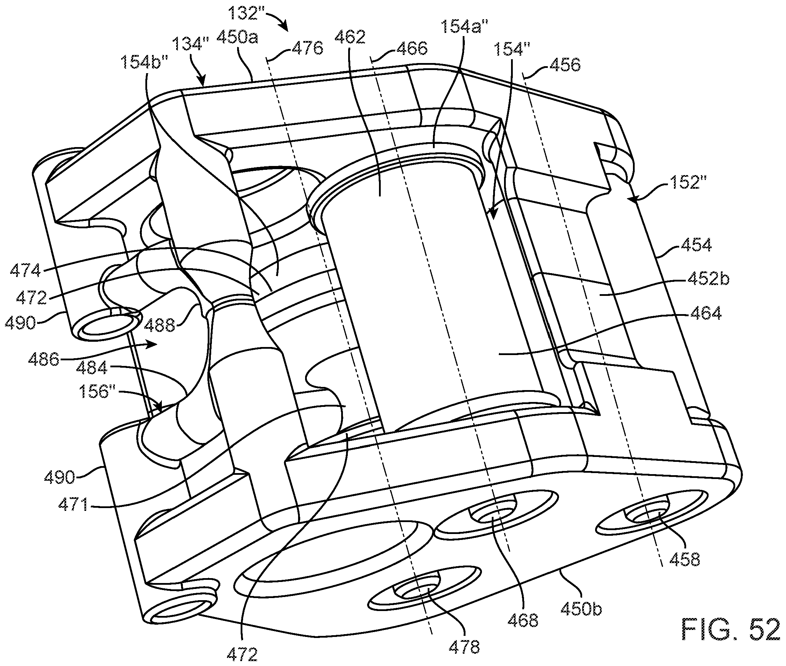

The anti-buckling mechanism 132' further comprises a tube forming mechanism 156" disposed at the distal end of the housing 134" distal to the flattening mechanism 154". The tube forming mechanism 156" comprises an arch 484 having an aperture 486 (best shown in Fig. 52) through which the axially split sheath 136' is disposed. The arch 484 has an arcuate bearing surface 488 surrounding the aperture 486 that contacts the outer surface of the axially split sheath 136'. The diameter of the aperture 486 is slightly greater than the diameter of the tubular distal portion 148a', but much smaller than the width of the flattened proximal portion 148b', of the axially split sheath 136', such that, as the axially split sheath 136' is axially translated in the distal direction 150" through the aperture 478 of the arch 484, its flattened proximal portion 148b' is continually transformed from its transition portion 148c', and then into its tubular distal portion 148a'. The arch 484 is affixed between the brackets 450a, 450b via rigid supports 490.

Although both of the catheter tracking and management systems 14, 14' are fully automated in that each of the shuttles is axially translated independently in response to direct input from the operator 24 at the master input device 194 at the control station 16, in alternative embodiments, a catheter tracking and management system may operate in a hybrid manual/automated (i.e., a semi-automated) mode (otherwise known as an automated assistance system) at least one of the shuttles may be automatically axially translated along the track in response to axially translating another one of the shuttles manually by the operator 24 along the track. This type of master-slave arrangement obviates the need for multiple operators (e.g., a primary operator, such as a physician, and a secondary operator, such as a trained assistant) to perform the medical procedure by using the manual motions of one endovascular device by the primary operator to control automated motions of another endovascular device. Thus, such a semi-automated master-slave arrangement allows the secondary operator to be eliminated, thereby improving the workflow of the medical procedure.

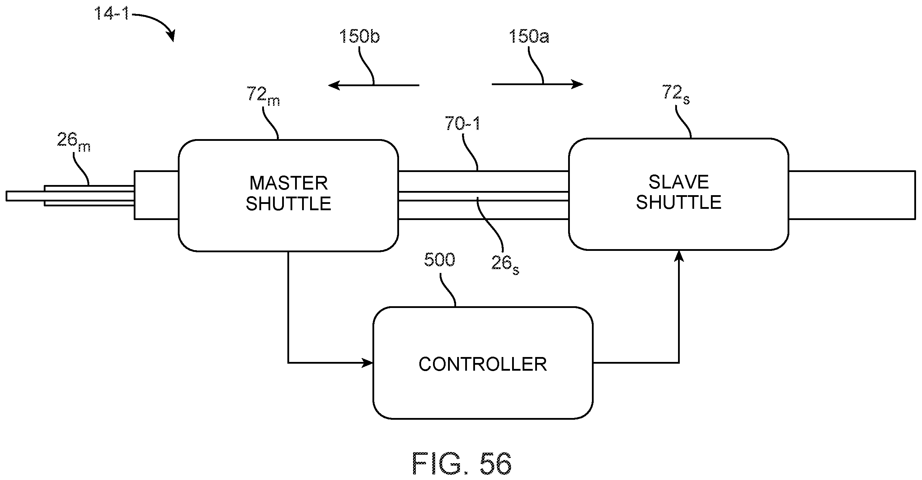

For example, in one generalized embodiment illustrated in **Fig. 56****,** one of the shuttles of a catheter tracking and management system 14-1 may be a master shuttle 72ₘ, while at least one of the other of the shuttles may be designed to be a slave shuttle 72ₛ. In response to manually axially translating the master shuttle 72ₘ, and thus the elongate medical device 26ₘ affixed to the master shuttle 72ₘ, along the track 70-1 (e.g., by manually operating a manual actuator of the master shuttle 72ₘ to axially index the master shuttle 72ₘ or by moving the master shuttle 72ₘ directly by hand), the slave shuttle 72ₛ, and thus the elongate medical device 26ₛ affixed to the slave shuttle 72ₛ, will be axially translated along the track 70-1 in synchronous coordination with the axial translation of the master shuttle 72ₘ along the track 70-1, and thus, the elongate medical device 26ₛ in synchronous coordinate with the axial translation of the elongate medical device 26ₘ.

For example, the slave shuttle 72ₛ may be axially translated along the track 70 in coordination with the axial translation of the master shuttle 72ₘ in accordance with an axial translation ratio (e.g., the slave shuttle 72ₛ may be axially translated along the track 70-1 half the distance, the same distance (unity axial translation ratio), twice the distance, etc., as the master shuttle 72ₘ is axially translated along the track 70-1), which may be positive (the master shuttle 72ₘ and slave shuttle 72ₛ are axially translated along the track 70 in the same direction) or negative (the master shuttle 72ₘ and slave shuttle 72ₛ are axially translated along the track 70 in opposite directions).

While the master shuttle 72ₘ is illustrated as being the subsequent (distal) one of the shuttles, and slave shuttle 72ₛ is illustrated as being the preceding (proximal) one of the shuttles, it should be appreciated in alternative embodiments, the master shuttle 72ₘ may be the preceding (proximal) one of the shuttles, while the slave shuttle 72ₘ may be the subsequent (distal) one of the shuttles. Furthermore, at any given time, the master/slave functions of any two of the shuttles 72 may be switched simply by manually translating one of the shuttles 72 rather than the other one of the shuttles 72. For example, a subsequent one of the shuttles may be switched from a slave shuttle 72ₛ to a master shuttle 72ₘ simply by manually translating the subsequent shuttle, in which case, the preceding shuttle will be switched from a master shuttle 72ₘ to a slave shuttle 72ₛ, and will be axially translated along the track 70-1 in synchronous coordination with the axial translation of the subsequent shuttle along the track 70-1. The preceding shuttle may then be switched from a slave shuttle 72ₛ to a master shuttle 72ₘ by manually translating by manually translating the preceding shuttle, in which case, the subsequent shuttle will be switched from a master shuttle 72ₘ to a slave shuttle 72ₛ, and will be axially translated along the track 70-1 in synchronous coordination with the axial translation of the preceding shuttle along the track 70-1.

To synchronize the axial displacement between the master shuttle 72ₘ and slave shuttle 72ₛ, a controller 500 is employed to actuate the axial drive mechanism (not shown in **Fig. 56****)** of the slave shuttle 72ₛ in response to the axial translation of the master shuttle 72ₘ manually along the track 70-1, such that the slave shuttle 72ₛ is axially translated in synchronous coordination with the axial translation of the master shuttle 72ₘ along the track 70-1.

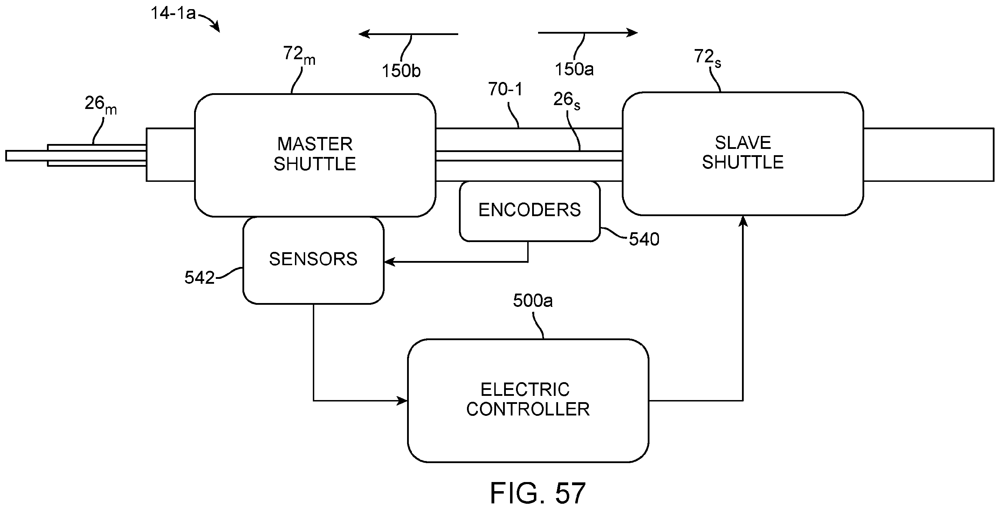

In one exemplary embodiment of a catheter tracking and management system 14-1a illustrated in **Fig. 57****,** such controller may be an electrical controller 500a, e.g., the processor of the control station 16, which can receive electrical signals from the master shuttle 72ₘ through a wired or wireless connection (e.g., the wired or wireless connection described above for communicating between the control station 16 and the shuttles 72a-72c in **Fig. 6****),** indicating the location of the master shuttle 72ₘ relative to the track 70. In this embodiment, the catheter tracking and management system 14-1 comprises one or more encoders 540 that encodes the location of the master shuttle 72ₘ relative to the track 70, and one or more sensors 542 configured for reading the encoders 540 and outputting an electrical signal 544 indicative of the location of the master shuttle 72ₘ relative to the track 70.

In one embodiment, the encoder(s) 540 are disposed along the track 70, whereas the sensor(s) 542 are disposed on the master shuttle 72ₘ. For example, the encoders 540 may comprise a series of fiducial elements extending along a length of the track 70 (e.g., similar to the fiducial elements 86 illustrated in **Figs. 7C-7D****),** and the sensors 542 may comprise a fiducial element reader disposed on the master shuttle 72ₘ (e.g., on the sled 90' illustrated in **Figs. 30-32****)** and configured for reading the fiducial elements 540 (e.g., optically, magnetically, capacitively, etc.). The controller 500a may then control the first axial drive mechanism (e.g., by controlling the axial drive mechanism 92' (shown in **Figs. 31-39****)** of the slave shuttle 72ₛ based on the electrical signal 498, such that the slave shuttle 72ₛ axially translates along the track 70-1 in synchrony with the master shuttle 72ₘ.

As discussed above with respect to the fiducial elements 86 illustrated in **Figs. 7C-7D****,** the fiducial elements 540 may be alternatively located on the rotating parts of the axial drive mechanism 92' (shown in **Figs. 31-39****)** of the master shuttle 72ₘ, while the fiducial element sensors 540 may be located on an inside surface of the casing 100' (shown in **Figs. 21-23****)** or otherwise a stationary frame structure within the master shuttle 72ₘ. In other alternative embodiments, active electronics components (e.g., transmitter-receiver pairs) capable of performing location sensing using signaling (e.g., optically, inductive, capacitive, radio frequency (RF), etc.) can be embedded into the track 70' and master shuttle 72ₘ. In still other alternative embodiments, fiducial elements (not shown) can be disposed on the distal end of the elongated medical device 26 associated with the master shuttle 72ₘ, which may be visually sensed by a vision-based system, such as a fluoroscopic imaging device (not shown) to directly track the locations of the elongated medical device 26 rather than directly tracking the location of the master shuttle 72ₘ relative to the track 70'.

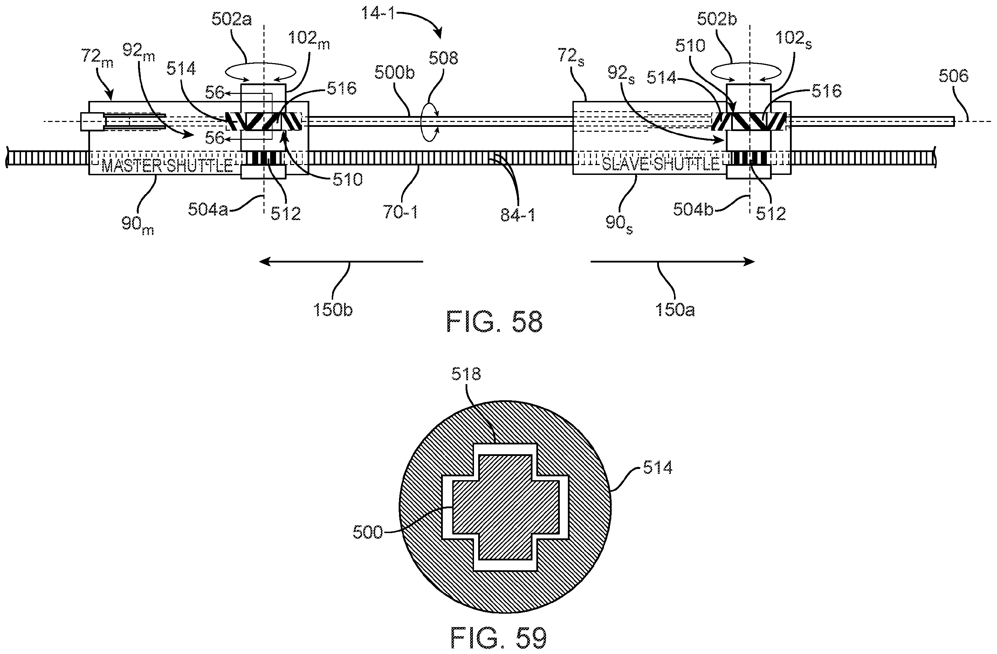

In another exemplary embodiment of a catheter tracking and management system 14-1b illustrated in **Fig. 58****,** the controller 500 may be a mechanical controller 500b. In this embodiment, the master shuttle 72ₘ comprises a sled 90ₘ configured for riding on a track 70-1, an axial drive mechanism 92ₘ carried by the sled 90ₘ and configured for being actuated to axially translate the master shuttle 72ₘ, and thus, the elongate medical device 26ₘ (shown in **Fig. 56****)** along the track 70-1, and a manual actuator 102ₘ carried by the sled 90ₘ and configured for actuating the axial drive mechanism 92ₘ. Similarly, the slave shuttle 72ₛ comprises a sled 90ₛ configured for riding on a track 70-1, an axial drive mechanism 92ₛ carried by the sled 90ₛ and configured for being actuated to axially translate the slave shuttle 72ₛ, and thus the elongate medical device 26ₛ (shown in **Fig. 56****),** along the track 70-1, and an optional manual actuator 102ₛ carried by the sled 90ₛ and configured for actuating the axial drive mechanism 92ₛ.

In an optional embodiment, the master shuttle 72ₘ further comprises a rotary drive mechanism (not shown) carried by the sled 90ₘ and configured for being actuated to rotate the elongate medical device 26ₘ about its longitudinal axis relative to the master shuttle 72ₘ. Similarly, the slave shuttle 72ₛ further comprises a rotary drive mechanism (not shown) carried by the sled 90ₛ and configured for being actuated to rotate the elongate medical device 26ₛ about its longitudinal axis relative to the slave shuttle 72ₛ. The master and slave shuttles 72ₘ, 72ₛ may also comprise manual actuators (not shown) configured for respectively actuating the rotary drive mechanisms. One or both of the master and slave shuttles 72ₘ, 72ₛ may also comprise an anti-buckling mechanism (not shown) configured for respectively preventing respective elongate medical device 26ₘ, 26ₛ from prolapsing in response to axial compression applied to the respective elongate medical device 26ₘ, 26ₛ.

In much the same manner described above with respect to the endovascular management and tracking systems 14', 14" described above, rotation of the manual actuator 102ₘ of the master shuttle 72ₘ in one of a clockwise or counterclockwise direction 502a about its rotational axis 504a will cause the master shuttle 72ₘ to axially translate the master shuttle 72ₘ via the axial drive mechanism 92ₘ along the track 70-1 either in the proximal direction 150a or the distal direction 150b, whereas rotation of the optional manual actuator 102ₘ of the slave shuttle 72ₛ in one of a clockwise or counterclockwise direction 502b about its rotational axis 504b will cause the slave shuttle 72ₛ to axially translate the slave shuttle 72ₛ via the axial drive mechanism 92ₛ along the track 70-1 either in the proximal direction 150a or the distal direction 150b.

However, in addition to axially translating the master shuttle 72ₘ along the track 70-1, rotation of the manual actuator 102ₘ of the master shuttle 72ₘ about its rotational axis 504a will also actuate the axial drive mechanism 92ₛ of the slave shuttle 72ₛ via the mechanical controller 500b to axially translate the slave shuttle 72ₛ along the track 70-1 either in the proximal direction 150a or the distal direction 150b in accordance with a positive or negative axial translation ratio, as described above with respect to **Fig. 56****.** To the extent that an optional manual actuator 102ₛ is provided for the slave shuttle 72ₛ, the functions of the shuttles 72ₘ, 72ₛ may be switched, such that the shuttle 72ₛ may serve as the master shuttle, and the shuttle 72ₘ may serve as the slave shuttle. In this case, in addition to axially translating the shuttle 72ₛ along the track 70-1, rotation of the optional manual actuator 102ₛ of the shuttle 72ₛ along its rotational axis 504b will also actuate the axial drive mechanism 92ₘ of the shuttle 72ₘ via the mechanical controller 500b to axially translate the shuttle 72ₘ along the track 70-1 either in the proximal direction 150a or the distal direction 150b in accordance with the positive or negative axial translation ratio.

To this end, the mechanical controller 500b takes the form of a control shaft mechanically coupled between the axial drive mechanisms 92ₘ, 92ₛ of the respective master and slave shuttles 72ₘ, 72ₛ. The control shaft 500b is configured for rotating (shown by the bi-directional arrow 506) about its longitudinal axis 508 in response to actuation of the axial drive mechanism 92ₘ of the master shuttle 72ₘ. The axial drive mechanism 92ₛ of the slave shuttle 72ₛ is configured for being actuated in response to rotation of the control shaft 500b about its longitudinal axis 508. As a result, the master and slave shuttles 72ₘ, 72ₛ are axially translated along the track 70-1 in synchrony in accordance with the positive or negative axial translation ratio.

As will be described in further detail below, the control shaft 500b is mechanically coupled between the axial drive mechanisms 92ₘ, 92ₛ, such that the control shaft 500b and one of the master and slave shuttles 72ₘ, 72ₛ are configured for being axially translated in unison, while the control shaft 500b and the other of the master and slave shuttles 72ₘ, 72ₛ are configured for being axially translated relative to each other. In the illustrated embodiment, the control shaft 500b and master shuttle 72ₘ are configured for axially translating in unison, while the control shaft 500b and slave shuttle 72ₛ are configured for axially translating relative to each other.

As will also be described in further detail below, the control shaft 500b is mechanically coupled between the axial drive mechanisms 92ₘ, 92ₛ of the master and slave shuttles 72ₘ, 72ₛ in a detachable manner, such that the endovascular management and tracking system 14-1 may be switched between a master-slave configuration, wherein axial translation of the slave shuttle 72ₛ along the track 70-1 is synchronized with the axial translation of the master shuttle 72ₘ along the track 70-1 in response to manipulation of the manual actuator 102ₘ of the master shuttle 72ₘ, and an independent configuration, wherein axial translation of the master and slave shuttles 72ₘ, 72ₛ in response to manipulation of the manual actuators 102ₘ, 102ₛ of the master and slave shuttles 72ₘ, 72ₛ.

As discussed above, the axial drive mechanism 92ₘ of the master shuttle 72ₘ is actuated via rotation of the manual actuator 102ₘ of the master shuttle 72ₘ in one of the clockwise or counterclockwise direction 502a. Thus, rotation of the manual actuator 102ₘ actuates the axial drive mechanism 92ₘ, which in turn, axially translates the master shuttle 72ₘ along the track 70-1, while also rotating the control shaft 500b about its longitudinal axis 508, which actuates the axial drive mechanism 92ₛ, which in turn, axially translates the slave shuttle 72ₛ along the track 70-1. If the optional manual actuator 102ₛ is provided for the slave shuttle 72ₘ, actuation of the axial drive mechanism 92ₛ may also rotate the optional manual actuator 102ₛ in one of the clockwise or counterclockwise direction 502b as an ancillary function to the axial translation of the slave shuttle 72ₛ along the track 70-1.

As will be described in further detail below, the axial drive mechanisms 92ₘ, 92ₛ of the respective master and slave shuttles 72ₘ, 72ₛ may be designed to provide a specific axial translation ratio (positive or negative) between the master and slave shuttles 72ₘ, 72ₛ. It should be appreciated that selectively rotating the manual actuator 102ₘ of the master shuttle 72ₘ in one of the clockwise or counterclockwise direction 502a not only selectively causes the master shuttle 72ₘ to axially translate in one of the proximal direction 150a and the distal direction 150b, but also, selectively causes the control shaft 500b to rotate about its longitudinal axis 508 in one of the two rotational directions 506, thereby selectively causing the slave shuttle 72ₘ to axially translate in one of the proximal direction 150a and the distal direction 150b.

In the illustrated embodiment, the axial drive mechanisms 92ₘ, 92ₛ respectively comprise worm drives 510 and pinion gears 512 that are operably coupled between the manual actuators 102ₘ, 102ₛ of the master and slave shuttles 72ₘ, 72ₛ, the control shaft 500b, and the track 70-1 to facilitate the afore-described synchronized axial translation of the master and slave shuttles 72ₘ, 72ₛ.

In particular, the pinion gears 512 are operably coupled between the manual actuators 102ₘ, 102ₛ of the master and slave shuttles 72ₘ, 72ₛ and teeth 84-1 of the track 70-1 to effect axial translation of the master and slave shuttles 72ₘ, 72ₛ along the track 70-1, whereas the worm drives 508 of the axial drive mechanisms 92ₘ, 92ₛ respectively comprise worm screws 514 between which the control shaft 500b is operably coupled, and worm gears 516 operably coupled between the respective manual actuators 102ₘ, 102ₛ and worm screws 514 to synchronize the axial displacement of the master and slave shuttles 72ₘ, 72ₛ along the track 70-1.

In the illustrated embodiment, the pinion gear 512 of the master shuttle 72ₘ is mechanically coupled between the manual actuator 102ₘ of the master shuttle 72ₘ and the teeth 84-1 of the track 70-1, such that rotation of the manual actuator 102ₘ in one of the clockwise or counterclockwise direction 502a about its rotational axis 504a rotates the pinion gear 512 of the master shuttle 72ₘ, which, in turn, engages the teeth 84-1 of the track 70-1 to axially translate the master shuttle 72ₘ along the track 70-1 in the proximal direction 150a or the distal direction 150b. Similarly, the pinion gear 512 of the slave shuttle 72ₛ is mechanically coupled between the optional manual actuator 102ₛ of the slave shuttle 72ₛ and the teeth 84-1 of the track 70-1, such that rotation of the optional manual actuator 102ₛ in one of the clockwise or counterclockwise direction 502b about its rotational axis 504b rotates the pinion gear 512 of the slave shuttle 72ₛ, which, in turn, engages the teeth 84-1 of the track 70-1 to axially translate the slave shuttle 72ₛ along the track 70-1 in the proximal direction 150a or the distal direction 150b. Although the pinion gears 512 are illustrated as being directly coupled to the manual actuators 102ₘ, 102ₛ of the master and slave shuttles 72ₘ, 72ₛ, it should be appreciated that the pinion gears 512 may be indirectly coupled to the manual actuators 102ₘ, 102ₛ of the master and slave shuttles 72ₘ, 72ₛ via a shaft (not shown) or even another gear mechanism (not shown).

The worm gear 516 of the master shuttle 72ₘ, is mechanically coupled between the manual actuator 102ₘ and the worm screw 514 of the master shuttle 72ₘ, such that rotation of the manual actuator 102ₘ in one of the clockwise or counterclockwise directions 502a about its rotational axis 504a rotates the worm gear 516, which in turn, rotates the worm screw 514, of the master shuttle 72ₘ.

The worm screws 514 of the axial drive mechanisms 92ₘ, 92ₛ respectively have bores 518 (shown in **Fig. 59**) through which the control shaft 500b is disposed. The bores 518 of the worm screws 514 and the outer periphery of the control shaft 500b are keyed, such that worms screws 514 and control shaft 500b rotate in unison. That is, rotation of the worm screw 514 of the master shuttle 72ₘ rotates the control shaft 500b, which, in turn, rotates the worm screw 514 of the slave shuttle 72ₛ. As one example, the cross-sections of the bores 518 and outer periphery of the control shaft 500b may be cross-shaped, as illustrated in **Fig. 59****.** Of course, other non-circular matching cross-sectional shapes can be used to key the bores 518 of the worm screws 514 and the outer periphery of the control shaft 500b.

The worm gear 516 of the slave shuttle 72ₛ is mechanically coupled between the optional manual actuator 102ₛ and the worm screw 514 of the slave shuttle 72ₛ, such that rotation of the worm screw 514 rotates the worm gear 516, which, in turn, rotates the optional manual actuator 102ₛ of the slave shuttle 72ₛ in one of the clockwise or counterclockwise direction 502b about its rotational axis 504b, which, in turn, rotates the pinion gear 512 of the slave shuttle 72ₛ, which, in turn, engages the teeth 84-1 of the track 70-1 to axially translate the slave shuttle 72ₛ along the track 70-1 in the proximal direction 150a or the distal direction 150b. In the case where the slave shuttle 72ₛ is not provided with a manual actuator 102ₛ, the worm gear 516 of the slave shuttle 72ₛ will be mechanically coupled between the worm screw 514 of the slave shuttle 72ₛ and the pinion gear 516, such that rotation of the worm screw 514 rotates the worm gear 516, which, in turn, rotates the pinion gear 512 of the slave shuttle 72ₛ, which, in turn, engages the teeth 84-1 of the track 70-1 to axially translate the slave shuttle 72ₛ along the track 70-1 in the proximal direction 150a or the distal direction 150b.

In the case where the functions of the shuttles 72ₘ, 72ₛ are switched, such that the shuttle 72ₛ may serve as the master shuttle, and the shuttle 72ₘ may serve as the slave shuttle, such that manipulation of the manual actuator 102ₛ causes the shuttle 72ₛ to axially translate along the track 70-1. In particular, rotation of the manual actuator 102ₛ in one of the clockwise or counterclockwise directions 502b about its rotational axis 504b rotates the worm gear 516, which in turn, rotates the worm screw 514, of the shuttle 72ₛ, which, in turn, rotates the control shaft 500b, which, in turn, rotates the worm screw 514 of the shuttle 72ₘ, which, in turn, rotates the worm gear 516 of the shuttle 72ₘ, which, in turn, rotates the manual actuator 102ₘ of the shuttle 72ₘ in one of the clockwise or counterclockwise direction 502a about its rotational axis 504a, which, in turn, rotates the pinion gear 512 of the shuttle 72ₘ, which, in turn, engages the teeth 84-1 of the track 70-1 to axially translate the shuttle 72ₘ along the track 70-1 in the proximal direction 150a or the distal direction 150b.

As briefly discussed above, the control shaft 500b and master shuttle 72ₘ are configured for axially translating in unison, while the control shaft 500b and slave shuttle 72ₛ are configured for axially translating relative to each other. As also briefly discussed above, the control shaft 500b may be mechanically coupled between the first and second axial drive mechanisms 92ₘ, 92ₛ, and in this case, between the worm screws 514, in a detachable manner, such that the endovascular management and tracking system 14-1 may be switched between the master-slave configuration and independent configuration. In the illustrated embodiment, one end of the control shaft 500b is slid through the bore 518 of the worm screw 514 of the master shuttle 72ₘ and affixed within a bushing 520 of the master shuttle 72ₘ via a frictional fit, thereby preventing the control shaft 500b from axially translating relative to the master shuttle 72ₘ, while the other end of the control shaft 500b is slid through bore 518 of the worm screw 514 of the slave shuttle 72ₛ, thereby allowing the control shaft 500b to axially translate relative to the slave shuttle 72ₛ. To remove the control shaft 500b from the master shuttle 72ₘ and slave shuttle 72ₛ, the one end of the control shaft 500b may be removed from the bushing 520 and slid out from the bore 518 of the worm screw 514 of the master shuttle 72ₘ, while the other end of the control shaft 500b may simply be slid out from the bore 518 of the worm screw 514 of the slave shuttle 72ₛ.

As also briefly discussed above, the axial drive mechanisms 92ₘ, 92ₛ of the respective master and slave shuttles 72ₘ, 72ₛ may be designed to provide a specific axial translation ratio (positive or negative) between the master and slave shuttles 72ₘ, 72ₛ. For example, the worm screws 514 of the master and slave shuttles 72ₘ, 72ₛ may have the same pitch, such that the axial translation ratio may be unity (the master and slave shuttles 72ₘ, 72ₛ axially translate the track 70-1 the same distance), or may have different pitches, such that the axial translation ratio may be greater than or less than unity (the master and slave shuttles 72ₘ, 72ₛ axially translate the track 70-1 different distances), and/or the worm screws 514 of the master and slave shuttles 72ₘ, 72ₛ may have pitches that are oriented in the same direction, such that the axial translation ratio is positive (the master and slave shuttles 72ₘ, 72ₛ axially translate the track 70-1 the same direction), or may have pitches that are oriented in the opposite direction, such that the axial translation ratio is negative (the master and slave shuttles 72ₘ, 72ₛ axially translate the track 70-1 in opposite directions). In one embodiment, the worm drives 508 may be removably disposed within either or both of the master and slave shuttles 72ₘ, 72ₛ, such that worm drives 508 having different pitches for the worm screws 514 may be swapped in and out of the master shuttle 72ₘ and/or slave shuttle 72ₛ, such that the synchronized axial translation of the master shuttle 72ₘ and slave shuttle 72ₛ can be customized (i.e., different relative axial translations between the master shuttle 72ₘ and slave shuttle 72ₛ can be selected) to the specific medical procedure to be performed.

The synchronization of elongate medical devices using the endovascular management and tracking system 14-1 illustrated in **Fig. 56** (either using an electrical controller or a mechanical controller) lends itself well to medical procedures that require simultaneous translation of coaxial medical devices relative to each other.

For example, for stent deployment procedures, one of the elongate medical devices may be a stent deployment catheter, and the other of the elongate medical devices may be a pusher member disposed affixed to a stent within the stent deployment catheter. When deploying the stent from the stent deployment catheter, it is desirable to proximally retract the stent deployment catheter while distally moving the pusher member, and thus, the stent out of the stent deployment catheter.

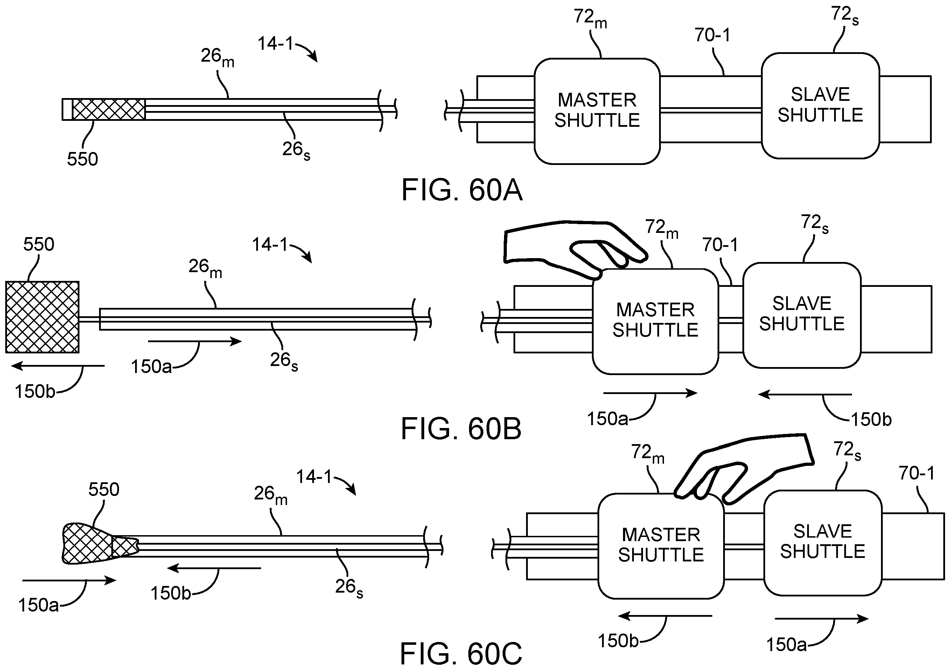

As applied to the endovascular management and tracking system 14-1 illustrated in **Fig. 60A****,** the elongate medical device 26ₘ affixed to the master shuttle 72ₘ (in this case, the distal or subsequent shuttle) may be the stent deployment catheter, while the elongate medical device 26ₛ affixed to the slave shuttle 72ₛ (in this case, the proximal or preceding shuttle) may be a pusher member to which a stent 550 is affixed. Thus, as illustrated in **Fig. 60B****,** in response to manually translating the master shuttle 72ₘ along the track 70-1 in the proximal direction 150a to retract the stent deployment catheter 26ₘ in the proximal direction 150a, the controller 500 will axially translate the slave shuttle 72ₛ along the track 70-1 in the distal direction 150b to distally advance the pusher member 26ₘ (i.e., at a negative axial translation ratio, e.g., unity), thereby deploying the stent 550 from the distal end of the stent deployment catheter 26ₘ, and allowing the stent 550 to expand into contact with the inner walls of a blood vessel (not shown). As illustrated in **Fig. 60C****,** if sub-optimal deployment of the stent 550 is occurring, deployment of the stent 550 may be reversed. In particular, in response to manually translating the master shuttle 72ₘ along the track 70-1 in the distal direction 150b to advance the stent deployment catheter 26ₘ in the distal direction 150b, the controller 500 will axially translate the slave shuttle 72ₛ along the track 70-1 in the proximal direction 150a to proximally retract the pusher member 26ₘ (i.e., at a negative axial translation ratio, e.g., unity), thereby resheathing the partially deployed stent 550 into the distal end of the stent deployment catheter 26ₘ.

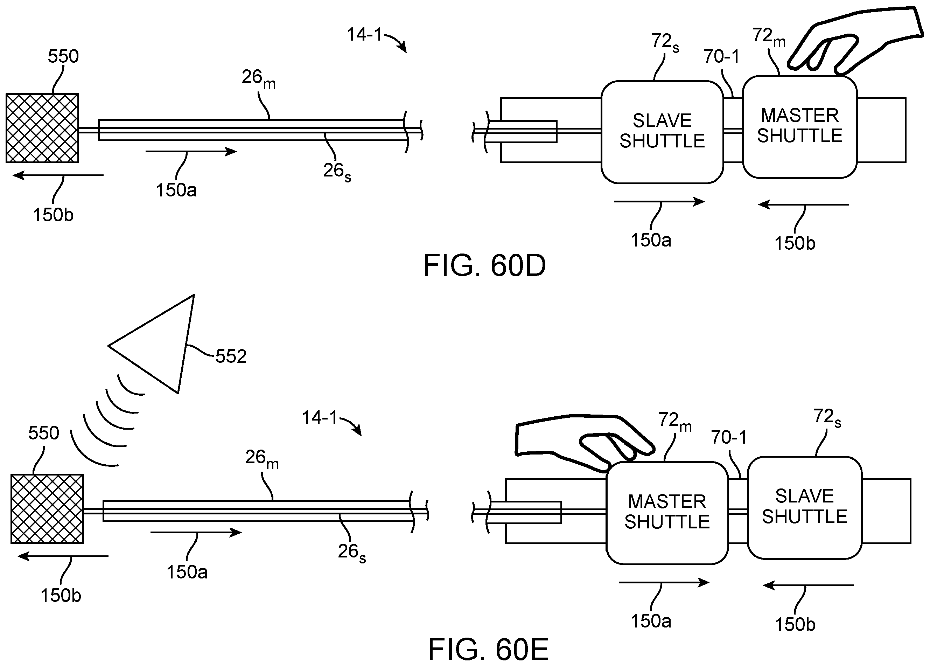

In an alternative embodiment illustrated in **Fig. 60D****,** the master shuttle 72ₘ may instead be the preceding shuttles, in which case, the elongate medical device 26ₛ affixed to the slave shuttle 72ₛ will be the pusher member to which the stent 550 is affixed, and the slave shuttle 72ₛ may be the subsequent shuttle, in which case, the elongate medical device 26ₘ affixed to the master shuttle 72ₘ will be the stent deployment catheter. In this case, in response to manually translating the master shuttle 72ₘ along the track 70-1 in the distal direction 150b to advance the pusher member 26ₘ in the distal direction 150b, the controller 500 will axially translate the slave shuttle 72ₛ along the track 70-1 in the proximal direction 150a to proximally retract the stent deployment catheter 26ₛ (i.e., at a negative axial translation ratio, e.g., unity), thereby deploying a stent 550 from the distal end of the stent deployment catheter 26ₘ, and allowing the stent 550 to expand into contact with the inner walls of a blood vessel (not shown). In the case where sub-optimal deployment of the stent 550 is occurring, in response to manually translating the master shuttle 72ₘ along the track 70-1 in the proximal direction 150a to retract the pusher member 26ₘ in the proximal direction 150a, the controller 500 will axially translate the slave shuttle 72ₛ along the track 70-1 in the distal direction 150b to distally advance the stent deployment catheter 26ₛ (i.e., at a negative axial translation ratio, e.g., unity), thereby resheathing the partially deployed stent 550 into the distal end of the stent deployment catheter 26ₛ.

In another embodiment, the axial translation ratio at which the controller 500 automatically translates the slave shuttle 72ₛ along the track 70-1 in response to the manual translation of the master shuttle 72ₘ along the track 70-1 may vary, e.g., depending upon one or more circumstances. In this case, the controller 500 may be electric, such that the axial translation ratio may be dynamically varied. In one example illustrated in **Fig. 60E****,** the endovascular management and tracking system 14-1 further comprises a medical imager (e.g., a fluoroscopic imager) 552 configured for imaging the stent 550 and providing the image to the controller 500. In this regard, the stent 550 may be composed of radiopaque elements. The controller 500 may be configured for identifying the stage of deployment of the stent 550 (e.g., whether or not the stent 550 has reached its intended deployment diameter) based on the image acquired by the medical imager 552. As the stent 550 is deployed from the stent deployment catheter in response to manual translation of the master shuttle 72ₘ, as discussed above with respect to **Fig. 60B** (or alternatively, **Fig. 60D**), the controller 500 dynamically varies the axial translation ratio to ensure optimal stent-to-vascular wall apposition.

As another example of a medical procedure to which the endovascular management and tracking system 14-1 illustrated in **Fig. 56** lends itself well, one of the elongate medical devices may be a guidewire, and the other of the elongate medical devices may be a catheter. When navigating the guidewire through the vasculature of the patient 20, it is desirable that the catheter follow the guidewire at a preset set-back distance from the tip of the guidewire.

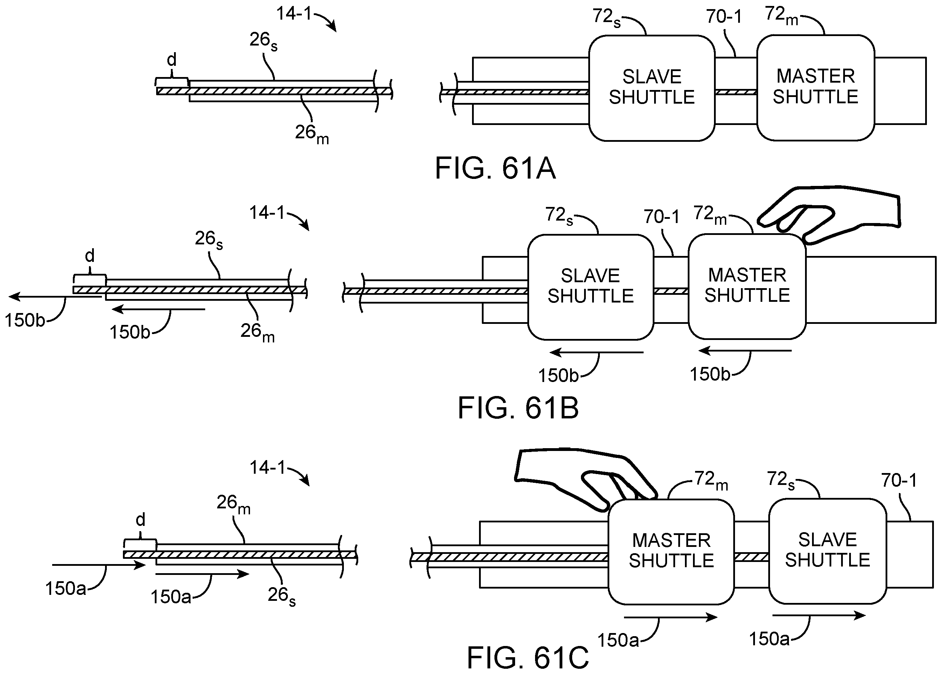

As applied to the endovascular management and tracking system 14-1 illustrated in **Fig. 61A****,** the elongate medical device 26ₘ affixed to the master shuttle 72ₘ (in this case, the preceding shuttle) may be the guidewire, while the elongate medical device 26ₛ affixed to the slave shuttle 72ₛ (in this case, the subsequent shuttle) may be the catheter. Thus, as illustrated in **Fig. 61B****,** in response to manually translating the master shuttle 72ₘ along the track 70-1 in the distal direction 150b to advance the guidewire 26ₘ in the distal direction 150b, the controller 500 will axially translate the slave shuttle 72ₛ along the track 70-1 in the distal direction 150b to distally advance the catheter 26ₛ (i.e., at a positive axial translation ratio, e.g., unity), thereby following the guidewire 26ₘ a predetermined offset distance d from the tip of the guidewire 26ₘ. As illustrated in **Fig. 61C****,** the subsequent shuttle may then be switched from a slave shuttle 72ₛ to a master shuttle 72ₘ by manually translating the master shuttle 72ₘ (now the subsequent shuttle) along the track 70-1 in the proximal direction 150a to retract the elongate medical device 26ₘ (now the catheter 26ₘ) in the proximal direction 150a. In response, the controller 500 will axially translate the slave shuttle 72ₛ (now the preceding shuttle) along the track 70-1 in the proximal direction 150a to proximally retract the guidewire 26ₛ (i.e., at a positive axial translation ratio, e.g., unity), thereby maintaining the position of the guidewire 26ₛ relative to the catheter 26ₘ (i.e., the predetermined offset distance d).

As still another example of a medical procedure to which the endovascular management and tracking system 14-1 illustrated in **Fig. 56** lends itself well, one of the elongate medical devices may be a stentriever, and the other of the elongate medical devices may be a catheter. During a stentriever withdrawal after a thrombus has been captured, it is desirable that the catheter automatically retract upon reaching a predetermined stentriever to catheter offset distance and maintaining this a predetermined stentriever to catheter offset distance as the stentriever is retrieved.

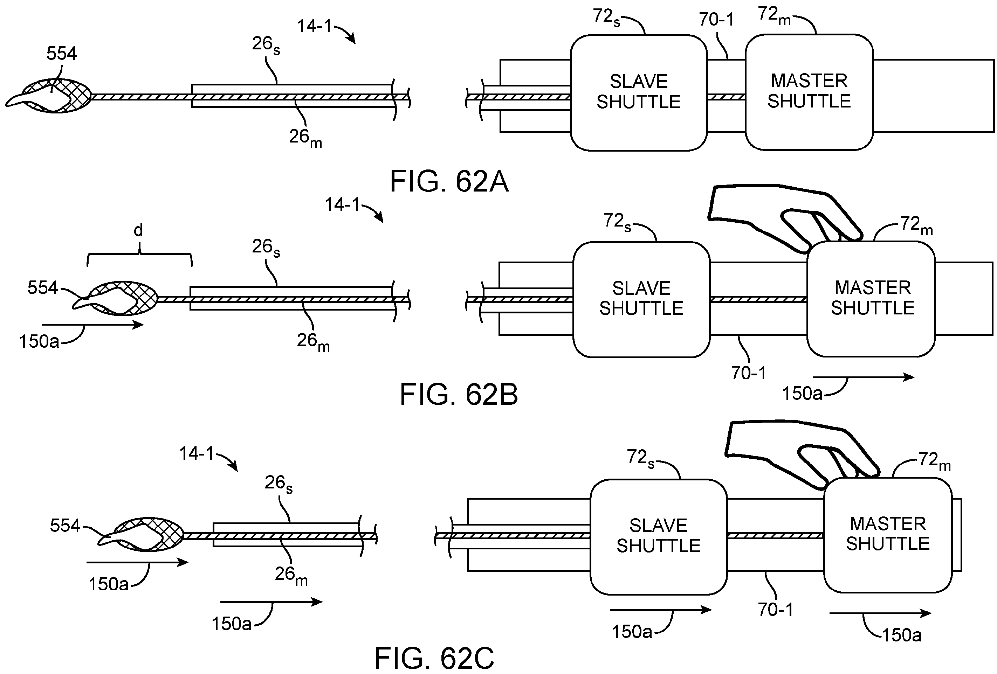

As applied to the endovascular management and tracking system 14-1 illustrated in **Fig. 62A****,** the elongate medical device 26ₘ affixed to the master shuttle 72ₘ (in this case, the preceding shuttle) may be a stentriever, while the elongate medical device 26ₛ affixed to the slave shuttle 72ₛ (in this case, the subsequent shuttle) may be the catheter. Thus, in response to manually translating the master shuttle 72ₘ along the track 70-1 in the proximal direction 150a to retract the stentriever 26ₘ, along with a captured thrombus 554, in the proximal direction 150a, the controller 500 will not axially translate the slave shuttle 72ₛ along the track 70-1, as illustrated in **Fig. 62B****,** until a predetermined stentreiver-to-catheter offset distance d is achieved. Then, in response to manually translating the master shuttle 72ₘ along the track 70-1 further in the proximal direction 150a to further retract the stentriever 26ₘ, along with a captured thrombus 554, in the proximal direction 150a, the controller 500 will axially translate the slave shuttle 72ₛ along the track 70-1 in the proximal direction 150a to proximally retract the catheter 26ₛ (i.e., at a positive axial translation ratio, e.g., unity), thereby maintaining the predetermined stentreiver-to-catheter offset distance d during the proximal retraction of the stentriever 26ₘ, as illustrated in **Fig. 62C****.**

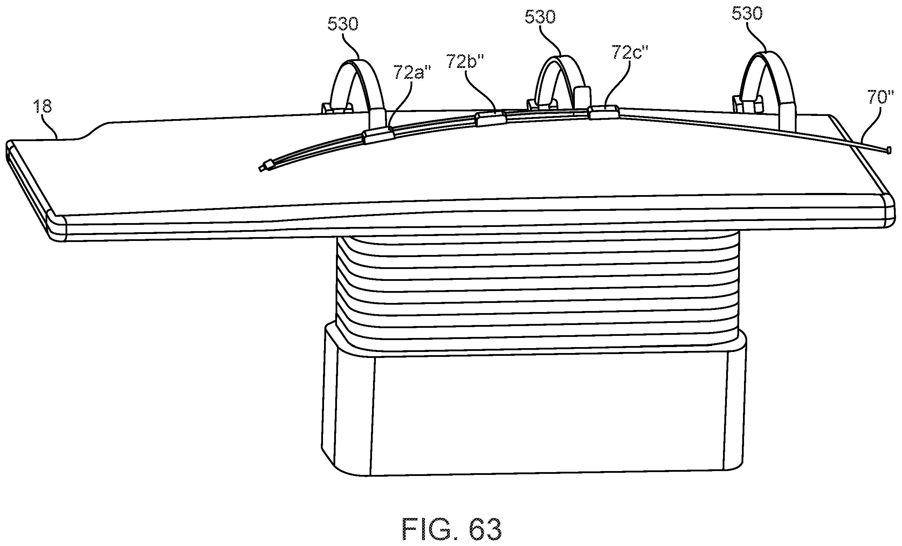

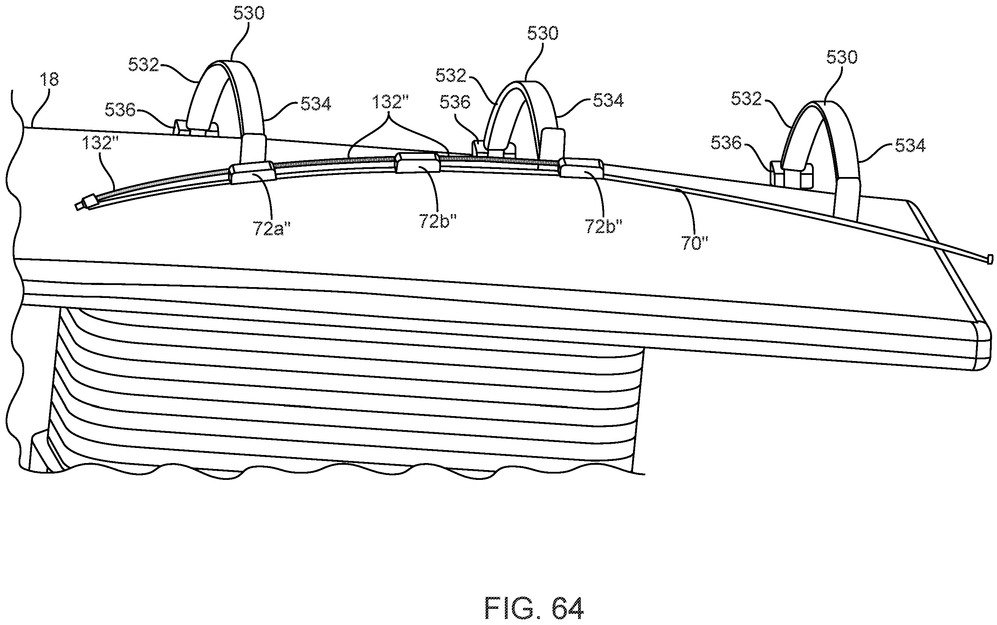

Although the previously described endovascular management and tracking systems 14, 14' have been described as having flexible tracks 70, 70' that are contoured to the hospital room environment, e.g., overlaid on the procedure table 18, the patient 20, and/or the drape 22, one embodiment of an endovascular management and tracking system 14" suspends a track 70" above a procedure table 18", as illustrated in **Figs. 63-65****.**

In particular, as with the endovascular management and tracking systems 14, 14', the endovascular management and tracking system 14" comprises the track 70" and a plurality of shuttles 72a"-72c" configured for being mechanically coupled to and axially translated along the track 70". Each of the shuttles 72a"-72c" may, e.g., be constructed in the same manner as the shuttles 72a-72c illustrated in **Fig. 6** or the shuttle 72' illustrated in **Figs. 21-24****.** The shuttles 72a"-72c" may also be provided with anti-buckling mechanisms 132" configured for respectively preventing respective elongate medical device (not shown) from prolapsing in response to axial compression applied to the respective elongate medical device.

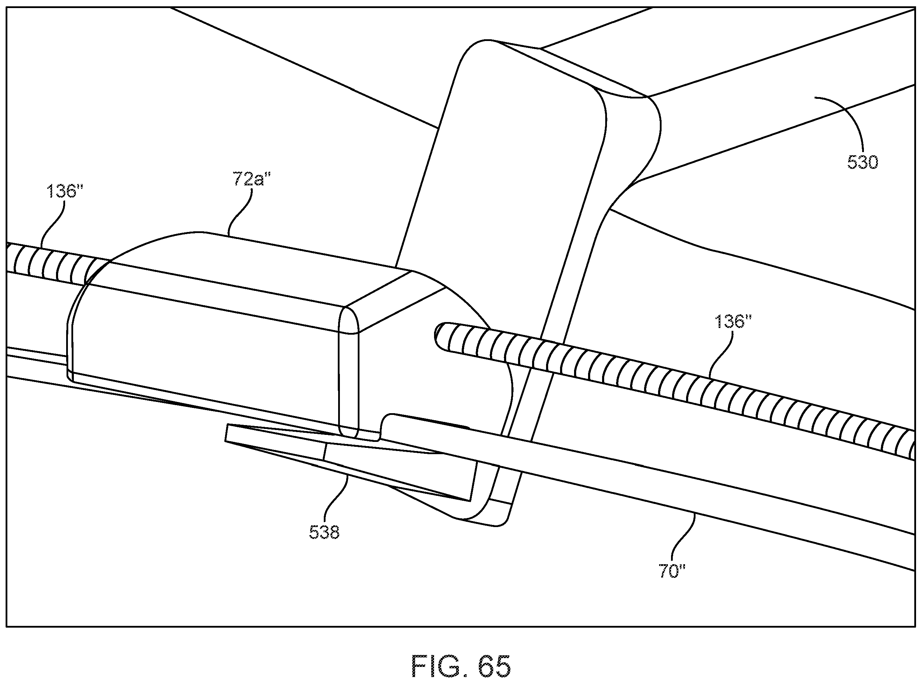

However, the endovascular management and tracking systems 14" further comprising a plurality of track support arms 530. One end 532 of each of the track support arms 530 is configured for being affixed to the procedure table 18" and the other end 534 of each of the track support arms 530 is configured for being detachable affixed to the track 70" (e.g., via a clamp mechanism 536), such that the track 70" may be suspended above the procedure table 18". The ends 532 of the track support arms 530 may be permanently affixed or detachably affixed to the procedure table 18". As best illustrated in **Fig. 65****,** the endovascular management and tracking systems 14" further comprises a plurality of feet 532 affixed to the other ends 534 of the track support arms 530, with the track 70" being configured for being affixed atop the feet 538. In an optional embodiment, the bottom surface of the track 70" has a channel (not shown) along the length of the track 70", and each of the feet 538 comprises a cleat (not shown) configured for being detachably affixed to the channel of the track 70".

The track support arms 530 are preferably rigid, such that the track 70" is stably suspended above the procedure table 18" as the shuttles 72a"-72c" are axially translated along the track 70". In the preferred embodiment, the track support arms 530 may be configured for being adjusted relative to the procedure table 18", such that the height of the other ends 534 of the track support arms 530 relative to the procedure table 18" can be selected, and/or the feet 538 may be configured for being adjusted relative to the other ends 534 of the track support arms 530, such that the height of the feet 538 relative to the procedure table 18" can be selected. In this manner, the contour of the track 70" in free space can be modified or customized. In the illustrated embodiment, the track 70" is contoured to form an arch that facilitates support of the shuttles 72a"-72c" against gravity.

Parts of the description and drawings referring to embodiments which are not covered by the claims are not presented as embodiments of claimed subject matter but as background examples useful for understanding the invention and may, if applicable, provide details to further specify embodiments claimed or described in this application.

## Claims

1. An endovascular management and tracking system (10), comprising:
a track (70, 70', 70"); and
at least one shuttle (72, 72') to which at least one elongate medical device (26, 26') can be respectively affixed, each of the at least one shuttle (72, 72') configured for being mechanically coupled to the track (70, 70', 70"), and comprising a sled (90, 90') configured for riding on the track (70, 70', 70"), an axial drive mechanism (92, 92') carried by the sled (90, 90') and configured for being actuated to axially translate the respective shuttle (72, 72') along the track (70, 70', 70"), and an actuator (94, 94', 102, 102') carried by the sled (90, 90') and configured for actuating the axial drive mechanism (92, 92'), **characterized in that**:
each of the at least one shuttle (72, 72') further comprises a locking mechanism (106) carried by the sled (90, 90') and configured for being manually operated to alternately engage and disengage the axial drive mechanism (92, 92') to the track (70, 70', 70").

2. The endovascular management and tracking system of claim 1, wherein each of the at least one elongate medical device (26, 26') is one of a guide sheath, a working catheter, and a guidewire.

3. The endovascular management and tracking system (10) of any of claims 1-2, wherein each of the at least one shuttle (72a-72c) comprises a casing (100) mounted to the sled (90, 90') and containing the axial drive mechanism (92, 92') and the actuator (94', 94', 102, 102').

4. The endovascular management and tracking system of any of claims 1-3, wherein each of the at least one shuttle (72a-72c) is configured for being threaded onto the track (70, 70', 70").

5. The endovascular management and tracking system of any of claims 1-4, wherein each of the at least one shuttle (72a-72c) is configured for being laterally mated to the track (70, 70', 70").

6. The endovascular management and tracking system of any of claims 1-5, wherein the at least one shuttle (72, 72') comprises a plurality of shuttles (72a-72c), the at least one elongate medical device (26, 26') comprises a plurality of elongate medical devices (26, 26'), and each subsequent one of the plurality of shuttles (72a-72c) is configured for slidably receiving a distal end of a respective one of the plurality of elongate medical devices (26a-26c) affixed to a preceding one of the plurality of shuttles (72a-72c), such that the respective elongate medical device (26, 26') affixed to the preceding shuttle (72b-72c) is coaxially disposed within a respective one of the plurality of elongate medical devices (26a, 26b) affixed to the subsequent shuttle (72a-72b).

7. The endovascular management and tracking system of any of claims 1-6, wherein each of the at least one shuttle (72, 72') further comprising an on-board fluid management control assembly (110) carried by the sled (90, 90') and configured for being fluidly coupled to the elongate medical device (26, 26') affixed to the respective shuttle (72, 72').

8. The endovascular management and tracking system of claim 7, wherein the on-board fluid management control assembly (110) comprises a rotary hemostasis valve (RHV) (112).

9. The endovascular management and tracking system of claim 8, wherein the RHV (112) is configured for being removed from the respective shuttle (72, 72').

10. The endovascular management and tracking system of any of claims 1-9, wherein each of the at least one shuttle (72, 72') comprises a rotary drive mechanism (96, 96') carried by the sled (90, 90') and configured for being actuated to rotate the respective elongate medical device (26, 26') about its longitudinal axis, and another actuator (98, 98') carried by the sled (90, 90') and configured for actuating the rotary drive mechanism (96, 96').

11. The endovascular management and tracking system of any of claims 1-10, wherein each of the at least one shuttle (72a-72c) is configured for being detachably mechanically coupled to the track (70, 70', 70").

12. The endovascular management and tracking system of any of claims 1-11, wherein each of the at least one elongate medical device (26, 26') is flexible, and wherein each of one or more of the at least one shuttle (72, 72') further comprises an anti-buckling mechanism (132, 132', 132", 132a - 132d, 132-1 - 132-4) configured for preventing the respective elongate medical device (26, 26') from prolapsing in response to axial compression applied to the respective elongate medical device (26, 26').

13. The endovascular management and tracking system of claim 12, wherein each of the one or more anti-buckling mechanisms (132, 132', 132", 132a - 132d, 132-1 - 132-4) comprises a tubular member through which the respective elongate medical device (26, 26') is configured for being slidably disposed.

14. The endovascular management and tracking system of any of claims 1-13, wherein the actuator (94, 94', 102, 102') is a manual actuator (102, 102').

15. The endovascular management and tracking system of any of claims 1-13, wherein the actuator (94, 94', 102, 102') is a motor (94, 94').

## Patentansprüche

1. Endovaskuläres Verwaltungs- und Verfolgungssystem (10), umfassend:
eine Schiene (70, 70', 70"); und
mindestens ein Schiffchen (72, 72'), an dem jeweils mindestens eine längliche medizinische Vorrichtung (26, 26') angebracht werden kann, wobei jedes von dem mindestens einen Schiffchen (72, 72') zur mechanischen Kopplung mit der Schiene (70, 70', 70") ausgestaltet ist und einen Schlitten (90, 90'), der zum Fahren auf der Schiene (70, 70', 70") ausgestaltet ist, einen Axialantriebsmechanismus (92, 92'), der von dem Schlitten (90, 90') getragen wird und so ausgestaltet ist, dass er betätigt wird, um das j eweilige Schiffchen (72, 72') axial entlang der Schiene (70, 70', 70") zu verschieben, und einen Aktuator (94, 94', 102, 102'), der von dem Schlitten (90, 90') getragen wird und zum Betätigen des Axialantriebsmechanismus (92, 92') ausgestaltet ist, umfasst, **dadurch gekennzeichnet, dass**:
jedes von dem mindestens einen Schiffchen (72, 72') ferner einen Verriegelungsmechanismus (106) umfasst, der von dem Schlitten (90, 90') getragen wird und so ausgestaltet ist, dass er manuell bedient wird, um den Axialantriebsmechanismus (92, 92') abwechselnd mit der Schiene (70, 70', 70") in Eingriff zu bringen und von ihr zu lösen.

2. Endovaskuläres Verwaltungs- und Verfolgungssystem nach Anspruch 1, wobei jede von der mindestens einen länglichen medizinischen Vorrichtung (26, 26') eines von einer Führungshülse, einem Arbeitskatheter und einem Führungsdraht ist.

3. Endovaskuläres Verwaltungs- und Verfolgungssystem (10) nach einem beliebigen der Ansprüche 1-2, wobei jedes von dem mindestens einen Schiffchen (72a-72c) ein Gehäuse (100) umfasst, das an dem Schlitten (90, 90') montiert ist und den Axialantriebsmechanismus (92, 92') und den Aktuator (94, 94', 102, 102') umfasst.

4. Endovaskuläres Verwaltungs- und Verfolgungssystem nach einem beliebigen der Ansprüche 1-3, wobei jedes von dem mindestens einen Schiffchen (72a-72c) dafür ausgestaltet ist, auf die Schiene (70, 70', 70") aufgeschraubt zu werden.

5. Endovaskuläres Verwaltungs- und Verfolgungssystem nach einem beliebigen der Ansprüche 1-4, wobei jedes von dem mindestens einen Schiffchen (72a-72c) dafür ausgestaltet ist, seitlich in die Schiene (70, 70', 70") einzugreifen.

6. Endovaskuläres Verwaltungs- und Verfolgungssystem nach einem beliebigen der Ansprüche 1-5, wobei das mindestens eine Schiffchen (72, 72') eine Vielzahl von Schiffchen (72a-72c) umfasst, die mindestens eine längliche medizinische Vorrichtung (26, 26') eine Vielzahl von länglichen medizinischen Vorrichtungen (26, 26') umfasst, und jedes nachfolgende der Vielzahl von Schiffchen (72a-72c) zur gleitenden Aufnahme eines distalen Endes einer jeweiligen der Vielzahl von länglichen medizinischen Vorrichtungen (26a-26c), die an einem vorhergehenden der Vielzahl von Schiffchen (72a-72c) angebracht ist, ausgestaltet ist, sodass die jeweilige medizinische Vorrichtung (26, 26'), die an dem vorhergehenden Schiffchen (72b-72c) angebracht ist, koaxial innerhalb einer jeweiligen der Vielzahl von länglichen medizinischen Vorrichtungen (26a, 26b), die an dem nachfolgenden Schiffchen (72a-72b) angebracht ist, angeordnet ist.

7. Endovaskuläres Verwaltungs- und Verfolgungssystem nach einem beliebigen der Ansprüche 1-6, wobei jedes von dem mindestens einen Schiffchen (72, 72') ferner eine bordseitige Fluidverwaltungssteueranordnung (110) umfasst, die von dem Schlitten (90, 90') getragen wird und zur fluidischen Kopplung mit der länglichen medizinischen Vorrichtung (26, 26'), die an dem jeweiligen Schiffchen (72, 72') angebracht ist, ausgestaltet ist.

8. Endovaskuläres Verwaltungs- und Verfolgungssystem nach Anspruch 7, wobei die bordseitige Fluidverwaltungssteueranordnung (110) ein Hämostase-Drehventil (RHV) (112) umfasst.

9. Endovaskuläres Verwaltungs- und Verfolgungssystem nach Anspruch 8, wobei das RHV (112) zur Entnahme aus dem jeweiligen Schiffchen (72, 72') ausgestaltet ist.

10. Endovaskuläres Verwaltungs- und Verfolgungssystem nach einem beliebigen der Ansprüche 1-9, wobei jedes von dem mindestens einen Schiffchen (72, 72') einen Drehantriebsmechanismus (96, 96'), der von dem Schlitten (90, 90') getragen wird und so ausgestaltet ist, dass er betätigt wird, um die jeweilige längliche medizinische Vorrichtung (26, 26') um ihre Längsachse zu bewegen, und einen weiteren Aktuator (98, 98'), der von dem Schlitten (90, 90') getragen wird und zum Betätigen des Drehantriebsmechanismus (96, 96') ausgestaltet ist, umfasst.

11. Endovaskuläres Verwaltungs- und Verfolgungssystem nach einem beliebigen der Ansprüche 1-10, wobei jedes von dem mindestens einen Schiffchen (72a-72c) zur lösbaren mechanischen Kopplung mit der Schiene (70, 70', 70") ausgestaltet ist.

12. Endovaskuläres Verwaltungs- und Verfolgungssystem nach einem beliebigen der Ansprüche 1-11, wobei jede von der mindestens einen länglichen medizinischen Vorrichtung (26, 26') flexibel ist, und wobei jedes von einem oder mehreren des mindestens einen Schiffchens (72, 72') ferner einen Knickschutzmechanismus (132, 132', 132", 132a-132d, 132-1-132-4) umfasst, der so ausgestaltet ist, dass er ein Prolabieren der jeweiligen länglichen medizinischen Vorrichtung (26, 26') als Reaktion auf Axialkompression, die auf die jeweilige längliche medizinische Vorrichtung (26, 26') ausgeübt wird, verhindert.

13. Endovaskuläres Verwaltungs- und Verfolgungssystem nach Anspruch 12, wobei jeder von dem einen oder den mehreren Knickschutzmechanismen (132, 132', 132", 132a-132d, 132-1-132-4) ein röhrenförmiges Element umfasst, wobei die jeweilige längliche medizinische Vorrichtung (26, 26') zur gleitenden Anordnung durch dieses hindurch ausgestaltet ist.

14. Endovaskuläres Verwaltungs- und Verfolgungssystem nach einem beliebigen der Ansprüche 1-13, wobei der Aktuator (94, 94', 102, 102') ein manueller Aktuator (102, 102') ist.

15. Endovaskuläres Verwaltungs- und Verfolgungssystem nach einem beliebigen der Ansprüche 1-13, wobei der Aktuator (94, 94', 102, 102') ein Motor (94, 94') ist.

## Revendications

1. Système de gestion et de suivi endovasculaire (10), comprenant :
une piste (70, 70', 70") ; et
au moins une navette (72, 72') à laquelle au moins un dispositif médical allongé (26, 26') peut être fixé respectivement, chacune des au moins une navette (72, 72') étant configurée pour être accouplée mécaniquement à la piste (70, 70', 70"), et comprenant un traîneau (90, 90') configuré pour circuler sur la piste (70, 70', 70"), un mécanisme d'entraînement axial (92, 92') porté par le traîneau (90, 90') et configuré pour être actionné pour déplacer la navette (72, 72') respective axialement le long de la piste (70, 70', 70"), et un actionneur (94, 94', 102, 102') porté par le traîneau (90, 90') et configuré pour actionner le mécanisme d'entraînement axial (92, 92'), **caractérisé en ce que** :
chacune des au moins une navette (72, 72') comprend en outre un mécanisme de verrouillage (106) porté par le traîneau (90, 90') et configuré pour être commandé manuellement pour engager et désengager alternativement le mécanisme d'entraînement axial (92, 92') par rapport à la piste (70, 70', 70").

2. Système de gestion et de suivi endovasculaire selon la revendication 1, dans lequel chacun des au moins un dispositif médical allongé (26, 26') est l'un parmi une gaine de guidage, un cathéter de travail, et un fil de guidage.

3. Système de gestion et de suivi endovasculaire (10) selon l'une quelconque des revendications 1 à 2, dans lequel chacune des au moins une navette (72a - 72c) comprend un boîtier (100) monté sur le traîneau (90, 90') et contenant le mécanisme d'entraînement axial (92, 92') et l'actionneur (94, 94', 102, 102').

4. Système de gestion et de suivi endovasculaire selon l'une quelconque des revendications 1 à 3, dans lequel chacune des au moins une navette (72a - 72c) est configurée pour être filetée sur la piste (70, 70', 70").

5. Système de gestion et de suivi endovasculaire selon l'une quelconque des revendications 1 à 4, dans lequel chacune des au moins une navette (72a - 72c) est configurée pour être appariée latéralement à la piste (70, 70', 70").

6. Système de gestion et de suivi endovasculaire selon l'une quelconque des revendications 1 à 5, dans lequel l'au moins une navette (72, 72') comprend une pluralité de navettes (72a - 72c), l'au moins un dispositif médical allongé (26, 26') comprend une pluralité de dispositifs médicaux allongés (26, 26'), et chaque navette suivante parmi la pluralité de navettes (72a - 72c) est configurée pour recevoir de façon coulissante une extrémité distale de l'un respectif parmi la pluralité de dispositif médicaux allongés (26a - 26c) fixé à une navette précédente parmi la pluralité de navettes (72a - 72c), de telle façon que le dispositif médical allongé (26, 26') respectif fixé à la navette précédente (72b - 72c) est disposé coaxialement à l'intérieur de l'un respectif parmi la pluralité de dispositif médicaux allongés (26a - 26c) fixé à la navette suivante (72a - 72b).

7. Système de gestion et de suivi endovasculaire selon l'une quelconque des revendications 1 à 6, dans lequel chacune des au moins une navette (72, 72') comprend en outre un ensemble de commande de gestion de fluide embarqué (110) porté par le traîneau (90, 90') et configuré pour être accouplé fluidiquement au dispositif médical allongé (26, 26') fixé à la navette (72, 72') respective.

8. Système de gestion et de suivi endovasculaire selon la revendication 7, dans lequel l'ensemble de commande de gestion de fluide embarqué (110) comprend une valve hémostatique rotative (RHV) (112).

9. Système de gestion et de suivi endovasculaire selon la revendication 8, dans lequel la RHV (112) est configurée pour être retirée de la navette (72, 72') respective.

10. Système de gestion et de suivi endovasculaire selon l'une quelconque des revendications 1 à 9, dans lequel chacune des au moins une navette (72, 72') comprend un mécanisme d'entraînement rotatif (96, 96') porté par le traîneau (90, 90') et configuré pour être actionné de manière à mettre en rotation le dispositif médical allongé (26, 26') respectif autour de son axe longitudinal, et un autre actionneur (98, 98') porté par le traîneau (90, 90') et configuré pour actionner le mécanisme d'entraînement rotatif (96, 96').

11. Système de gestion et de suivi endovasculaire selon l'une quelconque des revendications 1 à 10, dans lequel chacune des au moins une navette (72, 72') est configurée pour être accouplée mécaniquement de façon détachable à la piste (70, 70', 70").

12. Système de gestion et de suivi endovasculaire selon l'une quelconque des revendications 1 à 11, dans lequel chacun des au moins un dispositif médical allongé (26, 26') est flexible, et dans lequel chacune parmi une ou plusieurs des au moins une navette (72, 72') comprend en outre un mécanisme anti-tortillement (132, 132', 132", 132a - 132d, 132-1 - 132-4) configuré pour empêcher le dispositif médical allongé (26, 26') respectif de glisser en réponse à une compression axiale appliquée sur le dispositif médical allongé (26, 26') respectif.

13. Système de gestion et de suivi endovasculaire selon la revendication 12, dans lequel chacun des un ou plusieurs mécanisme anti-tortillement (132, 132', 132", 132a - 132d, 132-1 - 132-4) comprend un élément tubulaire à travers lequel le dispositif médical allongé (26, 26') respectif est configuré pour être disposé de façon coulissante.

14. Système de gestion et de suivi endovasculaire selon l'une quelconque des revendications 1 à 13, dans lequel l'actionneur (94, 94', 102, 102') est un actionneur manuel (102, 102').

15. Système de gestion et de suivi endovasculaire selon l'une quelconque des revendications 1 à 13, dans lequel l'actionneur (94, 94', 102, 102') est un moteur (94, 94').
